# EUROPEAN PATENT SPECIFICATION

(11) **EP 1 536 799 B1**
(45) Date of publication and mention of the grant of the patent: **10.05.2006**
(21) Application number: 03753402.1
(22) Date of filing: 10.09.2003
(51) Int. Cl.: A61K 31/517, C07D 403/04, A61P 3/06

(54) **NOVEL 2-AMINO-4-OXOQUINAZOLONES AS LXR NUCLEAR RECEPTOR BINDING COMPOUNDS WITH PARTIAL AGONISTIC PROPERTIES**
NEUE 2-AMINO-4-OXOCHINAZOLONE ALS PARTIELLE LXR-KERNREZEPTORAGONISTEN
NOUVELLES 2-AMINO-4-OXOQUINAZOLONES UTILISEES CO MME COMPOSES SE FIXANT AU RECEPTEUR NUCLEAIRE X DU FOIE ET DOTEES DE PROPRIETES AGONISTES PARTIELLES

(30) Priority: 10.09.2002 EP 02020255
(43) Date of publication of application: 08.06.2005
(73) Proprietor: PheneX Pharmaceuticals AG, 69120 Heidelberg (DE)
(72) Inventor: DEUSCHLE, Ulrich, 69123 Heidelberg (DE); KÖGL, Manfred, 69214 Eppelheim (DE); BLUME, Beatrix, 69221 Dossenheim (DE); ALBERS, Michael, 69221 Dossenheim (DE); KOBER, Ingo, 69251 Gaiberg (DE)
(74) Representative: Huhn, Michael
(86) International application number: PCT/EP2003/010036
(87) International publication number: WO 2004/024161

(56) References cited:
- WO-A-97/20823
- DATABASE CHEMCATS CHEMICAL ABSTRACTS SERVICES, COLUMBUS, OHIO, US; XP0002225148 & "Chem.Folio" 15 May 2001 (2001-05-15), LION BIOSCIENCE , HEIDELBERG, GERMANY
- COLLINS J L ET AL: "Identification of a Nonsteroidal Liver X Receptor Agonist through Parallel Array Synthesis of Tertiary Amines" JOURNAL OF MEDICINAL CHEMISTRY, AMERICAN CHEMICAL SOCIETY. WASHINGTON, US, vol. 45, 2002, pages 1963-1966, XP002225147 ISSN: 0022-2623
- GUPTA C M ET AL: "Drugs acting on the central nervous system. Syntheses of substituted quinazolones and quinazolines and triazepino- and triazocinoquinazolones" JOURNAL OF MEDICINAL CHEMISTRY, AMERICAN CHEMICAL SOCIETY. WASHINGTON, US, vol. 11, no. 2, 26 February 1968 (1968-02-26), pages 392-395, XP002156695 ISSN: 0022-2623

### SEQUENCE LISTING

<110> Phenex Pharmaceuticals AG
<120> Novel selective LXR Nuclear Receptor Binding Compounds with partial agonistic properties
<130> PX62420PC
<160> 23
<170> PatentIn version 3.1
<210> 1
   <211> 2010
   <212> DNA
   <213> Homo sapiens
<400> 1
   caagaagtgg cgaagttacc tttgagggta tttgagtagc ggcggtgtgt caggggctaa   60
   agaggaggac gaagaaaagc agagcaaggg aacccagggc aacaggagta gttcactccg  120
   cgagaggccg tccacgagac ccccgcgcgc aggcatgagc cccgcccccc acgcatgagc  180
   cccgcccccc gctgttgctt ggagaggggc gggacctgga gagaggctgc tccgtgaccc  240
   caccatgtcc tctcctacca cgagttccct ggataccccc ctgcctggaa atggcccccc  300
   tcagcctggc gccccttctt cttcacccac tgtaaaggag gagggtccgg agccgtggcc  360
   cgggggtccg gaccctgatg tcccaggcac tgatgaggcc agctcagcct gcagcacaga  420
   ctgggtcatc ccagatcccg aagaggaacc agagcgcaag cgaaagaagg gcccagcccc  480
   gaagatgctg ggccacgagc tttgccgtgt ctgtggggac aaggcctccg gcttccacta  540
   caacgtgctc agctgcgaag gctgcaaggg cttcttccgg cgcagtgtgg tccgtggtgg  600
   ggccaggcgc tatgcctgcc ggggtggcgg aacctgccag atggacgctt tcatgcggcg  660
   caagtgccag cagtgccggc tgcgcaagtg caaggaggca gggatgaggg agcagtgcgt  720
   cctttctgaa gaacagatcc ggaagaagaa gattcggaaa cagcagcagc aggagtcaca  780
   gtcacagtcg cagtcacctg tggggccgca gggcagcagc agctcagcct ctgggcctgg  840
   ggcttcccct ggtggatctg aggcaggcag ccagggctcc ggggaaggcg agggtgtcca  900
   gctaacagcg gctcaagaac taatgatcca gcagttggtg gcggcccaac tgcagtgcaa  960
   caaacgctcc ttctccgacc agcccaaagt cacgccctgg cccctgggcg cagaccccca 1020
   gtcccgagat gcccgccagc aacgctttgc ccacttcacg gagctggcca tcatctcagt 1080
   ccaggagatc gtggacttcg ctaagcaagt gcctggtttc ctgcagctgg gccgggagga 1140
   ccagatcgcc ctcctgaagg catccactat cgagatcatg ctgctagaga cagccaggcg 1200
   ctacaaccac gagacagagt gtatcacctt cttgaaggac ttcacctaca gcaaggacga 1260
   cttccaccgt gcaggcctgc aggtggagtt catcaacccc atcttcgagt tctcgcgggc 1320
   catgcggcgg ctgggcctgg acgacgctga gtacgccctg ctcatcgcca tcaacatctt 1380
   ctcggccgac cggcccaacg tgcaggagcc gggccgcgtg gaggcgttgc agcagcccta 1440
   cgtggaggcg ctgctgtcct acacgcgcat caagaggccg caggaccagc tgcgcttccc 1500
   gcgcatgctc atgaagctgg tgagcctgcg cacgctgagc tctgtgcact cggagcaggt 1560
   cttcgccttg cggctccagg acaagaagct gccgcctctg ctgtcggaga tctgggacgt 1620
   ccacgagtga ggggctggcc acccagcccc acagccttgc ctgaccaccc tccagcagat 1680
   agacgccggc accccttcct cttcctaggg tggaaggggc cctgggcgag cctgtagacc 1740
   tatcggctct catcccttgg gataagcccc agtccaggtc caggaggctc cctccctgcc 1800
   cagcgagtct tccagaaggg gtgaaagggt tgcaggtccc gaccactgac ccttcccggc 1860
   tgccctccct ccccagctta cacctcaagc ccagcacgca gcgtaccttg aacagaggga 1920
   ggggaggacc catggctctc cccccctagc ccgggagacc aggggccttc ctcttcctct 1980
   gcttttattt aataaaaata aaaacagaaa 2010
<210> 2
   <211> 1528
   <212> DNA
   <213> Homo sapiens
<400> 2
   cagtgccttg gtaatgacca gggctccaga aagagatgtc cttgtggctg ggggcccctg 60
   tgcctgacat tcctcctgac tctgcggtgg agctgtggaa gccaggcgca caggatgcaa 120
   gcagccaggc ccagggaggc agcagctgca tcctcagaga ggaagccagg atgccccact 180
   ctgctggggg tactgcaggg gtggggctgg aggctgcaga gcccacagcc ctgctcacca 240
   gggcagagcc cccttcagaa cccacagaga tccgtccaca aaagcggaaa aaggggccag 300
   cccccaaaat gctggggaac gagctatgca gcgtgtgtgg ggacaaggcc tcgggcttcc 360
   actacaatgt tctgagctgc gagggctgca agggattctt ccgccgcagc gtcatcaagg 420
   gagcgcacta catctgccac agtggcggcc actgccccat ggacacctac atgcgtcgca 480
   agtgccagga gtgtcggctt cgcaaatgcc gtcaggctgg catgcgggag gagtgtgtcc 540
   tgtcagaaga acagatccgc ctgaagaaac tgaagcggca agaggaggaa caggctcatg 600
   ccacatcctt gccccccagg cgttcctcac ccccccaaat cctgccccag ctcagcccgg 660
   aacaactggg catgatcgag aagctcgtcg ctgcccagca acagtgtaac cggcgctcct 720
   tttctgaccg gcttcgagtc acgccttggc ccatggcacc agatccccat agccgggagg 780
   cccgtcagca gcgctttgcc cacttcactg agctggccat cgtctctgtg caggagatag 840
   ttgactttgc taaacagcta cccggcttcc tgcagctcag ccgggaggac cagattgccc 900
   tgctgaagac ctctgcgatc gaggtgatgc ttctggagac atctcggagg tacaaccctg 960
   ggagtgagag tatcaccttc ctcaaggatt tcagttataa ccgggaagac tttgccaaag 1020
   cagggctgca agtggaattc atcaacccca tcttcgagtt ctccagggcc atgaatgagc 1080
   tgcaactcaa tgatgccgag tttgccttgc tcattgctat cagcatcttc tctgcagacc 1140
   ggcccaacgt gcaggaccag ctccaggtgg agaggctgca gcacacatat gtggaagccc 1200
   tgcatgccta cgtctccatc caccatcccc atgaccgact gatgttccca cggatgctaa 1260
   tgaaactggt gagcctccgg accctgagca gcgtccactc agagcaagtg tttgcactgc 1320
   gtctgcagga caaaaagctc ccaccgctgc tctctgagat ctgggatgtg cacgaatgac 1380
   tgttctgtcc ccatattttc tgttttcttg gccggatggc tgaggcctgg tggctgcctc 1440
   ctagaagtgg aacagactga gaagggcaaa cattcctggg agctgggcaa ggagatcctc 1500
   ccgtggcatt aaaagagagt caaagggt 1528
<210> 3
   <211> 2877
   <212> DNA
   <213> Homo sapiens
<400> 3
   gtggcatcct tccctttcta atcagagatt ttcttcctca gagattttgg cctagatttg 60
   caaaatgatg accacatctt tgatttgggg gattgctata gcagcatgct gttgtctatg 120
   gcttattctt ggaattagga gaaggcaaac gggtgaacca cctctagaga atggattaat 180
   tccatacctg ggctgtgctc tgcaatttgg tgccaatcct cttgagttcc tcagagcaaa 240
   tcaaaggaaa catggtcatg tttttacctg caaactaatg ggaaaatatg tccatttcat 300
   cacaaatccc ttgtcatacc ataaggtgtt gtgccacgga aaatattttg attggaaaaa 360
   atttcacttt gctacttctg cgaaggcatt tgggcacaga agcattgacc cgatggatgg 420
   aaataccact gaaaacataa acgacacttt catcaaaacc ctgcagggcc atgccttgaa 480
   ttccctcacg gaaagcatga tggaaaacct ccaacgtatc atgagacctc cagtctcctc 540
   taactcaaag accgctgcct gggtgacaga agggatgtat tctttctgct accgagtgat 600
   gtttgaagct gggtatttaa ctatctttgg cagagatctt acaaggcggg acacacagaa 660
   agcacatatt ctaaacaatc ttgacaactt caagcaattc gacaaagtct ttccagccct 720
   ggtagcaggc ctccccattc acatgttcag gactgcgcac aatgcccggg agaaactggc 780
   agagagcttg aggcacgaga acctccaaaa gagggaaagc atctcagaac tgatcagcct 840
   gcgcatgttt ctcaatgaca ctttgtccac ctttgatgat ctggagaagg ccaagacaca 900
   cctcgtggtc ctctgggcat cgcaagcaaa caccattcca gcgactttct ggagtttatt 960
   tcaaatgatt aggaacccag aagcaatgaa agcagctact gaagaagtga aaagaacatt 1020
   agagaatgct ggtcaaaaag tcagcttgga aggcaatcct atttgtttga gtcaagcaga 1080
   actgaatgac ctgccagtat taaatagtat aatcaaggaa tcgctgaggc tttccagtgc 1140
   ctccctcaac atccggacag ctaaggagga tttcactttg caccttgagg acggttccta 1200
   caacatccga aaagatagca tcatagctct ttacccacag ttaatgcact tagatccaga 1260
   aatctaccca gaccctttga cttttaaata tgataggtat cttgatgaaa acgggaagac 1320
   aaagactacc ttctattgta atggactcaa gttaaagtat tactacatgc cctttggatc 1380
   gggagctaca atatgtcctg gaagattgtt cgctatccac gaaatcaagc aatttttgat 1440
   tctgatgctt tcttattttg aattggagct tatagagggc caagctaaat gtccaccttt 1500
   ggaccagtcc cgggcaggct tgggcatttt gccgccattg aatgatattg aatttaaata 1560
   taaattcaag catttgtgaa tacatggctg gaataagagg acactagatg atattacagg 1620
   actgcagaac accctcacca cacagtccct ttggacaaat gcatttagtg gtggtagaaa 1680
   tgattcacca ggtccaatgt tgttcaccag tgcttgcttg tgaatcttaa cattttggtg 1740
   acagtttcca gatgctatca cagactctgc tagtgaaaag aactagtttc taggagcaca 1800
   ataatttgtt ttcatttgta taagtccatg aatgttcata tagccaggga ttgaagttta 1860
   ttattttcaa aggaaaacac ctttatttta ttttttttca aaatgaagat acacattaca 1920
   gccaggtgtg gtagcaggca cctgtagtct tagctactcg agaggccaaa gaaggaggat 1980
   ggcttgagcc caggagttca agaccagcct ggacagctta gtgagatccc gtctccgaag 2040
   aaaagatatg tattctaatt ggcagattgt tttttcctaa ggaaactgct ttatttttat 2100
   aaaactgcct gacaattatg aaaaaatgtt caaattcacg ttctagtgaa actgcattat 2160
   ttgttgacta gatggtgggg ttcttcgggt gtgatcatat atcataaagg atatttcaaa 2220
   tgattatgat tagttatgtc ttttaataaa aaggaaatat ttttcaactt cttctatatc 2280
   caaaattcag ggctttaaac atgattatct tgatttccca aaaacactaa aggtggtttt 2340
   attttccctt catgttttaa cttattgttg ctgaaaactc tatgtccggc tttaactatc 2400
   ttctctatat ttttatttca ttcacattaa tgagaagagt tttctcagag attaaaaaag 2460
   gtagtttttc tgtcattgtt aaatacacat tatcactgaa aaaatgtagc ttttatgatg 2520
   tatgttttaa agttaaaact ggatggaaat agccatttgg aagctttggt tatgaaacat 2580
   gtggagtgta ttaagtgcag cttgacatta tgttttattt aaatgctttt tatcgctaaa 2640
   tgacttgcag atgaaaaaaa ctaaggtgac tcgagtgttt aaatgcctgt gtacaacaat 2700
   gctttgataa aatattttaa ggtatgagtt atcagctcta tgtcaattga tatttctgtg 2760
   tagtatttat atttaaatta tatttacctt tttgcttatt ttacaaatat taagaaaata 2820
   ttctaacatt tgataatttt gaaatgattc atctttcaga aataaaagta tgaatct 2877
<210> 4
   <211> 8461
   <212> DNA
   <213> Homo sapiens
<400> 4
   gagggagcca gagagacggc agcggccccg gcctccctct ccgccgcgct tcagcctccc 60
   gctccgccgc gctccagcct cgctctccgc cgcccgcacc gccgcccgcg ccctcaccag 120
   agcagccatg gaggaggtgg tgattgccgg catgtccggg aagctgccag agtcggagaa 180
   cttgcaggag ttctgggaca acctcatcgg cggtgtggac atggtcacgg acgatgaccg 240
   tcgctggaag gcggggctct acggcctgcc ccggcggtcc ggcaagctga aggacctgtc 300
   taggtttgat gcctccttct tcggagtcca ccccaagcag gcacacacga tggaccctca 360
   gctgcggctg ctgctggaag tcacctatga agccatcgtg gacggaggca tcaacccaga 420
   ttcactccga ggaacacaca ctggcgtctg ggtgggcgtg agcggctctg agacctcgga 480
   ggccctgagc cgagaccccg agacactcgt gggctacagc atggtgggct gccagcgagc 540
   gatgatggcc aaccggctct ccttcttctt cgacttcaga gggcccagca tcgcactgga 600
   cacagcctgc tcctccagcc tgatggccct gcagaacgcc taccaggcca tccacagcgg 660
   gcagtgccct gccgccatcg tggggggcat caatgtcctg ctgaagccca acacctccgt 720
   gcagttcttg aggctgggga tgctcagccc cgagggcacc tgcaaggcct tcgacacagc 780
   ggggaatggg tactgccgct cggagggtgt ggtggccgtc ctgctgacca agaagtccct 840
   ggcccggcgg gtgtacgcca ccatcctgaa cgccggcacc aatacagatg gcttcaagga 900
   gcaaggcgtg accttcccct caggggatat ccaggagcag ctcatccgct cgttgtacca 960
   gtcggccgga gtggcccctg agtcatttga atacatcgaa gcccacggca caggcaccaa 1020
   ggtgggcgac ccccaggagc tgaatggcat cacccgagcc ctgtgcgcca cccgccagga 1080
   gccgctgctc atcggctcca ccaagtccaa catggggcac ccggagccag cctcggggct 1140
   ggcagccctg gccaaggtgc tgctgtccct ggagcacggg ctctgggccc ccaacctgca 1200
   cttccatagc cccaaccctg agatcccagc gctgttggat gggcggctgc aggtggtgga 1260
   ccagcccctg cccgtccgtg gcggcaacgt gggcatcaac tcctttggct tcgggggctc 1320
   caacgtgcac atcatcctga ggcccaacac gcagccgccc cccgcacccg ccccacatgc 1380
   caccctgccc cgtctgctgc gggccagcgg acgcacccct gaggccgtgc agaagctgct 1440
   ggagcagggc ctccggcaca gccaggacct ggctttcctg agcatgctga acgacatcgc 1500
   ggctgtcccc gccaccgcca tgcccttccg tggctacgct gtgctgggtg gtgagcgcgg 1560
   tggcccagag gtgcagcagg tgcccgctgg cgagcgcccg ctctggttca tctgctctgg 1620
   gatgggcaca cagtggcgcg ggatggggct gagcctcatg cgcctggacc gcttccgaga 1680
   ttccatccta cgctccgatg aggctgtgaa gccattcggc ctgaaggtgt cacagctgct 1740
   gctgagcaca gacgagagca cctttgatga catcgtccat tcgtttgtga gcctgactgc 1800
   catccagata ggcctcatag acctgctgag ctgcatgggg ctgaggccag atggcatcgt 1860
   cggccactcc ctgggggagg tggcctgtgg ctacgccgac ggctgcctgt cccaggagga 1920
   ggccgtcctc gctgcctact ggaggggaca gtgcatcaaa gaagcccatc tcccgccggg 1980
   cgccatggca gccgtgggct tgtcctggga ggagtgtaaa cagcgctgcc ccccggcggt 2040
   ggtgcccgcc tgccacaact ccaaggacac agtcaccatc tcgggacctc aggccccggt 2100
   gtttgagttc gtggagcagc tgaggaagga gggtgtgttt gccaaggagg tgcggaccgg 2160
   cggtatggcc ttccactcct acttcatgga ggccatcgca cccccactgc tgcaggagct 2220
   caagaaggtg atccgggagc cgaagccacg ttcagcccgc tggctcagca cctctatccc 2280
   cgaggcccag tggcacagca gcctggcacg cacgtcctcc gccgagtaca atgtcaacaa 2340
   cctggtgagc cctgtgctgt tccaggaggc cctgtggcac gtgcctgagc acgcggtggt 2400
   gctggagatc gcgccccacg ccctgctgca ggctgtcctg aagcgtggcc tgaagccgag 2460
   ctgcaccatc atccccctga tgaagaagga tcacagggac aacctggagt tcttcctggc 2520
   cggcatccgg aggctgcacc tctcaggcat cgacgccaac cccaatgcct tgttcccacc 2580
   tgtggagttc ccagctcccc gaggaactcc cctcatctcc ccactcatca agtgggacca 2640
   cagcctggcc tgggacgtgc cggccgccga ggacttcccc aacggttcag gttccccctc 2700
   agccgccatc tacaacatcg acaccagctc cgagtctcct gaccactacc tggtggacca 2760
   caccctcgac ggtcgcgtcc tcttccccgc cactggctac ctgagcatag tgtggaagac 2820
   gctggcccga cccctgggcc tgggcgtcga gcagctgcct gtggtgtttg aggatgtggt 2880
   gctgcaccag gccaccatcc tgcccaagac tgggacagtg tccctggagg tacggctcct 2940
   ggaggcctcc cgtgccttcg aggtgtcaga gaacggcaac ctggtagtga gtgggaaggt 3000
   gtaccagtgg gatgaccctg accccaggct cttcgaccac ccggaaagcc ccacccccaa 3060
   ccccacggag cccctcttcc tggcccaggc tgaagtttac aaggagctgc gtctgcgtgg 3120
   ctacgactac ggccctcatt tccagggcat cctggaggcc agcctggaag gtgactcggg 3180
   gaggctgctg tggaaggata actgggtgag cttcatggac accatgctgc agatgtccat 3240
   cctgggctcg gccaagcacg gcctgtacct gcccacccgt gtcaccgcca tccacatcga 3300
   ccctgccacc cacaggcaga agctgtacac actgcaggac aaggcccaag tggctgacgt 3360
   ggtggtgagc aggtggctga gggtcacagt ggccggaggc gtccacatct ccgggctcca 3420
   cactgagtcg gccccgcggc ggcagcagga gcagcaggtg cccatcctgg agaagttttg 3480
   cttcactccc cacacggagg aggggtgcct gtctgagcgc gctgccctgc aggaggagct 3540
   gcaactgtgc aaggggctgg tgcaggcact gcagaccaag gtgacccagc aggggctgaa 3600
   gatggtggtg cccggactgg atggggccca gatcccccgg gacccctcac agcaggaact 3660
   gccccggctg ttgtcggctg cctgcaggct tcagctcaac gggaacctgc agctggagct 3720
   ggcgcaggtg ctggcccagg agaggcccaa gctgccagag gaccctctgc tcagcggcct 3780
   cctggactcc ccggcactca aggcctgcct ggacactgcc gtggagaaca tgcccagcct 3840
   gaagatgaag gtggtggagg tgctggccgg ccacggtcac ctgtattccc gcatcccagg 3900
   cctgctcagc ccccatcccc tgctgcagct gagctacacg gccaccgacc gccaccccca 3960
   ggccctggag gctgcccagg ccgagctgca gcagcacgac gttgcccagg gccagtggga 4020
   tcccgcagac cctgccccca gcgccctggg cagcgccgac ctcctggtgt gcaactgtgc 4080
   tgtggctgcc ctcggggacc cggcctcagc tctcagcaac atggtggctg ccctgagaga 4140
   agggggcttt ctgctcctgc acacactgct ccgggggcac ccctcgggac atgtggcctt 4200
   cctcacctcc actgagccgc agtatggcca gggcatcctg agccaggacg cgtgggagag 4260
   cctcttctcc agggtgtccg tgcgcctggt gggcctgaag aagtccttct acggctccac 4320
   gctcttcctg tgccgccggc ccaccccgca ggacagcccc atcttcctgc cggtggacga 4380
   taccagcttc cgctgggtgg agtctctgaa gggcatcctg gctgacgaag actcttcccg 4440
   gcctgtgtgg ctgaaggcca tcaactgtgc cacctcgggc gtggtgggct tggtgaactg 4500
   tctccgccga gagcccggcg gaacgctccg gtgtgtgctg ctctccaacc tcagcagcac 4560
   ctcccacgtc ccggaggtgg acccgggctc cgcagaactg cagaaggtgt tgcagggaga 4620
   cctggtgatg aacgtctacc gcgacggggc ctggggggct ttccgccact tcctgctgga 4680
   ggaggacaag cctgaggagc cgacggcaca tgcctttgtg agcaccctca cccgggggga 4740
   cctgtcctcc atccgctggg tctgctcctc gctgcgccat gcccagccca cctgccctgg 4800
   cgcccagctc tgcacggtct actacgcctc cctcaacttc cgcgacatca tgctggccac 4860
   tggcaagctg tcccctgatg ccatcccagg gaagtggacc tcccaggaca gcctgctagg 4920
   tatggagttc tcgggccgag acgccagcgg caagcgtgtg atgggactgg tgcctgccaa 4980
   gggcctggcc acctctgtcc tgctgtcacc ggacttcctc tgggatgtgc cttccaactg 5040
   gacgctggag gaggcggcct cggtgcctgt cgtctacagc acggcctact acgcgctggt 5100
   ggtgcgtggg cgggtgcgcc ccggggagac gctgctcatc cactcgggct cgggcggcgt 5160
   gggccaggcc gccatcgcca tcgccctcag tctgggctgc cgcgtcttca ccaccgtggg 5220
   gtcggctgag aagcgggcgt acctccaggc caggttcccc cagctcgaca gcaccagctt 5280
   cgccaactcc cgggacacat ccttcgagca gcatgtgctg tggcacacgg gcgggaaggg 5340
   cgttgacctg gtcttgaact ccttggcgga agagaagctg caggccagcg tgaggtgctt 5400
   ggctacgcac ggtcgcttcc tggaaattgg caaattcgac ctttctcaga accacccgct 5460
   cggcatggct atcttcctga agaacgtgac attccacggg gtcctactgg atgcgttctt 5520
   caacgagagc agtgctgact ggcgggaggt gtgggcgctt gtgcaggccg gcatccggga 5580
   tggggtggta cggcccctca agtgcacggt gttccatggg gcccaggtgg aggacgcctt 5640
   ccgctacatg gcccaaggga agcacattgg caaagtcgtc gtgcaggtgc ttgcggagga 5700
   gccggaggca gtgctgaagg gggccaaacc caagctgatg tcggccatct ccaagacctt 5760
   ctgcccggcc cacaagagct acatcatcgc tggtggtctg ggtggcttcg gcctggagtt 5820
   ggcgcagtgg ctgatacagc gtggggtgca gaagctcgtg ttgacttctc gctccgggat 5880
   ccggacaggc taccaggcca agcaggtccg ccggtggagg cgccagggcg tacaggtgca 5940
   ggtgtccacc agcaacatca gctcactgga gggggcccgg ggcctcattg ccgaggcggc 6000
   gcagcttggg cccgtgggcg gcgtcttcaa cctggccgtg gtcttgagag atggcttgct 6060
   ggagaaccag accccagagt tcttccagga cgtctgcaag cccaagtaca gcggcaccct 6120
   gaacctggac agggtgaccc gagaggcgtg ccctgagctg gactactttg tggtcttctc 6180
   ctctgtgagc tgcgggcgtg gcaatgcggg acagagcaac tacggctttg ccaattccgc 6240
   catggagcgt atctgtgaga aacgccggca cgaaggcctc ccaggcctgg ccgtgcagtg 6300
   gggcgccatc ggcgacgtgg gcattttggt ggagacgatg agcaccaacg acacgatcgt 6360
   cagtggcacg ctgccccagc gcatggcgtc ctgcctggag gtgctggacc tcttcctgaa 6420
   ccagccccac atggtcctga gcagctttgt gctggctgag aaggctgcgg cctataggga 6480
   cagggacagc cagcgggacc tggtggaggc cgtggcacac atcctgggca tccgcgactt 6540
   ggctgctgtc aacctggaca gctcactggc ggacctgggc ctggactcgc tcatgagcgt 6600
   ggaggtgcgc cagacgctgg agcgtgagct caacctggtg ctgtccgtgc gcgaggtgcg 6660
   gcaactcacg ctccggaaac tgcaggagct gtcctcaaag gcggatgagg ccagcgagct 6720
   ggcatgcccc acgcccaagg aggatggtct ggcccagcag cagactcagc tgaacctgcg 6780
   ctccctgctg gtgaacccgg agggccccac cctgatgcgg ctcaactccg tgcagagctc 6840
   ggagcggccc ctgttcctgg tgcacccaat cgagggctcc accaccgtgt tccacagcct 6900
   ggcctcccgg ctcagcatcc ccacctatgg cctgcagtgc acccgagctg cgccccttga 6960
   cagcatccac agcctggctg cctactacat cgactgcatc aggcaggtgc agcccgaggg 7020
   cccctaccgc gtggccggct actcctacgg ggcctgcgtg gcctttgaaa tgtgctccca 7080
   gctgcaggcc cagcagagcc cagcccccac ccacaacagc ctcttcctgt tcgacggctc 7140
   gcccacctac gtactggcct acacccagag ctaccgggca aagctgaccc caggctgtga 7200
   ggctgaggct gagacggagg ccatatgctt cttcgtgcag cagttcacgg acatggagca 7260
   caacagggtg ctggaggcgc tgctgccgct gaagggccta gaggagcgtg tggcagccgc 7320
   cgtggacctg atcatcaaga gccaccaggg cctggaccgc caggagctga gctttgcggc 7380
   ccggtccttc tactacaagc tgcgtgccgc tgagcagtac acacccaagg ccaagtacca 7440
   tggcaacgtg atgctactgc gcgccaagac gggtggcgcc tacggcgagg acctgggcgc 7500
   ggactacaac ctctcccagg tatgcgacgg gaaagtatcc gtccacgtca tcgagggtga 7560
   ccaccgcacg ctgctggagg gcagcggcct ggagtccatc atcagcatca tccacagctc 7620
   cctggctgag ccacgcgtga gcgtgcggga gggctaggcc cgtgcccccg cctgccaccg 7680
   gaggtcactc caccatcccc accccacccc accccacccc cgccatgcaa cgggattgaa 7740
   gggtcctgcc ggtgggaccc tgtccggccc agtgccactg ccccccgagg ctgctagacg 7800
   taggtgttag gcatgtccca cccacccgcc gcctcccacg gcacctcggg gacaccagag 7860
   ctgccgactt ggagactcct ggtctgtgaa gagccggtgg tgcccgtgcc cgcaggaact 7920
   gggctgggcc tcgtgcgccc gtggggtctg cgcttggtct ttctgtgctt ggatttgcat 7980
   atttattgca ttgctggtag agacccccag gcctgtccac cctgccaaga ctcctcaggc 8040
   agcgtgtggg tcccgcactc tgcccccatt tccccgatgt cccctgcggg cgcgggcagc 8100
   cacccaagcc tgctggctgc ggccccctct cggccaggca ttggctcagc ccgctgagtg 8160
   gggggtcgtg ggccagtccc cgaggagctg ggcccctgca caggcacaca gggcccggcc 8220
   acacccagcg gccccccgca cagccacccg tggggtgctg cccttatgcc cggcgccggg 8280
   caccaactcc atgtttggtg tttgtctgtg tttgtttttc aagaaatgat tcaaattgct 8340
   gcttggattt tgaaatttac tgtaactgtc agtgtacacg tctggacccc gtttcatttt 8400
   tacaccaatt tggtaaaaat gctgctctca gcctcccaca attaaaccgc atgtgatctc 8460
   c 8461
<210> 5
   <211> 1444
   <212> DNA
   <213> Homo sapiens
<400> 5
   acggtcaccc gttgccagct ctagccttta aattcccggc tcggggacct ccacgcaccg 60
   cggctagcgc cgacaaccag ctagcgtgca aggcgccgcg gctcagcgcg taccggcggg 120
   cttcgaaacc gcagtcctcc ggcgaccccg aactccgctc cggagcctca gccccctgga 180
   aagtgatccc ggcatccgag agccaagatg ccggcccact tgctgcagga cgatatctct 240
   agctcctata ccaccaccac caccattaca gcgcctccct ccagggtcct gcagaatgga 300
   ggagataagt tggagacgat gcccctctac ttggaagacg acattcgccc tgatataaaa 360
   gatgatatat atgaccccac ctacaaggat aaggaaggcc caagccccaa ggttgaatat 420
   gtctggagaa acatcatcct tatgtctctg ctacacttgg gagccctgta tgggatcact 480
   ttgattccta cctgcaagtt ctacacctgg ctttgggggg tattctacta ttttgtcagt 540
   gccctgggca taacagcagg agctcatcgt ctgtggagcc accgctctta caaagctcgg 600
   ctgcccctac ggctctttct gatcattgcc aacacaatgg cattccagaa tgatgtctat 660
   gaatgggctc gtgaccaccg tgcccaccac aagttttcag aaacacatgc tgatcctcat 720
   aattcccgac gtggcttttt cttctctcac gtgggttggc tgcttgtgcg caaacaccca 780
   gctgtcaaag agaaggggag tacgctagac ttgtctgacc tagaagctga gaaactggtg 840
   atgttccaga ggaggtacta caaacctggc ttgctgatga tgtgcttcat cctgcccacg 900
   cttgtgccct ggtatttctg gggtgaaact tttcaaaaca gtgtgttcgt tgccactttc 960
   ttgcgatatg ctgtggtgct taatgccacc tggctggtga acagtgctgc ccacctcttc 1020
   ggatatcgtc cttatgacaa gaacattagc ccccgggaga atatcctggt ttcacttgga 1080
   gctgtgggtg agggcttcca caactaccac cactcctttc cctatgacta ctctgccagt 1140
   gagtaccgct ggcacatcaa cttcaccaca ttcttcattg attgcatggc cgccctcggt 1200
   ctggcctatg accggaagaa agtctccaag gccgccatct tggccaggat taaaagaacc 1260
   ggagatggaa actacaagag tggctgagtt tggggtccct caggttcctt tttcaaaaac 1320
   cagccaggca gaggttttaa tgtctgttta ttaactactg aataatgcta ccaggatgct 1380
   aaagatgatg atgttaaccc attccagtac agtattcttt taaaattcaa aagtattgaa 1440
   agcc 1444
<210> 6
   <211> 4154
   <212> DNA
   <213> Homo sapiens
<400> 6
   taacgaggaa cttttcgccg gcgccgggcc gcctctgagg ccagggcagg acacgaacgc 60
   gcggagcggc ggcggcgact gagagccggg gccgcggcgg cgctccctag gaagggccgt 120
   acgaggcggc gggcccggcg ggcctcccgg aggaggcggc tgcgccatgg acgagccacc 180
   cttcagcgag gcggctttgg agcaggcgct gggcgagccg tgcgatctgg acgcggcgct 240
   gctgaccgac atcgaagaca tgcttcagct tatcaacaac caagacagtg acttccctgg 300
   cctatttgac ccaccctatg ctgggagtgg ggcagggggc acagaccctg ccagccccga 360
   taccagctcc ccaggcagct tgtctccacc tcctgccaca ttgagctcct ctcttgaagc 420
   cttcctgagc gggccgcagg cagcgccctc acccctgtcc cctccccagc ctgcacccac 480
   tccattgaag atgtacccgt ccatgcccgc tttctcccct gggcctggta tcaaggaaga 540
   gtcagtgcca ctgagcatcc tgcagacccc caccccacag cccctgccag gggccctcct 600
   gccacagagc ttcccagccc cagccccacc gcagttcagc tccacccctg tgttaggcta 660
   ccccagccct ccgggaggct tctctacagg aagccctccc gggaacaccc agcagccgct 720
   gcctggcctg ccactggctt ccccgccagg ggtcccgccc gtctccttgc acacccaggt 780
   ccagagtgtg gtcccccagc agctactgac agtcacagct gcccccacgg cagcccctgt 840
   aacgaccact gtgacctcgc agatccagca ggtcccggtc ctgctgcagc cccacttcat 900
   caaggcagac tcgctgcttc tgacagccat gaagacagac ggagccactg tgaaggcggc 960
   aggtctcagt cccctggtct ctggcaccac tgtgcagaca gggcctttgc cgaccctggt 1020
   gagtggcgga accatcttgg caacagtccc actggtcgta gatgcggaga agctgcctat    1080
   caaccggctc gcagctggca gcaaggcccc ggcctctgcc cagagccgtg gagagaagcg    1140
   cacagcccac aacgccattg agaagcgcta ccgctcctcc atcaatgaca aaatcattga    1200
   gctcaaggat ctggtggtgg gcactgaggc aaagctgaat aaatctgctg tcttgcgcaa    1260
   ggccatcgac tacattcgct ttctgcaaca cagcaaccag aaactcaagc aggagaacct    1320
   aagtctgcgc actgctgtcc acaaaagcaa atctctgaag gatctggtgt cggcctgtgg    1380
   cagtggaggg aacacagacg tgctcatgga gggcgtgaag actgaggtgg aggacacact    1440
   gaccccaccc ccctcggatg ctggctcacc tttccagagc agccccttgt cccttggcag    1500
   caggggcagt ggcagcggtg gcagtggcag tgactcggag cctgacagcc cagtctttga    1560
   ggacagcaag gcaaagccag agcagcggcc gtctctgcac agccggggca tgctggaccg    1620
   ctcccgcctg gccctgtgca cgctcgtctt cctctgcctg tcctgcaacc ccttggcctc    1680
   cttgctgggg gcccgggggc ttcccagccc ctcagatacc accagcgtct accatagccc    1740
   tgggcgcaac gtgctgggca ccgagagcag agatggccct ggctgggccc agtggctgct    1800
   gcccccagtg gtctggctgc tcaatgggct gttggtgctc gtctccttgg tgcttctctt    1860
   tgtctacggt gagccagtca cacggcccca ctcaggcccc gccgtgtact tctggaggca    1920
   tcgcaagcag gctgacctgg acctggcccg gggagacttt gcccaggctg cccagcagct    1980
   gtggctggcc ctgcgggcac tgggccggcc cctgcccacc tcccacctgg acctggcttg    2040
   tagcctcctc tggaacctca tccgtcacct gctgcagcgt ctctgggtgg gccgctggct    2100
   ggcaggccgg gcagggggcc tgcagcagga ctgtgctctg cgagtggatg ctagcgccag    2160
   cgcccgagac gcagccctgg tctaccataa gctgcaccag ctgcacacca tggggaagca    2220
   cacaggcggg cacctcactg ccaccaacct ggcgctgagt gccctgaacc tggcagagtg    2280
   tgcaggggat gccgtgtctg tggcgacgct ggccgagatc tatgtggcgg ctgcattgag    2340
   agtgaagacc agtctcccac gggccttgca ttttctgaca cgcttcttcc tgagcagtgc    2400
   ccgccaggcc tgcctggcac agagtggctc agtgcctcct gccatgcagt ggctctgcca    2460
   ccccgtgggc caccgtttct tcgtggatgg ggactggtcc gtgctcagta ccccatggga    2520
   gagcctgtac agcttggccg ggaacccagt ggaccccctg gcccaggtga ctcagctatt    2580
   ccgggaacat ctcttagagc gagcactgaa ctgtgtgacc cagcccaacc ccagccctgg    2640
   gtcagctgat ggggacaagg aattctcgga tgccctcggg tacctgcagc tgctgaacag    2700
   ctgttctgat gctgcggggg ctcctgccta cagcttctcc atcagttcca gcatggccac    2760
   caccaccggc gtagacccgg tggccaagtg gtgggcctct ctgacagctg tggtgatcca    2820
   ctggctgcgg cgggatgagg aggcggctga gcggctgtgc ccgctggtgg agcacctgcc    2880
   ccgggtgctg caggagtctg agagacccct gcccagggca gctctgcact ccttcaaggc    2940
   tgcccgggcc ctgctgggct gtgccaaggc agagtctggt ccagccagcc tgaccatctg    3000
   tgagaaggcc agtgggtacc tgcaggacag cctggctacc acaccagcca gcagctccat    3060
   tgacaaggcc gtgcagctgt tcctgtgtga cctgcttctt gtggtgcgca ccagcctgtg 3120
   gcggcagcag cagcccccgg ccccggcccc agcagcccag ggcaccagca gcaggcccca 3180
   ggcttccgcc cttgagctgc gtggcttcca acgggacctg agcagcctga ggcggctggc 3240
   acagagcttc cggcccgcca tgcggagggt gttcctacat gaggccacgg cccggctgat 3300
   ggcgggggcc agccccacac ggacacacca gctcctcgac cgcagtctga ggcggcgggc 3360
   aggccccggt ggcaaaggag gcgcggtggc ggagctggag ccgcggccca cgcggcggga 3420
   gcacgcggag gccttgctgc tggcctcctg ctacctgccc cccggcttcc tgtcggcgcc 3480
   cgggcagcgc gtgggcatgc tggctgaggc ggcgcgcaca ctcgagaagc ttggcgatcg 3540
   ccggctgctg cacgactgtc agcagatgct catgcgcctg ggcggtggga ccactgtcac 3600
   ttccagctag accccgtgtc cccggcctca gcacccctgt ctctagccac tttggtcccg 3660
   tgcagcttct gtcctgcgtc gaagctttga aggccgaagg cagtgcaaga gactctggcc 3720
   tccacagttc gacctgcggc tgctgtgtgc cttcgcggtg gaaggcccga ggggcgcgat 3780
   cttgacccta agaccggcgg ccatgatggt gctgacctct ggtggccgat cggggcactg 3840
   caggggccga gccattttgg ggggcccccc tccttgctct gcaggcacct tagtggcttt 3900
   tttcctcctg tgtacaggga agagaggggt acatttccct gtgctgacgg aagccaactt 3960
   ggctttcccg gactgcaagc agggctctgc cccagaggcc tctctctccg tcgtgggaga 4020
   gagacgtgta catagtgtag gtcagcgtgc ttagcctcct gacctgaggc tcctgtgcta 4080
   ctttgccttt tgcaaacttt attttcatag attgagaagt tttgtacaga gaattaaaaa 4140
   tgaaattatt tata 4154
<210> 7
   <211> 10412
   <212> DNA
   <213> Homo sapiens
<400> 7
   gtaattgcga gcgagagtga gtggggccgg gacccgcaga gccgagccga cccttctctc 60
   ccgggctgcg gcagggcagg gcggggagct ccgcgcacca acagagccgg ttctcagggc 120
   gctttgctcc ttgttttttc cccggttctg ttttctcccc ttctccggaa ggcttgtcaa 180
   ggggtaggag aaagagacgc aaacacaaaa gtggaaaaca gttaatgacc agccacggcg 240
   tccctgctgt gagctctggc cgctgccttc cagggctccc gagccacacg ctgggggtgc 300
   tggctgaggg aacatggctt gttggcctca gctgaggttg ctgctgtgga agaacctcac 360
   tttcagaaga agacaaacat gtcagctgct gctggaagtg gcctggcctc tatttatctt 420
   cctgatcctg atctctgttc ggctgagcta cccaccctat gaacaacatg aatgccattt 480
   tccaaataaa gccatgccct ctgcaggaac acttccttgg gttcagggga ttatctgtaa 540
   tgccaacaac ccctgtttcc gttacccgac tcctggggag gctcccggag ttgttggaaa 600
   ctttaacaaa tccattgtgg ctcgcctgtt ctcagatgct cggaggcttc ttttatacag 660
   ccagaaagac accagcatga aggacatgcg caaagttctg agaacattac agcagatcaa 720
   gaaatccagc tcaaacttga agcttcaaga tttcctggtg gacaatgaaa ccttctctgg 780
   gttcctgtat cacaacctct ctctcccaaa gtctactgtg gacaagatgc tgagggctga 840
   tgtcattctc cacaaggtat ttttgcaagg ctaccagtta catttgacaa gtctgtgcaa 900
   tggatcaaaa tcagaagaga tgattcaact tggtgaccaa gaagtttctg agctttgtgg 960
   cctaccaagg gagaaactgg ctgcagcaga gcgagtactt cgttccaaca tggacatcct 1020
   gaagccaatc ctgagaacac taaactctac atctcccttc ccgagcaagg agctggctga 1080
   agccacaaaa acattgctgc atagtcttgg gactctggcc caggagctgt tcagcatgag 1140
   aagctggagt gacatgcgac aggaggtgat gtttctgacc aatgtgaaca gctccagctc 1200
   ctccacccaa atctaccagg ctgtgtctcg tattgtctgc gggcatcccg agggaggggg 1260
   gctgaagatc aagtctctca actggtatga ggacaacaac tacaaagccc tctttggagg 1320
   caatggcact gaggaagatg ctgaaacctt ctatgacaac tctacaactc cttactgcaa 1380
   tgatttgatg aagaatttgg agtctagtcc tctttcccgc attatctgga aagctctgaa 1440
   gccgctgctc gttgggaaga tcctgtatac acctgacact ccagccacaa ggcaggtcat 1500
   ggctgaggtg aacaagacct tccaggaact ggctgtgttc catgatctgg aaggcatgtg 1560
   ggaggaactc agccccaaga tctggacctt catggagaac agccaagaaa tggaccttgt 1620
   ccggatgctg ttggacagca gggacaatga ccacttttgg gaacagcagt tggatggctt 1680
   agattggaca gcccaagaca tcgtggcgtt tttggccaag cacccagagg atgtccagtc 1740
   cagtaatggt tctgtgtaca cctggagaga agctttcaac gagactaacc aggcaatccg 1800
   gaccatatct cgcttcatgg agtgtgtcaa cctgaacaag ctagaaccca tagcaacaga 1860
   agtctggctc atcaacaagt ccatggagct gctggatgag aggaagttct gggctggtat 1920
   tgtgttcact ggaattactc caggcagcat tgagctgccc catcatgtca agtacaagat 1980
   ccgaatggac attgacaatg tggagaggac aaataaaatc aaggatgggt actgggaccc 2040
   tggtcctcga gctgacccct ttgaggacat gcggtacgtc tgggggggct tcgcctactt 2100
   gcaggatgtg gtggagcagg caatcatcag ggtgctgacg ggcaccgaga agaaaactgg 2160
   tgtctatatg caacagatgc cctatccctg ttacgttgat gacatctttc tgcgggtgat 2220
   gagccggtca atgcccctct tcatgacgct ggcctggatt tactcagtgg ctgtgatcat 2280
   caagggcatc gtgtatgaga aggaggcacg gctgaaagag accatgcgga tcatgggcct 2340
   ggacaacagc atcctctggt ttagctggtt cattagtagc ctcattcctc ttcttgtgag 2400
   cgctggcctg ctagtggtca tcctgaagtt aggaaacctg ctgccctaca gtgatcccag 2460
   cgtggtgttt gtcttcctgt ccgtgtttgc tgtggtgaca atcctgcagt gcttcctgat 2520
   tagcacactc ttctccagag ccaacctggc agcagcctgt gggggcatca tctacttcac 2580
   gctgtacctg ccctacgtcc tgtgtgtggc atggcaggac tacgtgggct tcacactcaa 2640
   gatcttcgct agcctgctgt ctcctgtggc ttttgggttt ggctgtgagt actttgccct 2700
   ttttgaggag cagggcattg gagtgcagtg ggacaacctg tttgagagtc ctgtggagga 2760
   agatggcttc aatctcacca cttcggtctc catgatgctg tttgacacct tcctctatgg 2820
   ggtgatgacc tggtacattg aggctgtctt tccaggccag tacggaattc ccaggccctg 2880
   gtattttcct tgcaccaagt cctactggtt tggcgaggaa agtgatgaga agagccaccc 2940
   tggttccaac cagaagagaa tatcagaaat ctgcatggag gaggaaccca cccacttgaa 3000
   gctgggcgtg tccattcaga acctggtaaa agtctaccga gatgggatga aggtggctgt 3060
   cgatggcctg gcactgaatt tttatgaggg ccagatcacc tccttcctgg gccacaatgg 3120
   agcggggaag acgaccacca tgtcaatcct gaccgggttg ttccccccga cctcgggcac 3180
   cgcctacatc ctgggaaaag acattcgctc tgagatgagc accatccggc agaacctggg 3240
   ggtctgtccc cagcataacg tgctgtttga catgctgact gtcgaagaac acatctggtt 3300
   ctatgcccgc ttgaaagggc tctctgagaa gcacgtgaag gcggagatgg agcagatggc 3360
   cctggatgtt ggtttgccat caagcaagct gaaaagcaaa acaagccagc tgtcaggtgg 3420
   aatgcagaga aagctatctg tggccttggc ctttgtcggg ggatctaagg ttgtcattct 3480
   ggatgaaccc acagctggtg tggaccctta ctcccgcagg ggaatatggg agctgctgct 3540
   gaaataccga caaggccgca ccattattct ctctacacac cacatggatg aagcggacgt 3600
   cctgggggac aggattgcca tcatctccca tgggaagctg tgctgtgtgg gctcctccct 3660
   gtttctgaag aaccagctgg gaacaggcta ctacctgacc ttggtcaaga aagatgtgga 3720
   atcctccctc agttcctgca gaaacagtag tagcactgtg tcatacctga aaaaggagga 3780
   cagtgtttct cagagcagtt ctgatgctgg cctgggcagc gaccatgaga gtgacacgct 3840
   gaccatcgat gtctctgcta tctccaacct catcaggaag catgtgtctg aagcccggct 3900
   ggtggaagac atagggcatg agctgaccta tgtgctgcca tatgaagctg ctaaggaggg 3960
   agcctttgtg gaactctttc atgagattga tgaccggctc tcagacctgg gcatttctag 4020
   ttatggcatc tcagagacga ccctggaaga aatattcctc aaggtggccg aagagagtgg 4080
   ggtggatgct gagacctcag atggtacctt gccagcaaga cgaaacaggc gggccttcgg 4140
   ggacaagcag agctgtcttc gcccgttcac tgaagatgat gctgctgatc caaatgattc 4200
   tgacatagac ccagaatcca gagagacaga cttgctcagt gggatggatg gcaaagggtc 4260
   ctaccaggtg aaaggctgga aacttacaca gcaacagttt gtggcccttt tgtggaagag 4320
   actgctaatt gccagacgga gtcggaaagg attttttgct cagattgtct tgccagctgt 4380
   gtttgtctgc attgcccttg tgttcagcct gatcgtgcca ccctttggca agtaccccag 4440
   cctggaactt cagccctgga tgtacaacga acagtacaca tttgtcagca atgatgctcc 4500
   tgaggacacg ggaaccctgg aactcttaaa cgccctcacc aaagaccctg gcttcgggac 4560
   ccgctgtatg gaaggaaacc caatcccaga cacgccctgc caggcagggg aggaagagtg 4620
   gaccactgcc ccagttcccc agaccatcat ggacctcttc cagaatggga actggacaat 4680
   gcagaaccct tcacctgcat gccagtgtag cagcgacaaa atcaagaaga tgctgcctgt 4740
   gtgtccccca ggggcagggg ggctgcctcc tccacaaaga aaacaaaaca ctgcagatat 4800
   ccttcaggac ctgacaggaa gaaacatttc ggattatctg gtgaagacgt atgtgcagat 4860
   catagccaaa agcttaaaga acaagatctg ggtgaatgag tttaggtatg gcggcttttc 4920
   cctgggtgtc agtaatactc aagcacttcc tccgagtcaa gaagttaatg atgccatcaa 4980
   acaaatgaag aaacacctaa agctggccaa ggacagttct gcagatcgat ttctcaacag 5040
   cttgggaaga tttatgacag gactggacac caaaaataat gtcaaggtgt ggttcaataa 5100
   caagggctgg catgcaatca gctctttcct gaatgtcatc aacaatgcca ttctccgggc 5160
   caacctgcaa aagggagaga accctagcca ttatggaatt actgctttca atcatcccct 5220
   gaatctcacc aagcagcagc tctcagaggt ggctctgatg accacatcag tggatgtcct 5280
   tgtgtccatc tgtgtcatct ttgcaatgtc cttcgtccca gccagctttg tcgtattcct 5340
   gatccaggag cgggtcagca aagcaaaaca cctgcagttc atcagtggag tgaagcctgt 5400
   catctactgg ctctctaatt ttgtctggga tatgtgcaat tacgttgtcc ctgccacact 5460
   ggtcattatc atcttcatct gcttccagca gaagtcctat gtgtcctcca ccaatctgcc 5520
   tgtgctagcc cttctacttt tgctgtatgg gtggtcaatc acacctctca tgtacccagc 5580
   ctcctttgtg ttcaagatcc ccagcacagc ctatgtggtg ctcaccagcg tgaacctctt 5640
   cattggcatt aatggcagcg tggccacctt tgtgctggag ctgttcaccg acaataagct 5700
   gaataatatc aatgatatcc tgaagtccgt gttcttgatc ttcccacatt tttgcctggg 5760
   acgagggctc atcgacatgg tgaaaaacca ggcaatggct gatgccctgg aaaggtttgg 5820
   ggagaatcgc tttgtgtcac cattatcttg ggacttggtg ggacgaaacc tcttcgccat 5880
   ggccgtggaa ggggtggtgt tcttcctcat tactgttctg atccagtaca gattcttcat 5940
   caggcccaga cctgtaaatg caaagctatc tcctctgaat gatgaagatg aagatgtgag 6000
   gcgggaaaga cagagaattc ttgatggtgg aggccagaat gacatcttag aaatcaagga 6060
   gttgacgaag atatatagaa ggaagcggaa gcctgctgtt gacaggattt gcgtgggcat 6120
   tcctcctggt gagtgctttg ggctcctggg agttaatggg gctggaaaat catcaacttt 6180
   caagatgtta acaggagata ccactgttac cagaggagat gctttcctta acaaaaatag 6240
   tatcttatca aacatccatg aagtacatca gaacatgggc tactgccctc agtttgatgc 6300
   catcacagag ctgttgactg ggagagaaca cgtggagttc tttgcccttt tgagaggagt 6360
   cccagagaaa gaagttggca aggttggtga gtgggcgatt cggaaactgg gcctcgtgaa 6420
   gtatggagaa aaatatgctg gtaactatag tggaggcaac aaacgcaagc tctctacagc 6480
   catggctttg atcggcgggc ctcctgtggt gtttctggat gaacccacca caggcatgga 6540
   tcccaaagcc cggcggttct tgtggaattg tgccctaagt gttgtcaagg aggggagatc 6600
   agtagtgctt acatctcata gtatggaaga atgtgaagct ctttgcacta ggatggcaat 6660
   catggtcaat ggaaggttca ggtgccttgg cagtgtccag catctaaaaa ataggtttgg 6720
   agatggttat acaatagttg tacgaatagc agggtccaac ccggacctga agcctgtcca 6780
   ggatttcttt ggacttgcat ttcctggaag tgttctaaaa gagaaacacc ggaacatgct 6840
   acaataccag cttccatctt cattatcttc tctggccagg atattcagca tcctctccca 6900
   gagcaaaaag cgactccaca tagaagacta ctctgtttct cagacaacac ttgaccaagt 6960
   atttgtgaac tttgccaagg accaaagtga tgatgaccac ttaaaagacc tctcattaca 7020
   caaaaaccag acagtagtgg acgttgcagt tctcacatct tttctacagg atgagaaagt 7080
   gaaagaaagc tatgtatgaa gaatcctgtt catacggggt ggctgaaagt aaagaggaac 7140
   tagactttcc tttgcaccat gtgaagtgtt gtggagaaaa gagccagaag ttgatgtggg 7200
   aagaagtaaa ctggatactg tactgatact attcaatgca atgcaattca atgcaatgaa 7260
   aacaaaattc cattacaggg gcagtgcctt tgtagcctat gtcttgtatg gctctcaagt 7320
   gaaagacttg aatttagttt tttacctata cctatgtgaa actctattat ggaacccaat 7380
   ggacatatgg gtttgaactc acactttttt tttttttttt gttcctgtgt attctcattg 7440
   gggttgcaac aataattcat caagtaatca tggccagcga ttattgatca aaatcaaaag 7500
   gtaatgcaca tcctcattca ctaagccatg ccatgcccag gagactggtt tcccggtgac 7560
   acatccattg ctggcaatga gtgtgccaga gttattagtg ccaagttttt cagaaagttt 7620
   gaagcaccat ggtgtgtcat gctcactttt gtgaaagctg ctctgctcag agtctatcaa 7680
   cattgaatat cagttgacag aatggtgcca tgcgtggcta acatcctgct ttgattccct 7740
   ctgataagct gttctggtgg cagtaacatg caacaaaaat gtgggtgtct ccaggcacgg 7800
   gaaacttggt tccattgtta tattgtccta tgcttcgagc catgggtcta cagggtcatc 7860
   cttatgagac tcttaaatat acttagatcc tggtaagagg caaagaatca acagccaaac 7920
   tgctggggct gcaagctgct gaagccaggg catgggatta aagagattgt gcgttcaaac 7980
   ctagggaagc ctgtgcccat ttgtcctgac tgtctgctaa catggtacac tgcatctcaa 8040
   gatgtttatc tgacacaagt gtattatttc tggctttttg aattaatcta gaaaatgaaa 8100
   agatggagtt gtattttgac aaaaatgttt gtacttttta atgttatttg gaattttaag 8160
   ttctatcagt gacttctgaa tccttagaat ggcctctttg tagaaccctg tggtatagag 8220
   gagtatggcc actgccccac tatttttatt ttcttatgta agtttgcata tcagtcatga 8280
   ctagtgccta gaaagcaatg tgatggtcag gatctcatga cattatattt gagtttcttt 8340
   cagatcattt aggatactct taatctcact tcatcaatca aatatttttt gagtgtatgc 8400
   tgtagctgaa agagtatgta cgtacgtata agactagaga gatattaagt ctcagtacac 8460
   ttcctgtgcc atgttattca gctcactggt ttacaaatat aggttgtctt gtggttgtag 8520
   gagcccactg taacaatact gggcagcctt tttttttttt tttttaattg caacaatgca 8580
   aaagccaaga aagtataagg gtcacaagtc taaacaatga attcttcaac agggaaaaca 8640
   gctagcttga aaacttgctg aaaaacacaa cttgtgttta tggcatttag taccttcaaa 8700
   taattggctt tgcagatatt ggatacccca ttaaatctga cagtctcaaa tttttcatct 8760
   cttcaatcac tagtcaagaa aaatataaaa acaacaaata cttccatatg gagcattttt 8820
   cagagttttc taacccagtc ttatttttct agtcagtaaa catttgtaaa aatactgttt 8880
   cactaatact tactgttaac tgtcttgaga gaaaagaaaa atatgagaga actattgttt 8940
   ggggaagttc aagtgatctt tcaatatcat tactaacttc ttccactttt tccagaattt 9000
   gaatattaac gctaaaggtg taagacttca gatttcaaat taatctttct atatttttta 9060
   aatttacaga atattatata acccactgct gaaaaagaaa aaaatgattg ttttagaagt 9120
   taaagtcaat attgatttta aatataagta atgaaggcat atttccaata actagtgata 9180
   tggcatcgtt gcattttaca gtatcttcaa aaatacagaa tttatagaat aatttctcct 9240
   catttaatat ttttcaaaat caaagttatg gtttcctcat tttactaaaa tcgtattcta 9300
   attcttcatt atagtaaatc tatgagcaac tccttacttc ggttcctctg atttcaaggc 9360
   catattttaa aaaatcaaaa ggcactgtga actattttga agaaaacaca acattttaat 9420
   acagattgaa aggacctctt ctgaagctag aaacaatcta tagttataca tcttcattaa 9480
   tactgtgtta ccttttaaaa tagtaatttt ttacattttc ctgtgtaaac ctaattgtgg 9540
   tagaaatttt taccaactct atactcaatc aagcaaaatt tctgtatatt ccctgtggaa 9600
   tgtacctatg tgagtttcag aaattctcaa aatacgtgtt caaaaatttc tgcttttgca 9660
   tctttgggac acctcagaaa acttattaac aactgtgaat atgagaaata cagaagaaaa 9720
   taataagccc tctatacata aatgcccagc acaattcatt gttaaaaaac aaccaaacct 9780
   cacactactg tatttcatta tctgtactga aagcaaatgc tttgtgacta ttaaatgttg 9840
   cacatcattc attcactgta tagtaatcat tgactaaagc catttgtctg tgttttcttc 9900
   ttgtggttgt atatatcagg taaaatattt tccaaagagc catgtgtcat gtaatactga 9960
   accactttga tattgagaca ttaatttgta cccttgttat tatctactag taataatgta 10020
   atactgtaga aatattgctc taattctttt caaaattgtt gcatccccct tagaatgttt 10080
   ctatttccat aaggatttag gtatgctatt atcccttctt ataccctaag atgaagctgt 10140
   ttttgtgctc tttgttcatc attggccctc attccaagca ctttacgctg tctgtaatgg 10200
   gatctatttt tgcactggaa tatctgagaa ttgcaaaact agacaaaagt ttcacaacag 10260
   atttctaagt taaatcattt tcattaaaag gaaaaaagaa aaaaaatttt gtatgtcaat 10320
   aactttatat gaagtattaa aatgcatatt tctatgttgt aatataatga gtcacaaaat 10380
   aaagctgtga cagttctgtt ggtctacaga aa 10412
<210> 8
   <211> 3473
   <212> DNA
   <213> Homo sapiens
<400> 8
   ttctttccaa gggtctctgg gtgaggcccg tgaccttccc aagcctctcc ctgtcttgtg 60
   aaacctgggc gtgatatacc tcccttttag ggctgctgcg atcatttagg cagattaaac 120
   ctcataagtg gtttcccata caagaaagat gctagcagtg caacagacag aacacttacc 180
   tgcctgccct cccgccagga ggtggtcttc caacttttgc ccggagtcta cagagggtgg 240
   gccctctctg ctggggctcc gggacatggt caggagaggt tggtctgtct gtaccgccat 300
   tctcttggcc agactgtggt gtctggtccc tactcacacc ttcctgtcag agtatccaga 360
   ggccgcagag tatccacacc ctggctgggt gtactggcta cagatggctg tggctccagg 420
   tcacctgcgt gcctgggtga tgagaaataa tgtcacaaca aatatcccat ctgcattctc 480
   tgggacactg acccatgaag agaaagcagt tctcacagtt tttacaggca cagccacagc 540
   cgtgcatgta caggtggcag ctttagcttc tgctaaactg gagagctcag tgtttgtgac 600
   agactgcgtg tcctgcaaaa tcgaaaatgt ctgtgattca gctcttcagg gaaaaagggt 660
   gccgatgtct ggcctacagg gctcaagcat tgtcatcatg cccccatcca accgtccact 720
   cgccagtgcg gcatcctgca cgtggtcagt ccaagtccag ggagggcccc atcacctggg 780
   ggtggtcgct atcagtggca aagtcttgtc agcagctcat ggggcaggaa gggcctatgg 840
   ttgggggttt cctggcgatc ccatggagga aggatacaag accctcctga aaggaatttc 900
   cgggaagttc aatagtggtg agttggtggc cattatgggt ccttccgggg ccgggaagtc 960
   cacgctgatg aacatcctgg ctggatacag ggagacgggc atgaaggggg ccgtcctcat 1020
   caacggcctg ccccgggacc tgcgctgctt ccggaaggtg tcctgctaca tcatgcagga 1080
   tgacatgctg ctgccgcatc tcactgtgca ggaggccatg atggtgtcgg cacatctgaa 1140
   gcttcaggag aaggatgaag gcagaaggga aatggtcaag gagatactga cagcgctggg 1200
   cttgctgtct tgcgccaaca cgcggaccgg gagcctgtca ggtggtcagc gcaagcgcct 1260
   ggccatcgcg ctggagctgg tgaacaaccc tccagtcatg ttcttcgatg agcccaccag 1320
   cggcctggac agcgcctcct gcttccaggt ggtctcgctg atgaaagggc tcgctcaagg 1380
   gggtcgctcc atcatttgca ccatccacca gcccagcgcc aaactcttcg agctgttcga 1440
   ccagctttac gtcctgagtc aaggacaatg tgtgtaccgg ggaaaagtct gcaatcttgt 1500
   gccatatttg agggatttgg gtctgaactg cccaacctac cacaacccag cagattttgt 1560
   catggaggtt gcatccggcg agtacggtga tcagaacagt cggctggtga gagcggttcg 1620
   ggagggcatg tgtgactcag accacaagag agacctcggg ggtgatgccg aggtgaaccc 1680
   ttttctttgg caccggccct ctgaagaggt aaagcagaca aaacgattaa aggggttgag 1740
   aaaggactcc tcgtccatgg aaggctgcca cagcttctct gccagctgcc tcacgcagtt 1800
   ctgcatcctc ttcaagagga ccttcctcag catcatgagg gactcggtcc tgacacacct 1860
   gcgcatcacc tcgcacattg ggatcggcct cctcattggc ctgctgtact tggggatcgg 1920
   gaacgaagcc aagaaggtct tgagcaactc cggcttcctc ttcttctcca tgctgttcct 1980
   catgttcgcg gccctcatgc ctactgttct gacatttccc ctggagatgg gagtctttct 2040
   tcgggaacac ctgaactact ggtacagcct gaaggcctac tacctggcca agaccatggc 2100
   agacgtgccc tttcagatca tgttcccagt ggcctactgc agcatcgtgt actggatgac 2160
   gtcgcagccg tccgacgccg tgcgctttgt gctgtttgcc gcgctgggca ccatgacctc 2220
   cctggtggca cagtccctgg gcctgctgat cggagccgcc tccacgtccc tgcaggtggc 2280
   cactttcgtg ggcccagtga cagccatccc ggtgctcctg ttctcggggt tcttcgtcag 2340
   cttcgacacc atccccacgt acctacagtg gatgtcctac atctcctatg tcaggtatgg 2400
   gttcgaaggg gtcatcctct ccatctatgg cttagaccgg gaagatctgc actgtgacat 2460
   cgacgagacg tgccacttcc agaagtcgga ggccatcctg cgggagctgg acgtggaaaa 2520
   tgccaagctg tacctggact tcatcgtact cgggattttc ttcatctccc tccgcctcat 2580
   tgcctatttt gtcctcaggt acaaaatccg ggcagagagg taaaacacct gaatgccagg 2640
   aaacaggaag attagacact gtggccgagg gcacgtctag aatcgaggag gcaagcctgt 2700
   gcccgaccga cgacacagag actcttctga tccaacccct agaaccgcgt tgggtttgtg 2760
   ggtgtctcgt gctcagccac tctgcccagc tgggttggat cttctctcca ttcccctttc 2820
   tagctttaac taggaagatg taggcagatt ggtggttttt ttttttttaa catacagaat 2880
   tttaaatacc acaactgggg cagaatttaa agctgcaaca cagctggtga tgagaggctt 2940
   cctcagtcca gtcgctcctt agcaccaggc accgtgggtc ctggatgggg aactgcaagc 3000
   agcctctcag ctgatggctg cacagtcaga tgtctggtgg cagagagtcc gagcatggag 3060
   cgattccatt ttatgactgt tgtttttcac attttcatct ttctaaggtg tgtctctttt 3120
   ccaatgagaa gtcatttttg caagccaaaa gtcgatcaat cgcattcatt ttaagaaatt 3180
   ataccttttt agtacttgct gaagaatgat tcagggtaaa tcacatactt tgtttagaga 3240
   ggcgaggggt ttaaccgagt cacccagctg gtctcataca tagacagcac ttgtgaagga 3300
   ttgaatgcag gttccaggtg gagggaagac gtggacacca tctccactga gccatgcaga 3360
   catttttaaa agctatacaa aaaattgtga gaagacattg gccaactctt tcaaagtctt 3420
   tctttttcca cgtgcttctt attttaagcg aaatatattg tttgtttctt cct 3473
<210> 9
   <211> 2740
   <212> DNA
   <213> Homo sapiens
<400> 9
   aagtcccagt cctgctgtcc caagggactc cggggtcagg tggagcaggc agggcagtct 60
   gccacgggct ccccaactga agccactctg gggagggtcc ggccaccaga aaatttgccc 120
   agctttgctg cctgttggcc atgggtgacc tctcatcttt gacccccgga gggtccatgg 180
   gtctccaagt aaacagaggc tcccagagct ccctggaggg ggctcctgcc accgccccgg 240
   agcctcacag cctgggcatc ctccatgcct cctacagcgt cagccaccgc gtgaggccct 300
   ggtgggacat cacatcttgc cggcagcagt ggaccaggca gatcctcaaa gatgtctcct 360
   tgtacgtgga gagcgggcag atcatgtgca tcctaggaag ctcaggctcc gggaaaacca 420
   cgctgctgga cgccatgtcc gggaggctgg ggcgcgcggg gaccttcctg ggggaggtgt 480
   atgtgaacgg ccgggcgctg cgccgggagc agttccagga ctgcttctcc tacgtcctgc 540
   agagcgacac cctgctgagc agcctcaccg tgcgcgagac gctgcactac accgcgctgc 600
   tggccatccg ccgcggcaat cccggctcct tccagaagaa ggtggaggcc gtcatggcag 660
   agctgagtct gagccatgtg gcagaccgac tgattggcaa ctacagcttg gggggcattt 720
   ccacgggtga gcggcgccgg gtctccatcg cagcccagct gctccaggat cctaaggtca 780
   tgctgtttga tgagccaacc acaggcctgg actgcatgac tgctaatcag attgtcgtcc 840
   tcctggtgga actggctcgc aggaaccgaa ttgtggttct caccattcac cagccccgtt 900
   ctgagctttt tcagctcttt gacaaaattg ccatcctgag cttcggagag ctgattttct 960
   gtggcacgcc agcggaaatg cttgatttct tcaatgactg cggttaccct tgtcctgaac 1020
   attcaaaccc ttttgacttc tatatggacc tgacgtcagt ggatacccaa agcaaggaac 1080
   gggaaataga aacctccaag agagtccaga tgatagaatc tgcctacaag aaatcagcaa 1140
   tttgtcataa aactttgaag aatattgaaa gaatgaaaca cctgaaaacg ttaccaatgg 1200
   ttcctttcaa aaccaaagat tctcctggag ttttctctaa actgggtgtt ctcctgagga 1260
   gagtgacaag aaacttggtg agaaataagc tggcagtgat tacgcgtctc cttcagaatc 1320
   tgatcatggg tttgttcctc cttttcttcg ttctgcgggt ccgaagcaat gtgctaaagg 1380
   gtgctatcca ggaccgcgta ggtctccttt accagtttgt gggcgccacc ccgtacacag 1440
   gcatgctgaa cgctgtgaat ctgtttcccg tgctgcgagc tgtcagcgac caggagagtc 1500
   aggacggcct ctaccagaag tggcagatga tgctggccta tgcactgcac gtcctcccct 1560
   tcagcgttgt tgccaccatg attttcagca gtgtgtgcta ctggacgctg ggcttacatc 1620
   ctgaggttgc ccgatttgga tatttttctg ctgctctctt ggccccccac ttaattggtg 1680
   aatttctaac tcttgtgcta cttggtatcg tccaaaatcc aaatatagtc aacagtgtag 1740
   tggctctgct gtccattgcg ggggtgcttg ttggatctgg attcctcaga aacatacaag 1800
   aaatgcccat tccttttaaa atcatcagtt attttacatt ccaaaaatat tgcagtgaga 1860
   ttcttgtagt caatgagttc tacggactga atttcacttg tggcagctca aatgtttctg 1920
   tgacaactaa tccaatgtgt gccttcactc aaggaattca attcattgag aaaacctgcc 1980
   caggtgcaac atctagattc acaatgaact ttctgatttt gtattcattt attccagctc 2040
   ttgtcatcct aggaatagtt gttttcaaaa taagggatca tctcattagc aggtagtgaa 2100
   agccatggct gggaaaatgg aagtgaagct gccgactgtg catgactgct ctgaacgtct 2160
   gaaatgagag tgccatgtat ttctttcttg acaggacatc tcaagtcttt taaccattaa 2220
   gactccattt gtgcctcttg gatccaagca ggccttgaat gcaatggaag tggtttatag 2280
   tcccttgctc ttacaacttg cagggacatg tggttatttg gaaattgtga ctgagcggac 2340
   ccaagaatgt aaataatatt cataaaccta tgggagactc gtgtgactat tttttttcct 2400
   tgttctaggc acagaaaaaa ataggtcagc ttaaaaatat gtttacattg gataaaggat 2460
   taggcaaaaa taaaatgttt caaggattcc tgaccataag tgacagagaa agagagttgt 2520
   gggtttagat gaagcaaggt tatcatgcag aattgggtaa gaatgcttct gttcctggaa 2580
   gacccagagt taaatgcaga tgtccacacg aggggtcgga gttacctgat cacatcgaga 2640
   gagtgctggg cagatggatg gtgagcacca ctgctacaga gcacccagtg attttactga 2700
   ggattaaaat aaaaaaccgt aggaatgggc tcaacagtga 2740
<210> 10
   <211> 2679
   <212> DNA
   <213> Homo sapiens
<400> 10
   ctccaggaaa cagagtgaag acactggccc tggcaggcag cagctgggtc taagagagct 60
   gcagcccagg gtcacagacc tgtgggcccc atggccggga aggcggcaga ggagagaggg 120
   ctgccgaaag gggccactcc ccaggatacc tcgggcctcc aggatagatt gttctcctct 180
   gaaagtgaca acagcctgta cttcacctac agtggccagc ccaacaccct ggaggtcaga 240
   gacctcaact gccaggtgga cctggcctct caggtccctt ggtttgagca gctggctcag 300
   ttcaagatgc cctggacatc tcccagctgc cagaattctt gtgagctggg catccagaac 360
   ctaagcttca aagtgagaag tgggcagatg ctggccatca tagggagctc aggttgtggg 420
   agagcctcct tgctagatgt gatcactggc cgaggtcacg gcggcaagat caagtcaggc 480
   cagatctgga tcaatgggca gcccagctcg cctcagctgg tgaggaagtg tgtggcccac 540
   gtgcgccagc acaaccagct gctccccaac ttgactgtgc gagagacctt ggccttcatt 600
   gcccagatgc ggctgcccag aaccttctcc caggcccagc gtgacaaaag ggtggaggac 660
   gtgatcgcgg agctgcggct taggcagtgc gctgacaccc gcgtgggcaa catgtacgtg 720
   cgggggttgt cggggggtga gcgcaggaga gtcagcattg gggtgcagct cctgtggaac 780
   ccaggaatcc ttattctcga cgaacccacc tctgggctcg acagcttcac agcccacaac 840
   ctggtgaaga ccttgtccag gctggccaaa ggcaaccggc tggtgctcat ctccctccac 900
   cagcctcgct ctgacatctt caggctgttt gatctggtcc tcctgatgac gtctggcacc 960
   cccatctact taggggcggc ccagcacatg gtccagtatt tcacagccat cggctacccc 1020
   tgtcctcgct acagcaatcc tgctgacttc tatgtggacc tgaccagcat tgacaggcgc 1080
   agcagagagc aggaattggc caccagggag aaggctcagt cactcgcagc cctgtttcta 1140
   gaaaaagtgc gtgacttaga tgactttcta tggaaagcag agacgaagga tcttgacgag 1200
   gacacctgtg tggaaagcag cgtgacccca ctagacacca actgcctccc gagtcctacg 1260
   aagatgcctg gggcggtgca gcagtttacg acgctgatcc gtcgtcagat ttccaacgac 1320
   ttccgagacc tgcccaccct cctcatccat ggggcggagg cctgtctgat gtcaatgacc 1380
   atcggcttcc tctattttgg ccatgggagc atccagctct ccttcatgga tacagccgcc 1440
   ctcttgttca tgatcggtgc tctcatccct ttcaacgtca ttctggatgt catctccaaa 1500
   tgttactcag agagggcaat gctttactat gaactggaag acgggctgta caccactggt 1560
   ccatatttct ttgccaagat cctcggggag cttccggagc actgtgccta catcatcatc 1620
   tacgggatgc ccacctactg gctggccaac ctgaggccag gcctccagcc cttcctgctg 1680
   cacttcctgc tggtgtggct ggtggtcttc tgttgcagga ttatggccct ggccgccgcg 1740
   gccctgctcc ccaccttcca catggcctcc ttcttcagca atgccctcta caactccttc 1800
   tacctcgccg ggggcttcat gataaacttg agcagcctgt ggacagtgcc cgcgtggatt 1860
   tccaaagtgt ccttcctgcg gtggtgtttt gaagggctga tgaagattca gttcagcaga 1920
   agaacttata aaatgcctct cgggaacctc accatcgcgg tctcaggaga taaaatcctc 1980
   agtgccatgg agctggactc gtaccctctc tacgccatct acctcatcgt cattggcctc 2040
   agcggtggct tcatggtcct gtactacgtg tccttaaggt tcatcaaaca gaaaccaagt 2100
   caagactggt gattcacgcc agacgtctgc ccgctggtgg gggacctgag cagacccttc 2160
   aactgcactc cctcctcagg agccccttcc tggggacagt gaggacaatg accctacaga 2220
   tgctcagcta catccggccc agggtgctgc ggtggcacag accagccaca ggatggcagt 2280
   agaataaaga cagtcgaaag ggatttctgc tcactggcag gagactgcga tgactgggag 2340
   aaaacctgca ctcggtggca cctacaacgt tgctaattta tttccttttg atatgcattt 2400
   atataggcaa ctcgatatag gatgggagca aactaggaat gaattgggta gctagactgt 2460
   gcaggaattg ttggaacctg gagggaacaa taacagtacc tagcagattt ggcttcatct 2520
   tccaggggcc ccacactccg tggtgagcca ccatcaatac agaaagtgac ctaagatgta 2580
   ccagcaagat gccatccctt ctttttgtgt ggggtcatgg gctccaaaag ccaacgtgaa 2640
   caattaaaaa tgtattgagc atctaaaaaa aaaaaaaaa 2679
<210> 11
   <211> 1156
   <212> DNA
   <213> Homo sapiens
<400> 11
   cgcagcggag gtgaaggacg tccttcccca ggagccgact ggccaatcac aggcaggaag 60
   atgaaggttc tgtgggctgc gttgctggtc acattcctgg caggatgcca ggccaaggtg 120
   gagcaagcgg tggagacaga gccggagccc gagctgcgcc agcagaccga gtggcagagc 180
   ggccagcgct gggaactggc actgggtcgc ttttgggatt acctgcgctg ggtgcagaca 240
   ctgtctgagc aggtgcagga ggagctgctc agctcccagg tcacccagga actgagggcg 300
   ctgatggacg agaccatgaa ggagttgaag gcctacaaat cggaactgga ggaacaactg 360
   accccggtgg cggaggagac gcgggcacgg ctgtccaagg agctgcaggc ggcgcaggcc 420
   cggctgggcg cggacatgga ggacgtgtgc ggccgcctgg tgcagtaccg cggcgaggtg 480
   caggccatgc tcggccagag caccgaggag ctgcgggtgc gcctcgcctc ccacctgcgc 540
   aagctgcgta agcggctcct ccgcgatgcc gatgacctgc agaagcgcct ggcagtgtac 600
   caggccgggg cccgcgaggg cgccgagcgc ggcctcagcg ccatccgcga gcgcctgggg 660
   cccctggtgg aacagggccg cgtgcgggcc gccactgtgg gctccctggc cggccagccg 720
   ctacaggagc gggcccaggc ctggggcgag cggctgcgcg cgcggatgga ggagatgggc 780
   agccggaccc gcgaccgcct ggacgaggtg aaggagcagg tggcggaggt gcgcgccaag 840
   ctggaggagc aggcccagca gatacgcctg caggccgagg ccttccaggc ccgcctcaag 900
   agctggttcg agcccctggt ggaagacatg cagcgccagt gggccgggct ggtggagaag 960
   gtgcaggctg ccgtgggcac cagcgccgcc cctgtgccca gcgacaatca ctgaacgccg 1020
   aagcctgcag ccatgcgacc ccacgccacc ccgtgcctcc tgcctccgcg cagcctgcag 1080
   cgggagaccc tgtccccgcc ccagccgtcc tcctggggtg gaccctagtt taataaagat 1140
   tcaccaagtt tcacgc 1156
<210> 12
   <211> 417
   <212> DNA
   <213> Homo sapiens
<400> 12
   acctcccaac caagccctcc agcaaggatt caggagtgcc cctcgggcct cgccatgagg 60
   ctcttcctgt cgctcccggt cctggtggtg gttctgtcga tcgtcttgga aggcccagcc 120
   ccagcccagg ggaccccaga cgtctccagt gccttggata agctgaagga gtttggaaac 180
   acactggagg acaaggctcg ggaactcatc agccgcatca aacagagtga actttctgcc 240
   aagatgcggg agtggttttc agagacattt cagaaagtga aggagaaact caagattgac 300
   tcatgaggac ctgaagggtg acatccagga ggggcctctg aaatttccca caccccagcg 360
   cctgtgctga ggactcccgc catgtggccc caggtgccac caataaaaat cctaccg 417
<210> 13
   <211> 753
   <212> DNA
   <213> Homo sapiens
<400> 13
   gttgtggctg tggagcggaa gtgggtctca accactataa atcctctctg tgcccgtccg    60
   gagctggtga ggacagcctg ccagagtctg gtctctggac actatgggca cacgactcct   120
   cccagctctg tttcttgtcc tcctggtatt gggatttgag gtccagggga cccaacagcc   180
   ccagcaagat gagatgccta gcccgacctt cctcacccag gtgaaggaat ctctctccag   240
   ttactgggag tcagcaaaga cagccgccca gaacctgtac gagaagacat acctgcccgc   300
   tgtagatgag aaactcaggg acttgtacag caaaagcaca gcagccatga gcacttacac   360
   aggcattttt actgaccaag ttctttctgt gctgaaggga gaggagtaac agccagaccc   420
   cccatcagtg gacaagggga gagtccccta ctcccctgat cccccaggtt cagactgagc   480
   tcccccttcc cagtagctct tgcatcctcc tcccaactct agcctgaatt cttttcaata   540
   aaaaatacaa ttcaagttgc ttctcatgga tggcactgct tttctgagga ctcaagggcc   600
   aagatggagg ggctgactca gtccagccaa catttaatga gcacctactt tatgtatgga   660
   gctctaaccc atgggtccat gggaataaag cagtgaatag taacaataaa taatcgtaac   720
   aaaaaaaaaa aaaaaaaaaa aaaaaaaaaa aaa                                753
<210> 14
   <211> 5950
   <212> DNA
   <213> Homo sapiens
<400> 14
   gaattccagg ggacatctgt tgcgcaccta ctgtgctcac gctggggcct ctgggatgga    60
   caggattctg ccaaggcaga catctgggtc aagacagtcc tgcacagttg ttcaggttgt   120
   ggccaaggtt gcgtttgcag atttgccatg taaaaataca ggatgctcag ttacatttga   180
   atttcagatt aatagcaaaa aaaacttttt ggtataattc tgaaatattt catgggacat   240
   atttatacta aaacgtcatg cactgttgat ttgaaattca aatgtaattg ggcctcctct   300
   atttcgtctg gcaagcgtag acaaaagaat ccagtccagg ccaggcgcag tggctcaagc   360
   ctgtaatccc agcactttgg gaggccgagg cgggcggatc acgaggtcag gagatcgaga   420
   ccatcctggc taacacggtg aaaccccgtc tctactaaaa atacaaaaaa ttagctgggc   480
   gtggtggcgg gtgcctgtag tcccagctac tcgggaggct gaggcaggag aatggcgtga   540
   acctgggagg cggagcttgc agtgagccga gatcgcgcca cagcactcca gcctgggcga   600
   cagagccaga ctctgtctca aaaaaaaaaa aaagaatcca gtccatagtc ccctgagcca   660
   tgtgccctgg ggtgcagctg ggtccttcag gagaaaaatg ctctatttct ggcactggga   720
   ccgagcctga tgtgggtttt ttgttggttt ttgttgttgt tattgttttt gagacaaggt 780
   ctcgctccac cacacccggc taatttttgt atttttagta gagacggggt ttcactacgt 840
   tggccaggct ggtcttgaac tcctgacctc aagtgagccg cctgcctcgg cctcccaaag 900
   tgctgggatt acaggtggga gccaccgccc tggccctggg cctgatgttg atgacctcct 960
   actatgtgca cctgcagctc tcctgcatag gcctcagccg tcctgcatga ggacactggg 1020
   aggcaggtgc tccctatcaa ccccgtgtta cagttgaaca aactgagccc cagaaaagaa 1080
   aacgtatttg cccaggtcac acggtgaaga agtgagggat tcgaagccca ggtccatctg 1140
   aagccagagt cacccagagg agaaagagtt ggaattgaga actcaaggaa tgcttggaag 1200
   tgatcgggct cgagcccacc taggaagaaa cagaggctgg agacatgaga ctgtgttgct 1260
   atttcctctc atcaaccctt gggccctatt gaggccctac cacaagcctg gccctgcagc 1320
   ccagtgacta ggagaaatta gacacaagat aataataaca gcaatgatct tttttttttt 1380
   tctgagacgg agtcttgctc tttcgcccag gctggactgc agtggcgcga tctcggctca 1440
   atgcaagctc cacctcccag gttcacgcca ttctcctgcc tcagcctccc gagtagctag 1500
   gactacaggc gcctgccacc acgcctggct aatttttcat atttttagta gagatggggt 1560
   ttcaccgtgt tagccaagat ggtctcaatc tcctgacctc gtgatccgcc tgcctcggcc 1620
   tcccaaagtg ttggggttac aggcatgagc caccgcgcct ggccaacagc aatgatcttt 1680
   gagcacctat attgccagtc tccacggtaa gagctttctt cattttttgt tttgttttgt 1740
   ttcaagacag agtcttgctc tgtcacccag gctggagtgc agtggtgtga tcgcggctca 1800
   ctgcagcctt cacttcccgg gttcaagcca ttctcctgcc tcagcctccc aagtagctgg 1860
   gattacaggc acgcatcact acttctggct aatttttgta tttttagtag ggacagggtt 1920
   tttcaccatg ttggccaggt tggtctcaaa ctcctggcct catatgatct gcccacctcg 1980
   gcctcccaaa gtgctgggat tacaggcgtg agccactgcg cctttctttg tatttgttca 2040
   agtaatatac tgaaatatgt actgtgcctc ccactttatg gaggaggaaa ctgaggccag 2100
   caaatgaggc tgtcatggga ggtggagaca ggatttgaac ctgcctcagt gcaggaggct 2160
   caagagcctc tgtcttctct cagggcactg tgtgggaggg tgagaaggag ggaggcccac 2220
   agaggcatga cctctgattg ccactgtcac ctgggccctg ctctctgaag tctctgccaa 2280
   gcggggaggt ggccggggga gggccctgct ctgtgcagcc tcccctcccc cggcccgcag 2340
   agttgagcac agagggacag aggcacggaa cccccagaaa tgtccctcct cagaaacagg 2400
   ctccaggccc tgcctgccct gtgcctctgc gtgctggtcc tggcctgcat tgggggtgag 2460
   aagaagtggg tggagggatg tggggcccac acctggtggg tgtgagtgtg gctgtgtgtc 2520
   ctgtggctct gtagccacgt gagacatgag tacggagtgt gtgcgtttca tggcgtgcgt 2580
   atgcatgtgc gtgtcgggga gtgtgtgtgt cggtggctga gagtgaagtg tgaatgtcac 2640
   attggtacaa actgggatca tctgtgtgtg tgcacgtgcg tgcgtggaag tgggagtatg 2700
   cagtcgtggt aaaaaagtgc atgtctgtgt gcatatgtgt atttgtgtgc acctgtctct 2760
   ctgtggggta tgtgtgtgca aaatatttga gtgtgtggac atgtgtgagg gggtgagtgt 2820
   gtgctggtgt gtacgtctgt gttttgcata tgcatttttt tttttttttt ttagacggag 2880
   tctcactctg tcacccaggc tggagtgcag tggtagcagt ggtgcgatct tggctcactg 2940
   catcatccgc ctacccgttt caagggattc tcctgcctca gtcttcagag tatttgggac 3000
   tacagacaca cgccaccatg cctggctaat tttttttttt tgagacggag tctcgctctg 3060
   ttacccaggc tggagtgcag tggcgtgatc ttggctcact gcaagctccg cctcccgggt 3120
   tcacgccatt ctcctgcctc agcctcccga gtagctggga ctacaggagc ccaccaccac 3180
   gcctggctaa ttttttgtat ttttactaga gacggggttt cgccgtgtta gccaggatgg 3240
   tctccatatc ctgacctcgt gatccgcctg cctcggcctt ccaaagtgct aggattatag 3300
   gcgtgagcca ctgcgcctgg ccaatgcctg gctaattttt ttatattttt ggtagagaca 3360
   gggttttgcc atgttgccca ggctggtctt gaaatcctga cctcaggtga tccgcccgcc 3420
   ttggcctccc aaagtgctgg gattacaggc atgagccacc acgcccggcc atgtacttta 3480
   tgttaaaatg ggatcatatt ctagatcagc attatccagt agaaatttaa atttttaata 3540
   cagggccagg cacggtggct catgcctgta atcccagcac tttcggaggc cgaggcgggt 3600
   ggatcgcaag gtcaggagat ttgagatcat cctggctaac agatgggtaa aaacccatct 3660
   ctactaaaaa tacaaaaaat tagccatgca tggtggcatg cgcctgtagt cccagctact 3720
   cgggaggctg aggccggaga atcacttgaa cccgggaggc agaggttgca gtgagccgag 3780
   atcgcgccac tgcattccaa cctgggtgac agagcgagac tccgtctgaa aaaaaaaaaa 3840
   aatttaacac gtatgtagac aatgtgcaag gcaccattcc atgtgcatcg tatgtagtaa 3900
   ctcttaattc tcacgataac cctgaggtag atattattac cccgttctac aaaaggagaa 3960
   acagtcctgg ggagacagga taagtcaccg gccaaggcac acagctagct acatgtggcc 4020
   cccgcgtgac ggctggtctc tgtaggcgag gctttgtcca gatgcgtggg tagaaggtct 4080
   ggcccggaaa gaggaactga cagcaaggct aagccaatgt ctgcccctgg gggcagaaag 4140
   tcacctcctg ctctccctcc actgtccaca gaggtagctc agacagggtg ggggtcacag 4200
   gagaacgaag ggagaagggg gtagttcctg ggcagcaaaa tcaggtggtg aagggaggca 4260
   tcagaggatg gcaattagag aggccattag aggggaacca caggcagaca gggtgacagg 4320
   agggactact gacacaaggt gaagagatgg cccagccgga cggggtggct cacatctgta 4380
   atcccagcat tttgggagcc cgaggtgggt ggatcacttg aggtcaggag ttcgaggccc 4440
   caacatggca aaaccccatc tcttctaaaa atacaaaaat tagccgggca tgatggcaga 4500
   tgcctgtaat ccctgctact cgggaggctg aggcaggaaa attgcctgaa tccaggaggt 4560
   ggaggttgca atgagacgag atcatgacac tgcactccac cctgggcaac agagcaagag 4620
   actgactctg tctcataaaa aaaaagaaaa aagaaaaaaa aaaagagatg gctgatggtt 4680
   aaagaggggt tagcggtcag gggacacata agggtaaagg caggaggcaa gaggactggc 4740
   agggggctgc ccctgggcca ccgggagcga cacaggatga gcatggaggg aaagggagaa 4800
   ggggattcta gggtcccagc ctacccaagt tgccctctgg ttccacctag catgccagcc 4860
   agaggcccag gaaggaaccc tgagcccccc accaaagcta aagatgagtc gctggagcct 4920
   ggtgaggggc aggatgaagg agctgctgga gacagtggtg aacaggacca gagacgggtg 4980
   gcaatggttc tggtgagggt gtgctggcct gggtggtggg aggggactcc tgggtctgag 5040
   ggaggagggg ctggggcctg gacccctgag tctcagggag gaggaaaggg tgggagtggg 5100
   gctgtgaccc ctaggtctgg gaggagtgga gggttagagc tgagagcagg aactcctagg 5160
   tcacagagag gagcggataa atggggcaga gaacacctgg ggagagctgg ggcctccact 5220
   gtgatgtcct ctctcctgta ggagcccgag caccttccgg ggcttcatgc agacctacta 5280
   tgacgaccac ctgagggacc tgggtccgct caccaaggcc tggttcctcg aatccaaaga 5340
   cagcctcttg aagaagaccc acagcctgtg ccccaggctt gtctgtgggg acaaggacca 5400
   gggttaaaat gttcataaaa gccaggtgtg gttgtggcgg gtgcctgtag tcccagctac 5460
   tcaggaggct gaggtaggat gatggcttga gcccaggagt tcgagaccag cctgggcaac 5520
   acagcgagat ctcttggggg taaaacaaaa agaaaaaaaa aagttcatac ttctccaata 5580
   aataaagtct cacctgtgtc cctgtctgga tccttcccca gtgtggccag aaaaaaaccc 5640
   accccactgc ctcccaggaa tcaatgagta gaagaggtga cacctgatgg ggaaggaaga 5700
   gtagggaggt cgggaagggt atcaaggaat aacaccctat tgtgggcttg cggagaatgg 5760
   gggacttcaa ggcgtgtcag tttcaggagg gtgagggcag gagcgtgggt ggagtcagca 5820
   ggtccccatg atggccctca ctgagagctt cgcccttgtc tcctacaagc tctgactcca 5880
   ttcccagtgg gcacccagca cctccaaccc ctccacagcc cccaacccag cctctgtcgg 5940
   aggcgaattc 5950
<210> 15
   <211> 3549
   <212> DNA
   <213> Homo sapiens
<400> 15
   cccctcttcc tcctcctcaa gggaaagctg cccacttcta gctgccctgc catccccttt 60
   aaagggcgac ttgctcagcg ccaaaccgcg gctccagccc tctccagcct ccggctcagc 120
   cggctcatca gtcggtccgc gccttgcagc tcctccagag ggacgcgccc cgagatggag 180
   agcaaagccc tgctcgtgct gactctggcc gtgtggctcc agagtctgac cgcctcccgc 240
   ggaggggtgg ccgccgccga ccaaagaaga gattttatcg acatcgaaag taaatttgcc 300
   ctaaggaccc ctgaagacac agctgaggac acttgccacc tcattcccgg agtagcagag 360
   tccgtggcta cctgtcattt caatcacagc agcaaaacct tcatggtgat ccatggctgg 420
   acggtaacag gaatgtatga gagttgggtg ccaaaacttg tggccgccct gtacaagaga 480
   gaaccagact ccaatgtcat tgtggtggac tggctgtcac gggctcagga gcattaccca 540
   gtgtccgcgg gctacaccaa actggtggga caggatgtgg cccggtttat caactggatg 600
   gaggaggagt ttaactaccc tctggacaat gtccatctct tgggatacag ccttggagcc 660
   catgctgctg gcattgcagg aagtctgacc aataagaaag tcaacagaat tactggcctc 720
   gatccagctg gacctaactt tgagtatgca gaagccccga gtcgtctttc tcctgatgat 780
   gcagattttg tagacgtctt acacacattc accagagggt cccctggtcg aagcattgga 840
   atccagaaac cagttgggca tgttgacatt tacccgaatg gaggtacttt tcagccagga 900
   tgtaacattg gagaagctat ccgcgtgatt gcagagagag gacttggaga tgtggaccag 960
   ctagtgaagt gctcccacga gcgctccatt catctcttca tcgactctct gttgaatgaa 1020
   gaaaatccaa gtaaggccta caggtgcagt tccaaggaag cctttgagaa agggctctgc 1080
   ttgagttgta gaaagaaccg ctgcaacaat ctgggctatg agatcaataa agtcagagcc 1140
   aaaagaagca gcaaaatgta cctgaagact cgttctcaga tgccctacaa agtcttccat 1200
   taccaagtaa agattcattt ttctgggact gagagtgaaa cccataccaa tcaggccttt 1260
   gagatttctc tgtatggcac cgtggccgag agtgagaaca tcccattcac tctgcctgaa 1320
   gtttccacaa ataagaccta ctccttccta atttacacag aggtagatat tggagaacta 1380
   ctcatgttga agctcaaatg gaagagtgat tcatacttta gctggtcaga ctggtggagc 1440
   agtcccggct tcgccattca gaagatcaga gtaaaagcag gagagactca gaaaaaggtg 1500
   atcttctgtt ctagggagaa agtgtctcat ttgcagaaag gaaaggcacc tgcggtattt 1560
   gtgaaatgcc atgacaagtc tctgaataag aagtcaggct gaaactgggc gaatctacag 1620
   aacaaagaac ggcatgtgaa ttctgtgaag aatgaagtgg aggaagtaac ttttacaaaa 1680
   catacccagt gtttggggtg tttcaaaagt ggattttcct gaatattaat cccagcccta 1740
   cccttgttag ttattttagg agacagtctc aagcactaaa aagtggctaa ttcaatttat 1800
   ggggtatagt ggccaaatag cacatcctcc aacgttaaaa gacagtggat catgaaaagt 1860
   gctgttttgt cctttgagaa agaaataatt gtttgagcgc agagtaaaat aaggctcctt 1920
   catgtggcgt attgggccat agcctataat tggttagaac ctcctatttt aattggaatt 1980
   ctggatcttt cggactgagg ccttctcaaa ctttactcta agtctccaag aatacagaaa 2040
   atgcttttcc gcggcacgaa tcagactcat ctacacagca gtatgaatga tgttttagaa 2100
   tgattccctc ttgctattgg aatgtggtcc agacgtcaac caggaacatg taacttggag 2160
   agggacgaag aaagggtctg ataaacacag aggttttaaa cagtccctac cattggcctg 2220
   catcatgaca aagttacaaa ttcaaggaga tataaaatct agatcaatta attcttaata 2280
   ggctttatcg tttattgctt aatccctctc tcccccttct tttttgtctc aagattatat 2340
   tataataatg ttctctgggt aggtgttgaa aatgagcctg taatcctcag ctgacacata 2400
   atttgaatgg tgcagaaaaa aaaaagatac cgtaatttta ttattagatt ctccaaatga 2460
   ttttcatcaa tttaaaatca ttcaatatct gacagttact cttcagtttt aggcttacct 2520
   tggtcatgct tcagttgtac ttccagtgcg tctcttttgt tcctggcttt gacatgaaaa 2580
   gataggtttg agttcaaatt ttgcattgtg tgagcttcta cagattttag acaaggaccg 2640
   tttttactaa gtaaaagggt ggagaggttc ctggggtgga ttcctaagca gtgcttgtaa 2700
   accatcgcgt gcaatgagcc agatggagta ccatgagggt tgttatttgt tgtttttaac 2760
   aactaatcaa gagtgagtga acaactattt ataaactaga tctcctattt ttcagaatgc 2820
   tcttctacgt ataaatatga aatgataaag atgtcaaata tctcagaggc tatagctggg 2880
   aacccgactg tgaaagtatg tgatatctga acacatacta gaaagctctg catgtgtgtt 2940
   gtccttcagc ataattcgga agggaaaaca gtcgatcaag ggatgtattg gaacatgtcg 3000
   gagtagaaat tgttcctgat gtgccagaac ttcgaccctt tctctgagag agatgatcgt 3060
   gcctataaat agtaggacca atgttgtgat taacatcatc aggcttggaa tgaattctct 3120
   ctaaaaataa aatgatgtat gatttgttgt tggcatcccc tttattaatt cattaaattt 3180
   ctggatttgg gttgtgaccc agggtgcatt aacttaaaag attcactaaa gcagcacata 3240
   gcactgggaa ctctggctcc gaaaaacttt gttatatata tcaaggatgt tctggcttta 3300
   cattttattt attagctgta aatacatgtg tggatgtgta aatggagctt gtacatattg 3360
   gaaaggtcat tgtggctatc tgcatttata aatgtgtggt gctaactgta tgtgtcttta 3420
   tcagtgatgg tctcacagag ccaactcact cttatgaaat gggctttaac aaaacaagaa 3480
   agaaacgtac ttaactgtgt gaagaaatgg aatcagcttt taataaaatt gacaacattt 3540
   tattaccac 3549
<210> 16
   <211> 1790
   <212> DNA
   <213> Homo sapiens
<400> 16
   gtgaatctct ggggccagga agaccctgct gcccggaaga gcctcatgtt ccgtgggggc 60
   tgggcggaca tacatatacg ggctccaggc tgaacggctc gggccactta cacaccactg 120
   cctgataacc atgctggctg ccacagtcct gaccctggcc ctgctgggca atgcccatgc 180
   ctgctccaaa ggcacctcgc acgaggcagg catcgtgtgc cgcatcacca agcctgccct 240
   cctggtgttg aaccacgaga ctgccaaggt gatccagacc gccttccagc gagccagcta 300
   cccagatatc acgggcgaga aggccatgat gctccttggc caagtcaagt atgggttgca 360
   caacatccag atcagccact tgtccatcgc cagcagccag gtggagctgg tggaagccaa 420
   gtccattgat gtctccattc agaacgtgtc tgtggtcttc aaggggaccc tgaagtatgg 480
   ctacaccact gcctggtggc tgggtattga tcagtccatt gacttcgaga tcgactctgc 540
   cattgacctc cagatcaaca cacagctgac ctgtgactct ggtagagtgc ggaccgatgc 600
   ccctgactgc tacctgtctt tccataagct gctcctgcat ctccaagggg agcgagagcc    660
   tgggtggatc aagcagctgt tcacaaattt catctccttc accctgaagc tggtcctgaa    720
   gggacagatc tgcaaagaga tcaacgtcat ctctaacatc atggccgatt ttgtccagac    780
   aagggctgcc agcatccttt cagatggaga cattggggtg gacatttccc tgacaggtga    840
   tcccgtcatc acagcctcct acctggagtc ccatcacaag ggtcatttca tctacaagaa    900
   tgtctcagag gacctccccc tccccacctt ctcgcccaca ctgctggggg actcccgcat    960
   gctgtacttc tggttctctg agcgagtctt ccactcgctg gccaaggtag ctttccagga   1020
   tggccgcctc atgctcagcc tgatgggaga cgagttcaag gcagtgctgg agacctgggg   1080
   cttcaacacc aaccaggaaa tcttccaaga ggttgtcggc ggcttcccca gccaggccca   1140
   agtcaccgtc cactgcctca agatgcccaa gatctcctgc caaaacaagg gagtcgtggt   1200
   caattcttca gtgatggtga aattcctctt tccacgccca gaccagcaac attctgtagc   1260
   ttacacattt gaagaggata tcgtgactac cgtccaggcc tcctattcta agaaaaagct   1320
   cttcttaagc ctcttggatt tccagattac accaaagact gtttccaact tgactgagag   1380
   cagctccgag tccatccaga gcttcctgca gtcaatgatc accgctgtgg gcatccctga   1440
   ggtcatgtct cggctcgagg tagtgtttac agccctcatg aacagcaaag gcgtgagcct   1500
   cttcgacatc atcaaccctg agattatcac tcgagatggc ttcctgctgc tgcagatgga   1560
   ctttggcttc cctgagcacc tgctggtgga tttcctccag agcttgagct agaagtctcc   1620
   aaggaggtcg ggatggggct tgtagcagaa ggcaagcacc aggctcacag ctggaaccct   1680
   ggtgtctcct ccagcgtggt ggaagttggg ttaggagtac ggagatggag attggctccc   1740
   aactcctccc tatcctaaag gcccactggc attaaagtgc tgtatccaag               1790
<210> 17
   <211> 2688
   <212> DNA
   <213> Homo sapiens
<400> 17
   tttaaagctg ggaggttctg ccaccaagca cggccttccc actgggaaca caaacttgct     60
   ggcgggaaga gcccggaaag aaacctgtgg atctcccttc gagatcatcc aaagagaaga    120
   aaggtgacct cacattcgtg ccccttagca gcactctgca gaaatgcctc ctcagctgca    180
   aaacggcctg aacctctcgg ccaaagttgt ccagggaagc ctggacagcc tgccccaggc    240
   agtgagggag tttctcgaga ataacgctga gctgtgtcag cctgatcaca tccacatctg    300
   tgacggctct gaggaggaga atgggcggct tctgggccag atggaggaag agggcatcct    360
   caggcggctg aagaagtatg acaactgctg gttggctctc actgacccca gggatgtggc    420
   caggatcgaa agcaagacgg ttatcgtcac ccaagagcaa agagacacag tgcccatccc    480
   caaaacaggc ctcagccagc tcggtcgctg gatgtcagag gaggattttg agaaagcgtt 540
   caatgccagg ttcccagggt gcatgaaagg tcgcaccatg tacgtcatcc cattcagcat 600
   ggggccgctg ggctcgcctc tgtcaaagat cggcatcgag ctgacggatt caccctacgt 660
   ggtggccagc atgcggatca tgacgcggat gggcacgccc gtcctggaag cactgggcga 720
   tggggagttt gtcaaatgcc tccattctgt ggggtgccct ctgcctttac aaaagccttt 780
   ggtcaacaac tggccctgca acccggagct gacgctcatc gcccacctgc ctgaccgcag 840
   agagatcatc tcctttggca gtgggtacgg cgggaactcg ctgctcggga agaagtgctt 900
   tgctctcagg atggccagcc ggctggccaa ggaggaaggg tggctggcag agcacatgct 960
   ggttctgggt ataaccaacc ctgagggtga gaagaagtac ctggcggccg catttcccag 1020
   cgcctgcggg aagaccaacc tggccatgat gaaccccagc ctccccgggt ggaaggttga 1080
   gtgcgtcggg gatgacattg cctggatgaa gtttgacgca caaggtcatt taagggccat 1140
   caacccagaa aatggctttt tcggtgtcgc tcctgggact tcagtgaaga ccaaccccaa 1200
   tgccatcaag accatccaga agaacacaat ctttaccaat gtggccgaga ccagcgacgg 1260
   gggcgtttac tgggaaggca ttgatgagcc gctagcttca ggtgtcacca tcacgtcctg 1320
   gaagaataag gagtggagct cagaggatgg ggaaccttgt gcccacccca actcgaggtt 1380
   ctgcacccct gccagccagt gccccatcat tgatgctgcc tgggagtctc cggaaggtgt 1440
   tcccattgaa ggcattatct ttggaggccg tagacctgct ggtgtccctc tagtctatga 1500
   agctctcagc tggcaacatg gagtctttgt gggggcggcc atgagatcag aggccacagc 1560
   ggctgcagaa cataaaggca aaatcatcat gcatgacccc tttgccatgc ggcccttctt 1620
   tggctacaac ttcggcaaat acctggccca ctggcttagc atggcccagc acccagcagc 1680
   caaactgccc aagatcttcc atgtcaactg gttccggaag gacaaggaag gcaaattcct 1740
   ctggccaggc tttggagaga actccagggt gctggagtgg atgttcaacc ggatcgatgg 1800
   aaaagccagc accaagctca cgcccatagg ctacatcccc aaggaggatg ccctgaacct 1860
   gaaaggcctg gggcacatca acatgatgga gcttttcagc atctccaagg aattctggga 1920
   gaaggaggtg gaagacatcg agaagtatct ggaggatcaa gtcaatgccg acctcccctg 1980
   tgaaatcgag agagagatcc ttgccttgaa gcaaagaata agccagatgt aatcagggcc 2040
   tgagtgcttt acctttaaaa tcattccctt tcccatccat aaggtgcagt aggagcaaga 2100
   gagggcaagt gttcccaaat tgacgccacc ataataatca tcaccacacc gtgagcagat 2160
   ctgaaaggca cactttgatt tttttaagga taagaaccac agaacactgg gtagtagcta 2220
   atgaaattga gaagggaaat cttagcatgc ctccaaaaat tcacatccaa tgcatagttt 2280
   gttcaaattt aaggttactc aggcattgat cttttcagtg ttttttcact ttagctatgt 2340
   ggattagcta gaatgcacac caaaaaaata cttgagctgt atatatatat gtgtgtgtgt 2400
   gtgtgtgtgt gtgtgtgtgt gtgtgcatgt atgtgcacat gtgtctgtgt ggtatatttg 2460
   tgtatgtgta tttgtatgta ctgttattga aaatatattt aatacctttg gaaaaatctt 2520
   gggcaagatg acctactagt tttccttgaa aaaaagttgc tttgttatta atattgtgct 2580
   taaattattt ttatacacca ttgttcctta cctttacata attgcaatat ttccccctta 2640
   ctacttcttg gaaaaaaatt acaaaatgaa gttttataga aaagatgg 2688
<210> 18
   <211> 3095
   <212> DNA
   <213> Homo sapiens
<400> 18
   tagcagagca atcaccacca agcctggaat aactgcaagg gctctgctga catcttcctg 60
   aggtgccaag gaaatgagga tggaggaagg aatgaatgtt ctccatgact ttgggatcca 120
   gtcaacacat tacctccagg tgaattacca agactcccag gactggttca tcttggtgtc 180
   cgtgatcgca gacctcagga atgccttcta cgtcctcttc cccatctggt tccatcttca 240
   ggaagctgtg ggcattaaac tcctttgggt agctgtgatt ggagactggc tcaacctcgt 300
   ctttaagtgg attctctttg gacagcgtcc atactggtgg gttttggata ctgactacta 360
   cagcaacact tccgtgcccc tgataaagca gttccctgta acctgtgaga ctggaccagg 420
   gagcccctct ggccatgcca tgggcacagc aggtgtatac tacgtgatgg tcacatctac 480
   tctttccatc tttcagggaa agataaagcc gacctacaga tttcggtgct tgaatgtcat 540
   tttgtggttg ggattctggg ctgtgcagct gaatgtctgt ctgtcacgaa tctaccttgc 600
   tgctcatttt cctcatcaag ttgttgctgg agtcctgtca ggcattgctg ttacagaaac 660
   tttcagccac atccacagca tctataatgc cagcctcaag aaatattttc tcattacctt 720
   cttcctgttc agcttcgcca tcggatttta tctgctgctc aagggactgg gtgtagacct 780
   cctgtggact ctggagaaag cccagaggtg gtgcgagcag ccagaatggg tccacattga 840
   caccacaccc tttgccagcc tcctcaagaa cctgggcacg ctctttggcc tggggctggc 900
   tctcaactcc agcatgtaca gggagagctg caaggggaaa ctcagcaagt ggctcccatt 960
   ccgcctcagc tctattgtag cctccctcgt cctcctgcac gtctttgact ccttgaaacc 1020
   cccatcccaa gtcgagctgg tcttctacgt cttgtccttc tgcaagagtg cggtagtgcc 1080
   cctggcatcc gtcagtgtca tcccctactg cctcgcccag gtcctgggcc agccgcacaa 1140
   gaagtcgttg taagagatgt ggagtcttcg gtgtttaaag tcaacaacca tgccagggat 1200
   tgaggaggac tactatttga agcaatgggc actggtattt ggagcaagtg acatgccatc 1260
   cattctgccg tcgtggaatt aaatcacgga tggcagattg gagggtcgcc tggcttattc 1320
   ccatgtgtga ctccagcctg ccctcagcac agactctttc agatggaggt gccatatcac 1380
   gtacaccata tgcaagtttc ccgccaggag gtcctcctct ctctacttga atactctcac 1440
   aagtagggag ctcactccca ctggaacagc ccattttatc tttgaatggt cttctgccag 1500
   cccattttga ggccagaggt gctgtcagct caggtggtcc tcttttacaa tcctaatcat 1560
   attgggtaat gtttttgaaa agctaatgaa gctattgaga aagacctgtt gctagaagtt 1620
   gggttgttct ggattttccc ctgaagactt acttattctt ccgtcacata tacaaaagca 1680
   agacttccag gtagggccag ctcacaagcc caggctggag atcctaactg agaattttct 1740
   acctgtgttc attcttaccg agaaaaggag aaaggagctc tgaatctgat aggaaaagaa 1800
   ggctgcctaa ggaggagttt ttagtatgtg gcgtatcatg caagtgctat gccaagccat 1860
   gtctaaatgg ctttaattat atagtaatgc actctcagta atgggggacc agcttaagta 1920
   taattaatag atggttagtg gggtaattct gcttctagta ttttttttac tgtgcataca 1980
   tgttcatcgt atttccttgg atttctgaat ggctgcagtg acccagatat tgcactaggt 2040
   caaaacattc aggtatagct gacatctcct ctatcacatt acatcatcct ccttataagc 2100
   ccagctctgc tttttccaga ttcttccact ggctccacat ccaccccact ggatcttcag 2160
   aaggctagag ggcgactctg gtggtgcttt tgtatgtttc aattaggctc tgaaatcttg 2220
   ggcaaaatga caaggggagg gccaggattc ctctctcagg tcactccagt gttactttta 2280
   attcctagag ggtaaatatg actcctttct ctatcccaag ccaaccaaga gcacattctt 2340
   aaaggaaaag tcaacatctt ctctcttttt tttttttttt gagacagggt ctcactatgt 2400
   tgcccaggct gctcttgaat tcctgggctc aagcagtcct cccaccctac cacagcgtcc 2460
   cgcgtagctg gcatacaggt gcaagccact atgtccagct agccaactcc tccttgcctg 2520
   cttttctttt tttttctttt tttgagacgg cgcacctatc acccaggctg gagtggagtg 2580
   gcacgatctt ggctcactgc aacctcttcc tcctggttca agcgattctc atgtctcagc 2640
   ctcctcagta gctaggacta ccggcgtgca ccaccatgcc aggctaattt ttatattttt 2700
   agaattttag aagagatggg atttcatcat gttggccagg ctggtctcga actcctgacc 2760
   tcaagtgatc cacctgcctt ggcctcccaa ggtgctagga ttacaggcat gagccaccgc 2820
   accgggccct ccttgcctgt ttttcaatct catctgatat gcagagtatt tctgccccac 2880
   ccacctaccc cccaaaaaaa gctgaagcct atttatttga aagtccttgt ttttgctact 2940
   aattatatag tataccatac attatcattc aaaacaacca tcctgctcat aacatctttg 3000
   aaaagaaaaa tatatatgtg cagtatttta ttaaagcaac attttattta agaataaagt 3060
   cttgttaatt actatatttt agatgcaatg tgatc 3095
<210> 19
   <211> 2128
   <212> DNA
   <213> Homo sapiens
<400> 19
   gggggtccca tcgggcccgc cctcgcacgt cactccggga cccccgcggc ctccgcaggt 60
   tctgcgctcc aggccggagt cagagactcc aggatcggtt ctttcatctt cgccgcccct 120
   gcgcgtccag ctcttctaag acgagatgcc gtcgggcttc caacagatag gctccgaaga 180
   tggggaaccc cctcagcagc gagtgactgg gaccctggtc cttgctgtgt tctctgcggt 240
   gcttggctcc ctgcagtttg ggtacaacat tggggtcatc aatgcccctc agaaggtgat 300
   tgaacagagc tacaatgaga cgtggctggg gaggcagggg cctgagggac ccagctccat 360
   ccctccaggc accctcacca ccctctgggc cctctccgtg gccatctttt ccgtgggcgg 420
   catgatttcc tccttcctca ttggtatcat ctctcagtgg cttggaagga aaagggccat 480
   gctggtcaac aatgtcctgg cggtgctggg gggcagcctc atgggcctgg ccaacgctgc 540
   tgcctcctat gaaatgctca tccttggacg attcctcatt ggcgcctact cagggctgac 600
   atcagggctg gtgcccatgt acgtggggga gattgctccc actcacctgc ggggcgccct 660
   ggggacgctc aaccaactgg ccattgttat cggcattctg atcgcccagg tgctgggctt 720
   ggagtccctc ctgggcactg ccagcctgtg gccactgctc ctgggcctca cagtgctacc 780
   tgccctcctg cagctggtcc tgctgccctt ctgtcccgag agcccccgct acctctacat 840
   catccagaat ctcgaggggc ctgccagaaa gagtctgaag cgcctgacag gctgggccga 900
   tgtttctgga gtgctggctg agctgaagga tgagaagcgg aagctggagc gtgagcggcc 960
   actgtccctg ctccagctcc tgggcagccg tacccaccgg cagcccctga tcattgcggt 1020
   cgtgctgcag ctgagccagc agctctctgg catcaatgct gttttctatt attcgaccag 1080
   catcttcgag acagcagggg taggccagcc tgcctatgcc accataggag ctggtgtggt 1140
   caacacagtc ttcaccttgg tctcggtgtt gttggtggag cgggcggggc gccggacgct 1200
   ccatctcctg ggcctggcgg gcatgtgtgg ctgtgccatc ctgatgactg tggctctgct 1260
   cctgctggag cgagttccag ccatgagcta cgtctccatt gtggccatct ttggcttcgt 1320
   ggcatttttt gagattggcc ctggccccat tccttggttc atcgtggccg agctcttcag 1380
   ccagggaccc cgcccggcag ccatggctgt ggctggtttc tccaactgga cgagcaactt 1440
   catcattggc atgggtttcc agtatgttgc ggaggctatg gggccctacg tcttccttct 1500
   atttgcggtc ctcctgctgg gcttcttcat cttcaccttc ttaagagtac ctgaaactcg 1560
   aggccggacg tttgaccaga tctcagctgc cttccaccgg acaccctctc ttttagagca 1620
   ggaggtgaaa cccagcacag aacttgagta tttagggcca gatgagaacg actgaggggc 1680
   caggcagggg tgggagagcc agctctctct acccggccca gagacccctt cctttcctct 1740
   gcagcacttt aaccctctct tccctattat ttccgggtgg aaaagaatcc ctgcagcctg 1800
   gtagaattgg gaagctgggg gaagggtggt ctgagcaccc cctcattccc ctcgtgtgac 1860
   tctcttggat tatttatgtg ttgtggtttg gccgtggcca tcagggtggg ccactctccc 1920
   ctccctcttc cttcccccat cccctttcct ccccaccttc cccagactca gctccagaat 1980
   accttcttcg ctgctagaga agggggattg gagggaagac aggtctagac tttctcagtg 2040
   ggacaaacca gagcagagag caggacagga gacaagaaat ccagtttccc accaccttgg 2100
   actcctccca caatctggga ctttcact 2128
<210> 20
   <211> 1496
   <212> DNA
   <213> Homo sapiens
<400> 20
   ggcacgagga aaatcaagat aaaaatgttc acaattaagc tccttctttt tattgttcct     60
   ctagttattt cctccagaat tgatcaagac aattcatcat ttgattctct atctccagag    120
   ccaaaatcaa gatttgctat gttagacgat gtaaaaattt tagccaatgg cctccttcag    180
   ttgggacatg gtcttaaaga ctttgtccat aagacgaagg gccaaattaa tgacatattt    240
   caaaaactca acatatttga tcagtctttt tatgatctat cgctgcaaac cagtgaaatc    300
   aaagaagaag aaaaggaact gagaagaact acatataaac tacaagtcaa aaatgaagag    360
   gtaaagaata tgtcacttga actcaactca aaacttgaaa gcctcctaga agaaaaaatt    420
   ctacttcaac aaaaagtgaa atatttagaa gagcaactaa ctaacttaat tcaaaatcaa    480
   cctgaaactc cagaacaccc agaagtaact tcacttaaaa cttttgtaga aaaacaagat    540
   aatagcatca aagaccttct ccagaccgtg gaagaccaat ataaacaatt aaaccaacag    600
   catagtcaaa taaaagaaat agaaaatcag ctcagaagga ctagtattca agaacccaca    660
   gaaatttctc tatcttccaa gccaagagca ccaagaacta ctccctttct tcagttgaat    720
   gaaataagaa atgtaaaaca tgatggcatt cctgctgaat gtaccaccat ttataacaga    780
   ggtgaacata caagtggcat gtatgccatc agacccagca actctcaagt ttttcatgtc    840
   tactgtgatg ttatatcagg tagtccatgg acattaattc aacatcgaat agatggatca    900
   caaaacttca atgaaacgtg ggagaactac aaatatggtt ttgggaggct tgatggagaa    960
   ttttggttgg gcctagagaa gatatactcc atagtgaagc aatctaatta tgttttacga   1020
   attgagttgg aagactggaa agacaacaaa cattatattg aatattcttt ttacttggga   1080
   aatcacgaaa ccaactatac gctacatcta gttgcgatta ctggcaatgt ccccaatgca   1140
   atcccggaaa acaaagattt ggtgttttct acttgggatc acaaagcaaa aggacacttc   1200
   aactgtccag agggttattc aggaggctgg tggtggcatg atgagtgtgg agaaaacaac   1260
   ctaaatggta aatataacaa accaagagca aaatctaagc cagagaggag aagaggatta   1320
   tcttggaagt ctcaaaatgg aaggttatac tctataaaat caaccaaaat gttgatccat   1380
   ccaacagatt cagaaagctt tgaatgaact gaggcaaatt taaaaggcaa taatttaaac   1440
   attaacctca ttccaagtta atgtggtcta ataatctggt attaaatcct taagag       1496
<210> 21
   <211> 1415
   <212> DNA
   <213> Homo sapiens
<400> 21
   ggaggaggga gagagagaga agagaagaaa aagaaaaaag aacatcaata aaaagaagtc     60
   agatttgttc gaaatcttga ggagtcttca ggccagctcc ctgtcggatg gcttttatga    120
   aaaaatatct cctccccatt ctggggctct tcatggccta ctactactat tctgcaaacg    180
   aggaattcag accagagatg ctccaaggaa agaaagtgat tgtcacaggg gccagcaaag    240
   ggatcggaag agagatggct tatcatctgg cgaagatggg agcccatgtg gtggtgacag    300
   cgaggtcaaa agaaactcta cagaaggtgg tatcccactg cctggagctt ggagcagcct    360
   cagcacacta cattgctggc accatggaag acatgacctt cgcagagcaa tttgttgccc    420
   aagcaggaaa gctcatggga ggactagaca tgctcattct caaccacatc accaacactt    480
   ctttgaatct ttttcatgat gatattcacc atgtgcgcaa aagcatggaa gtcaacttcc     540
   tcagttacgt ggtcctgact gtagctgcct tgcccatgct gaagcagagc aatggaagca    600
   ttgttgtcgt ctcctctctg gctgggaaag tggcttatcc aatggttgct gcctattctg    660
   caagcaagtt tgctttggat gggttcttct cctccatcag aaaggaatat tcagtgtcca    720
   gggtcaatgt atcaatcact ctctgtgttc ttggcctcat agacacagaa acagccatga    780
   aggcagtttc tgggatagtc catatgcaag cagctccaaa ggaggaatgt gccctggaga    840
   tcatcaaagg gggagctctg cgccaagaag aagtgtatta tgacagctca ctctggacca    900
   ctcttctgat cagaaatcca tgcaggaaga tcctggaatt tctctactca acgagctata    960
   atatggacag attcataaac aagtaggaac tccctgaggg ctgggcatgc tgagggattt   1020
   tgggactgtt ctgtctcatg tttatctgag ctcttatcta tgaagacatc ttcccagagt   1080
   gtccccagag acatgcaagt catgggtcac acctgacaaa tggaaggagt tcctctaaca   1140
   tttgcaaaat ggaaatgtaa taataatgaa tgtcatgcac cgctgcagcc agcagttgta   1200
   aaattgttag taaacatagg tataattacc agatagttat attaaattta tatcttatat   1260
   ataataatat gtgatgatta atacaatatt aattataata aaggtcacat aaactttata   1320
   aattcataac tggtagctat aacttgagct tattcaggat ggtttcttta aaaccataaa   1380
   ctgtacaaat gaaatttttc aatatttgtt tctta                              1415
<210> 22
   <211> 1405
   <212> DNA
   <213> Homo sapiens
<400> 22
   acaattcaga ggctgctgcc tgcttaggag gttgtagaaa gctctgtagg ttctctctgt      60
   gtgtcctaca ggagtcttca ggccagctcc ctgtcggatg gcttttatga aaaaatatct     120
   cctccccatt ctggggctct tcatggccta ctactactat tctgcaaacg aggaattcag     180
   accagagatg ctccaaggaa agaaagtgat tgtcacaggg gccagcaaag ggatcggaag     240
   agagatggct tatcatctgg cgaagatggg agcccatgtg gtggtgacag cgaggtcaaa     300
   agaaactcta cagaaggtgg tatcccactg cctggagctt ggagcagcct cagcacacta     360
   cattgctggc accatggaag acatgacctt cgcagagcaa tttgttgccc aagcaggaaa     420
   gctcatggga ggactagaca tgctcattct caaccacatc accaacactt ctttgaatct     480
   ttttcatgat gatattcacc atgtgcgcaa aagcatggaa gtcaacttcc tcagttacgt     540
   ggtcctgact gtagctgcct tgcccatgct gaagcagagc aatggaagca ttgttgtcgt     600
   ctcctctctg gctgggaaag tggcttatcc aatggttgct gcctattctg caagcaagtt     660
   tgctttggat gggttcttct cctccatcag aaaggaatat tcagtgtcca gggtcaatgt     720
   atcaatcact ctctgtgttc ttggcctcat agacacagaa acagccatga aggcagtttc     780
   tgggatagtc catatgcaag cagctccaaa ggaggaatgt gccctggaga tcatcaaagg     840
   gggagctctg cgccaagaag aagtgtatta tgacagctca ctctggacca ctcttctgat     900
   cagaaatcca tgcaggaaga tcctggaatt tctctactca acgagctata atatggacag     960
   attcataaac aagtaggaac tccctgaggg ctgggcatgc tgagggattt tgggactgtt    1020
   ctgtctcatg tttatctgag ctcttatcta tgaagacatc ttcccagagt gtccccagag    1080
   acatgcaagt catgggtcac acctgacaaa tggaaggagt tcctctaaca tttgcaaaat    1140
   ggaaatgtaa taataatgaa tgtcatgcac cgctgcagcc agcagttgta aaattgttag    1200
   taaacatagg tataattacc agatagttat attaaattta tatcttatat ataataatat    1260
   gtgatgatta atacaatatt aattataata aaggtcacat aaactttata aattcataac    1320
   tggtagctat aacttgagct tattcaggat ggtttcttta aaaccataaa ctgtacaaat    1380
   gaaatttttc aatatttgtt tctta                                          1405
<210> 23
   <211> 1944
   <212> DNA
   <213> Homo sapiens
<400> 23
   ccctctcgcg ccccaggccg gtgtaccccc gcactccgcg ccccggccta gaagctctct      60
   ctccccgctc cccggcccgg cccccgcccc gccccgcccc agcccgctgg gccgccatgg     120
   agcgctggcc ttggccgtcg ggcggcgcct ggctgctcgt ggctgcccgc gcgctgctgc     180
   agctgctgcg ctcagacctg cgtctgggcc gcccgctgct ggcggcgctg gcgctgctgg     240
   ccgcgctcga ctggctgtgc cagcgcctgc tgcccccgcc ggccgcactc gccgtgctgg    300
   ccgccgccgg ctggatcgcg ttgtcccgcc tggcgcgccc gcagcgcctg ccggtggcca    360
   ctcgcgcggt gctcatcacc ggctgtgact ctggttttgg caaggagacg gccaagaaac    420
   tggactccat gggcttcacg gtgctggcca ccgtattgga gttgaacagc cccggtgcca    480
   tcgagctgcg tacctgctgc tcccctcgcc taaggctgct gcagatggac ctgaccaaac    540
   caggagacat tagccgcgtg ctagagttca ccaaggccca caccaccagc accggcctgt    600
   ggggcctcgt caacaacgca ggccacaatg aagtagttgc tgatgcggag ctgtctccag    660
   tggccacttt ccgtagctgc atggaggtga atttctttgg cgcgctcgag ctgaccaagg    720
   gcctcctgcc cctgctgcgc agctcaaggg gccgcatcgt gactgtgggg agcccagcgg    780
   gggacatgcc atatccgtgc ttgggggcct atggaacctc caaagcggcc gtggcgctac    840
   tcatggacac attcagctgt gaactccttc cctggggggt caaggtcagc atcatccagc    900
   ctggctgctt caagacagag tcagtgagaa acgtgggtca gtgggaaaag cgcaagcaat    960
   tgctgctggc caacctgcct caagagctgc tgcaggccta cggcaaggac tacatcgagc   1020
   acttgcatgg gcagttcctg cactcgctac gcctggccat gtccgacctc accccagttg   1080
   tagatgccat cacagatgcg ctgctggcag ctcggccccg ccgccgctat taccccggcc   1140
   agggcctggg gctcatgtac ttcatccact actacctgcc tgaaggcctg cggcgccgct   1200
   tcctgcaggc cttcttcatc agtcactgtc tgcctcgagc actgcagcct ggccagcctg   1260
   gcactacccc accacaggac gcagcccagg gcccaaacct gagccccggc ccttccccag   1320
   cagtggctcg gtgagccatg tgcacctatg gcccagccac tgcagcacag gaggctccgt   1380
   gagcccttgg ttcctccccg aaaaccccca gcattacgat cccccaagtg tcctggaccc   1440
   tggcctaaag aatcccaccc ccacttcatg cccactgccg atgcccaatc caggcccggt   1500
   gaggccaagg tttcccagtg agcctctgcg cctctccact gtttcatgag cccaaacacc   1560
   ctcctggcac aacgctctac cctgcagctt ggagaactcc gctggatggg gagtctcatg   1620
   caagacttca ctgcagcctt tcacaggact ctgcagatag tgcctctgca aactaaggag   1680
   tgactaggtg ggttggggac cccctcagga ttgtttctcg gcaccagtgc ctcagtgctg   1740
   caattgaggg ctaaatccca agtgtctctt gactggctca agaattaggg ccccaactac   1800
   acacccccaa gccacaggga agcatgtact gtacttccca attgccacat tttaaataaa   1860
   gacaaatttt tatttcttct aaaaaaaaaa aaaaaaaaaa aaaaaaaaaa aaaaaaaaaa   1920
   aaaaaaaaaa aaaaaaaaaa aaaa                                          1944

## Description

Liver X Receptor (LXR) is a prototypical type 2 nuclear receptor which activates genes upon binding to promoter region of target genes in a prototypical heterodimeric fashion with Retinoid X Receptor (hereinafter RXR, Forman et al., Cell, 81, 687-93, 1995). The term LXR includes all subtypes of this receptor. Specifically LXR includes LXRa (also known as LXRalpha, RLD-1 and NR1H3) and LXRb (also known as LXRbeta, NER, NER1, UR, OR-1, R1P15 and NH1H2) and ligands of LXR should be understood to include ligands of LXRa or LXRb. The relevant physiological ligands of LXR seem to be oxidized derivatives of cholesterol, including 22-hydroxycholesterol and 24,25(S)-epoxycholesterol (Lehmann, et al., Biol. Chem. 272(6), 3137-40, 1997). The oxysterol ligands bound to LXR were found to regulate the expression of several genes that participate in cholesterol metabolism (Janowski, et al., Nature, 383, 728-31, 1996).

LXR is proposed to be a hepatic oxysterol sensor. Upon activation (e.g. binding of oxysterols) it influences the conversion of dietary cholesterol into bile acids by upregulating the transcription of key genes which are involved in bile acid synthesis such as CYP7A1. Hence, activation of LXR in the liver could result in an increased synthesis of bile acids from cholesterol which could lead to decreased levels of hepatic cholesterol. This proposed LXR function in hepatic cholesterol metabolism was experimentally confirmed using knockout mice. Mice lacking the receptor LXRa lost their ability to respond normally to an increase in dietary cholesterol and did not induce transcription of the gene encoding CYP7A1. This resulted in accumulation of large quantities of cholesterol in the livers and impaired hepatic function (Peet, et al., Cell, 93, 693-704, 1998).

Besides its important function in liver, LXR plays an important role in the regulation of cholesterol homeostasis in macrophages and intestinal mucosa cells where it upregulates cholesterol transporters from the ABC (=ATP binding cassette) family of membrane proteins (Repa, et al., J Biol Chem. 2002 May 24;277(21):18793-800).

These transporters are believed to be crucially involved in the uptake of cholesterol from the diet since mutations in their genes leads to diseases such as sitosterolemia (Berge, et al., Science (2000);290(5497):1771-5.).

Other members of the ABC transporter family seem to be responsible for the efflux of cholesterol from loaded macrophages, a process which is thought to prevent the generation of atherosclerotic lesions. Stimulation of LXR by synthetic ligands might result in an increased cholesterol efflux from macrophages and a decreased building up of cholesterol loaded atherosclerotic plaques (Venkateswaran, et al., PNAS (2000) 24;97(22):12097-102; Sparrow, et al., J Biol Chem (2002) 277(12):10021-7; Joseph, et al., PNAS (2002);99(11):7604-9). Direct evidence that synthetic LXR ligands inhibit the development of atherosclerosis has been provided in two animal models of atherosclerosis: A significant reduction in the formation of atherosclerotic plaques were shown in two studies in animal models using full LXR agonists Joseph et al. PNAS (2002) 99:7604-9 and Terasaka et al. (2003) Terasaka et al. FEBS Lett. (2003) 536:6-11. In addition, two recent reports have highlighted the potential use of LXR agonists in diabetes (Cao et al., (2003) J Biol Chem. 278:1131-6 and inflammatory disorders (Joseph et al., (2003) Nat Med. 9:213-9.

However, in animal studies it was observed that activation of LXR in the liver by full agonists like T0901317 does not only increase bile acid synthesis but also stimulates the de novo synthesis of fatty acids and triglycerids through the upregulation of key enzymes such as Fatty Acid Synthase (FAS) or Stearyl-CoA Desaturase (SCD-1) (Schultz, et al., Genes Dev (2000) 14(22):2831-8). Elevation of serum triglyceride levels is an indendent risk factor for atherosclerosis (for review see Miller (1999) Hosp Pract (Off Ed) 34: 67-73.).

Thus, LXR activity needs to be selectively modulated for therapeutic benefit. In particular, compounds need to be found that stimulate reverse cholesterol transport, but do not significantly increase trigclyceride levels. This might be particular relevant for the usage of such compounds in diabetic patients since a even more severe lopogenic effect was reported for the full agonist T0901317 in db/db mice which serve as an animal model for diabetes (Chisholm et al. (2003) J.Lipid Res (epub August 16)).

Therefore, an ideal synthetic LXR binding compound should have properties that retain the agonistic activity on hepatic bile acid formation and ABC-transporter - mediated decrease in cholesterol uptake from the diet and increased cholesterol efflux from macrophages. In parallel such a compound should lack the hyperlipidemic potential which is exerted through increased fatty acid and triclyceride synthesis.

To date only few compounds have been described which bind the LXR receptor and thus show utility for treating diseases or conditions which are due to or influenced by said nuclear receptor (Collins, et al., J Med Chem. (2002) 45(10):1963-6; Schultz, et al., Genes Dev (2000) 14(22):2831-8; Sparrow, et al., J Biol Chem (2002) 277(12):10021-7). No non-steroidal compounds have so far been described which show selectivity regarding the induction of ABC transporter genes without simultaneous induction of lipogenic genes like FAS and SREBP-1c (Kaneko et al. (2003) J Biol Chem (epub July 7).

It is thus an object of the invention to provide for compounds which by means of binding the LXR receptor act as partial agonists of said receptor with a selective property regarding the upregulation of genes like the ABC transporters in macrophages and/or other cell types and a stronlgy reduced liability to increase the expression of genes involved in triglyceride synthetic pathways (like FAS and SREBP-1c). These compounds should show utility for treating diseases or conditions which are due to or influenced by said nuclear receptor.

It is further an object of the invention to provide for compounds that may be used for the manufacture of a medicament for the treatment of cholesterol associated conditions or diseases. It is still a further object of the invention to provide for compounds that lower serum cholesterol and/or increase High Density Lipoproteins (HDL) and/or decrease Low Density Lipoproteins (LDL). It is also an object of the invention to provide for compounds that may be used for the treatment of lipid disorders including hypercholesterolemia, atherosclerosis, Alzheimer's disease, skin disorders, inflammation, obesity and diabetes.

The present invention provides, *inter alia,* novel LXR nuclear receptor protein binding compounds according to the general formula (I) shown below. Said compounds are also binders of mammalian homologues of said receptor. Further the object of the invention was solved by providing for amongst the LXR nuclear receptor protein binding compounds according to the general formula (I) such compounds which act as partial agonists or mixed agonists / antagonists of the human LXR receptor or a mammalian homologue thereof. Further the object of the invention was solved by providing for amongst the LXR receptor protein binding compounds according to the general formula (I) such compounds which act as partial agonists of the human LXR receptor resulting therefore in the induction of ABC transporter proteins such as ABCA1 or ABCG1 in cell types such as macrophages but lacking a strong potential to induce genes involved in triglyceride synthetic pathways such as fatty acid synthase (FAS) or SREBP1c.

The invention provides for LXR agonists that may be used for the manufacture of a medicament for the treatment of cholesterol associated conditions or diseases. In a preferred embodiment compounds are provided that lower serum cholesterol and/or increase High Density lipoproteins (HDL) and/or decrease Low Density Lipoproteins (LDL). Also compounds are provided that may be used for the treatment of lipid disorders including hypercholesterolemia, atherosclerosis, Alzheimer's disease, skin disorders, inflammation, obesity and diabetes.

The invention provides for a compound of the formula (I), or pharmaceutical acceptable salts or solvates thereof, hereinafter also referred to as the "compounds according to the invention" including particular and preferred embodiments thereof.

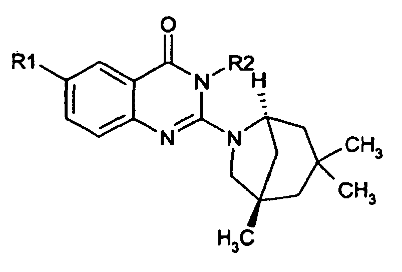

The compounds of the invention can also exist as solvates and hydrates. Thus, these compounds may crystallize with, for example, waters of hydration, or one, a number of, or any fraction thereof of molecules of the mother liquor solvent. The solvates and hydrates of such compounds are included within the scope of this invention.

In one embodiment of the invention in formula (I) above, R₁ is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl,
and R₂ is H, C₁ to C₈ alkyl, C₁ to C₆ substituted alkyl, C₇ to C₁₂ alkylphenyl or C₇ to C₁₂ substituted phenylalkyl.

In an other embodiment of the invention in formula (I) above R₁ is halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected cartioxamide, N-(C₁ to C₆ alkyl)carboxamide, protected N-(C₁ to C₆ alkyl)carboxamide, N, N-di(C₁ to C₆ alkyl)carboxamide, trifluoromethyl, N-(C₁ to C₆ alkyl)sulfonyl)amino, N- (phenylsulfonyl)amino or phenyl,
and R₂ is H, C₁ to C₈ alkyl, C₁ to C₈ substituted alkyl.

The symbol "H" denotes a hydrogen atom.

The term "C₁ to C₇ acyl" encompasses groups such as formyl, acetyl, propionyl, butyryl, pentanoyl, pivaloyl, hexanoyl, heptanoyl, benzoyl and the like. Preferred acyl groups are acetyl and benzoyl.

The term "C₁ to C₇ substituted acyl" denotes the acyl group substituted by one or more, and preferably one or two, halogen, hydroxy, protected hydroxy, oxo, protected oxo, cyclohexyl, naphthyl, amino, protected amino, (monosubstituted)amino, protected (monosubstituted)amino, (disubstituted)amino, guanidino, heterocyclic ring, substituted heterocyclic ring, imidazolyl, indolyl, pyrrolidinyl, C₁ to C₇ alkoxy, C₁ to C₇ acyl, C₁ to C₇ acyloxy, nitro, C₁ to C₆ alkyl ester, carboxy, protected carboxy, carbamoyl, carboxamide, protected carboxamide, N-(C₁ to C₆ alkyl)carboxamide, protected N-(C₁ to C₆ alkyl)carboxamide, N,N-di(C₁ to C₆ alkyl)carboxamide, cyano, methylsulfonylamino, thiol, C₁ to C₄ alkylthio or C₁ to C₄ alkylsulfonyl groups. The substituted acyl groups may be substituted once or more, and preferably once or twice, with the same or with different substituents.

The term "substituted phenyl" specifies a phenyl group substituted with one or more, and preferably one or two, moieties chosen from the groups consisting of halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, wherein the phenyl is substituted or unsubstituted, such that, for example, a biphenyl results.

Examples of the term "substituted phenyl" includes a mono- or di(halo)phenyl group such as 2, 3 or 4-chlorophenyl, 2,6-dichlorophenyl, 2,5-dichlorophenyl, 3,4-dichlorophenyl, 2, 3 or 4-bromophenyl, 3,4-dibromophenyl, 3-chloro-4-fluorophenyl, 2, 3 or 4-fluorophenyl and the like; a mono or di(hydroxy)phenyl group such as 2, 3 or 4-hydroxyphenyl, 2,4-dihydroxyphenyl, the protected-hydroxy derivatives thereof and the like; a nitrophenyl group such as 2, 3 or 4-nitrophenyl; a cyanophenyl group, for example, 2, 3 or 4-cyanophenyl; a mono- or di(alkyl)phenyl group such as 2, 3 or 4-methylphenyl, 2,4-dimethylphenyl, 2, 3 or 4-(iso-propyl)phenyl, 2, 3 or 4-ethylphenyl, 2, 3 or 4-(n-propyl)phenyl and the like; a mono or di(alkoxyl)phenyl group, for example, 2,6-dimethoxyphenyl, 2, 3 or 4-methoxyphenyl, 2, 3 or 4-ethoxyphenyl, 2, 3 or 4-(isopropoxy)phenyl, 2, 3 or 4-(t-butoxy)phenyl, 3-ethoxy-4-methoxyphenyl and the like; 2, 3 or 4-trifluoromethylphenyl; a mono- or dicarboxyphenyl or (protected carboxy)phenyl group such as 2, 3 or 4-carboxyphenyl or 2,4-di(protected carboxy)phenyl; a mono-or di(hydroxymethyl)phenyl or (protected hydroxymethyl)phenyl such as 2, 3, or 4-(protected hydroxymethyl)phenyl or 3,4-di(hydroxymethyl)phenyl; a mono- or di(aminomethyl)phenyl or (protected aminomethyl)phenyl such as 2, 3 or 4-(aminomethyl)phenyl or 2,4-(protected aminomethyl)phenyl; or a mono- or di(N-(methylsulfonylamino))phenyl such as 2, 3 or 4-(N-(methylsulfonylamino))phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy 4-chlorophenyl and the like.

The term "heteroaryl" means a heterocyclic aromatic derivative which is a five-membered or six-membered ring system having from 1 to 4 heteroatoms, such as oxygen, sulfur and/or nitrogen, in particular nitrogen, either alone or in conjunction with sulfur or oxygen ring atoms. Examples of heteroaryls include pyridinyl, pyrimidinyl, and pyrazinyl, pyridazinyl, pyrrolo, furano, thiopheno, oxazolo, isoxazolo, phthalimido, thiazolo and the like.

The term "substituted heteroaryl" means the above-described heteroaryl is substituted with, for example, one or more, and preferably one or two, substituents which are the same or different which substituents can be halogen, hydroxy, protected hydroxy, cyano, nitro, C₁ to C₆ alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino or N-(phenylsulfonyl)amino groups.

The term "substituted naphthyl" specifies a naphthyl group substituted with one or more, and preferably one or two, moieties either on the same ring or on different rings chosen from the groups consisting of halogen, hydroxy, protected hydroxy, cyano, nitro, C₁ to C₆ alkyl, C₁ to C₇ alkoxy, C₁ to C₇ acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino or N-(phenylsulfonyl)amino.

Examples of the term "substituted naphthyl" includes a mono or di(halo)naphthyl group such as 1, 2, 3, 4, 5, 6, 7 or 8-chloronaphthyl, 2, 6-dichloronaphthyl, 2, 5-dichloronaphthyl, 3, 4-dichloronaphthyl, 1, 2, 3, 4, 5, 6, 7 or 8-bromonaphthyl, 3, 4-dibromonaphthyl, 3-chloro-4-fluoronaphthyl, 1, 2, 3, 4, 5, 6, 7 or 8-fluoronaphthyl and the like; a mono or di(hydroxy)naphthyl group such as 1, 2, 3, 4, 5, 6, 7 or 8-hydroxynaphthyl, 2, 4-dihydroxynaphthyl, the protected-hydroxy derivatives thereof and the like; a nitronaphthyl group such as 3- or 4-nitronaphthyl; a cyanonaphthyl group, for example, 1, 2, 3, 4, 5, 6, 7 or 8-cyanonaphthyl; a mono- or di(alkyl)naphthyl group such as 2, 3, 4, 5, 6, 7 or 8-methylnaphthyl, I, 2, 4-dimethylnaphthyl, 1, 2, 3, 4, 5, 6, 7 or 8-(isopropyl)naphthyl, 1, 2, 3, 4, 5, 6, 7 or 8-ethylnaphthyl, 1, 2, 3, 4, 5, 6, 7 or 8-(n-propyl)naphthyl and the like; a mono or di(alkoxy)naphthyl group, for example, 2, 6-dimethoxynaphthyl, 1, 2, 3, 4, 5, 6, 7 or 8-methoxynaphthyl, 1, 2, 3, 4, 5, 6, 7 or 8-ethoxynaphthyl, 1, 2, 3, 4, 5, 6, 7 or 8-(isopropoxy)naphthyl, 1, 2, 3, 4, 5, 6, 7 or 8-(t-butoxy)naphthyl, 3-ethoxy-4-methoxynaphthyl and the like; 1, 2, 3, 4, 5, 6, 7 or 8-trifluoromethylnaphthyl; a mono-or dicarboxynaphthyl or (protected carboxy)naphthyl group such as 1, 2, 3, 4, 5, 6, 7 or 8-carboxynaphthyl or 2, 4-di(-protected carboxy)naphthyl; a mono-or di(hydroxymethyl)naphthyl or (protected hydroxymethyl)naphthyl such as 1, 2, 3, 4, 5, 6, 7 or 8-(protected hydroxymethyl)naphthyl or 3, 4-di(hydroxymethyl)naphthyl; a mono- or di(amino)naphthyl or (protected amino)naphthyl such as 1, 2, 3, 4, 5, 6, 7 or 8-(amino)naphthyl or 2, 4-(protected amino)-naphthyl, a mono- or di(aminomethyl)naphthyl or (protected aminomethyl)naphthyl such as 2, 3, or 4-(aminomethyl)naphthyl or 2, 4-(protected aminomethyl)-naphthyl; or a mono- or di-(N-methylsulfonylamino) naphthyl such as 1, 2, 3, 4, 5, 6, 7 or 8-(N-methylsulfonylamino)naphthyl. Also, the term "substituted naphthyl" represents disubstituted naphthyl groups wherein the substituents are different, for example, 3-methyl-4-hydroxynaphth-1-yl, 3-chloro-4-hydroxynaphth-2-yl, 2-methoxy-4-bromonaphth-1-yl, 4-ethyl-2-hydroxynaphth-1-yl, 3-hydroxy-4-nitronaphth-2-yl, 2-hydroxy-4-chloronaphth-1-yl, 2-methoxy-7-bromonaphth-1-yl, 4-ethyl-5-hydroxynaphth-2-yl, 3-hydroxy-8-nitronaphth-2-yl, 2-hydroxy-5-chloronaphth-1-yl and the like.

The term "C₁ to C₈ alkyl" denotes such radicals as methyl, ethyl, n-propyl, isopropyl, n-butyl, iso-butyl, sec-butyl, tert-butyl, amyl, tert-amyl, hexyl , n-heptyl, 2-heptyl, 3-heptyl, 4-heptyl, 2-methyl-1 hexyl, 2-methyl-2hexyl, 2-methyl-3-hexyl, n-octyl and the like.

The term "C₂ to C₆ alkenyl" denotes such radicals as propenyl or butenyl.

Examples of the above substituted alkyl groups include the 2-oxo-prop-1-yl, 3-oxo-but-1-yl, cyanomethyl, nitromethyl, chloromethyl, hydroxymethyl, tetrahydropyranyloxymethyl, trityloxymethyl, propionyloxymethyl, amino, methylamino, aminomethyl, dimethylamino, carboxymethyl, allyloxycarbonylmethyl, allyloxycarbonylaminomethyl, methoxymethyl, ethoxymethyl, t-butoxymethyl, acetoxymethyl, chloromethyl, bromomethyl, iodomethyl, trifluoromethyl, 6-hydroxyhexyl, 2,4-dichloro(n-butyl), 2-aminopropyl, 1-chloroethyl, 2-chloroethyl, 1-bromoethyl, 2-chloroethyl, 1-fluoroethyl, 2-fluoroethyl, 1- iodoethyl, 2-iodoethyl, 1-chloropropyl, 2-chloropropyl, 3- chloropropyl, 1-bromopropyl, 2-bromopropyl, 3-bromopropyl, 1-fluoropropyl, 2-fluoropropyl, 3-fluoropropyl, 1- iodopropyl, 2-iodopropyl, 3-iodopropyl, 2-aminoethyl, 1- aminoethyl, N-benzoyl-2-aminoethyl, N-acetyl-2-aminoethyl, N-benzoyl-1-aminoethyl, N-acetyl-1-aminoethyl and the like.

The term "C₁ to C₈ substituted alkyl" denotes that the above C₁ to C₈ alkyl groups are substituted by one or more, and preferably one or two, halogen, hydroxy, protected hydroxy, oxo, protected oxo, C₃ to C₇ cycloalkyl, naphthyl, amino, protected amino, (monosubstituted)amino, protected (monosubstituted)amino, (disubstituted)amino, guanidino, protected guanidino, heterocyclic ring, substituted heterocyclic ring, imidazolyl, indolyl, pynrolidinyl, C₁ to C₇ alkoxy, C₁ to C₇ acyl, C₁ to C₇ acyloxy, nitro, carboxy, protected carboxy, carbamoyl, carboxamide, protected carboxamide, N-(C₁ to C₆ alkyl)carboxamide, protected N-(C₁ to C₆ alkyl)carboxamide, N,N-di(C₁ to C₆ alkyl)carboxamide, cyano, methylsulfonylamino, thiol, C₁ to C₄ alkylthio or C₁ to C₄ alkylsulfonyl groups. The substituted alkyl groups may be substituted once or more, and preferably once or twice, with the same or with different substituents.

The term "C₇ to C₁₂ phenylalkyl" denotes a C₁ to C₆ alkyl group substituted at any position by a phenyl, substituted phenyl.
Examples of such a group include benzyl, 2-phenylethyl, 3-phenyl(n-propyl), 4-phenylhexyl, 3-phenyl(n-amyl), 3-phenyl(sec-butyl) and the like. Preferred C₇ to C₁₂ phenylalkyl groups are the benzyl and the phenylethyl groups.
The term "C₇ to C₁₂ substituted phenylalkyl" denotes a C₇ to C₁₂ phenylalkyl group substituted on the C₁ to C₆ alkyl portion with one or more, and preferably one or two, groups chosen from halogen, hydroxy, protected hydroxy, oxo, protected oxo, amino, protected amino, (monosubstituted)amino, protected (monosubstituted)amino, (disubstituted)amino, guanidino, protected guanidino, heterocyclic ring, substituted heterocyclic ring, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, nitro, carboxy, protected carboxy, carbamoyl, carboxamide, protected carboxamide, N-(C₁ to C₆ alkyl)carboxamide, protected N-(C₁ to C₆ alkyl)carboxamide, N, N-(C₁ to C₆ dialkyl)carboxamide, cyano, N-(C₁ to C₆ alkylsulfonyl)amino, thiol, C₁ to C₄ alkylthio, C₁ to C₄ alkylsulfonyl groups; and/or the phenyl group may be substituted with one or more, and preferably one or two, substituents chosen from halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl) carboxamide, protected N-(C₁ to C₆ alkyl) carboxamide, N, N-di(C₁ to C₆ alkyl)carboxamide, trifluoromethyl, N-((C₁ to C₆ alkyl)sulfonyl)amino, N-(phenylsulfonyl)amino, cyclic C₂ to C₇ alkylene or a phenyl group, substituted or unsubstituted, for a resulting biphenyl group. The substituted alkyl or phenyl groups may be substituted with one or more, and preferably one or two, substituents which can be the same or different.

Examples of the term "C₇ to C₁₂ substituted phenylalkyl" include groups such as 2-phenyl-1-chloroethyl, 2-(4-methoxyphenyl)ethyl, 4-(2,6-dihydroxy phenyl)n-hexyl, 2-(5-cyano-3-methoxyphenyl)n-pentyl, 3-(2,6-dimethylphenyl)n-propyl, 4-chloro-3-aminobenzyl, 6-(4-methoxyphenyl)-3-carboxy(n-hexyl), 5-(4-aminomethylphenyl)- 3-(aminomethyl)n-pentyl, 5-phenyl-3-oxo-n-pent-1-yl and the like.

The term "heterocycle" or "heterocyclic ring" denotes optionally substituted five-membered to eight-membered rings that have 1 to 4 heteroatoms, such as oxygen, sulfur and/or nitrogen, in particular nitrogen, either alone or in conjunction with sulfur or oxygen ring atoms. These five-membered to eight-membered rings may be saturated, fully unsaturated or partially unsaturated, with fully saturated rings being preferred. Preferred heterocyclic rings include morpholino, piperidinyl, piperazinyl, 2-amino-imidazoyl, tetrahydrofurano, pyrrolo, tetrahydrothiophen-yl, hexylmethyleneimino and heptylmethyleneimino.

The term "substituted heterocycle" or "substituted heterocyclic ring" means the above-described heterocyclic ring is substituted with, for example, one or more, and preferably one or two, substituents which are the same or different which substituents can be halogen, hydroxy, protected hydroxy, cyano, nitro, C₁ to C₁₂ alkyl, C₁ to C₁₂ alkoxy, C₁ to C₁₂ substituted alkoxy, C₁ to C₁₂ acyl, C₁ to C₁₂ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino carboxamide, protected carboxamide, N-(C₁ to C₁₂ alkyl)carboxamide, protected N-(C₁ to C₁₂ alkyl)carboxamide, N, N-di(C₁ to C₁₂ alkyl)carboxamide, trifluoromethyl, N-((C₁ to C₁₂ alkyl)sulfonyl)amino, N-(phenylsulfonyl)amino, heterocycle or substituted heterocycle groups.

The term "C₁ to C₈ alkoxy" as used herein denotes groups such as methoxy, ethoxy, n-propoxy, isopropoxy, n-butoxy, t-butoxy and like groups. A preferred alkoxy is methoxy. The term "C₁ to C₈ substituted alkoxy" means the alkyl portion of the alkoxy can be substituted in the same manner as in relation to C₁ to C₈ substituted alkyl.

The term "C₁ to C₈ aminoacyl" encompasses groups such as formyl, acetyl, propionyl, butyryl, pentanoyl, pivaloyl, hexanoyl, heptanoyl, octanoyl, benzoyl and the like.

The term "C₁ to C₈ substituted aminoacyl" denotes the acyl group substituted by one or more, and preferably one or two, halogen, hydroxy, protected hydroxy, oxo, protected oxo, cyclohexyl, naphthyl, amino, protected amino, (monosubstituted)amino, protected (monosubstituted)amino, (disubstituted)amino, guanidino, heterocyclic ring, substituted heterocyclic ring, imidazolyl, indolyl, pyrrolidinyl, C₁ to C₁₂ alkoxy, C₁ to C₁₂ acyl, C₁ to C₁₂ acyloxy, nitro, C₁ to C₁₂ alkyl ester, carboxy, protected carboxy, carbamoyl, carboxamide, protected carboxamide, N-(C₁ to C₁₂ alkyl)carboxamide, protected N-(C₁ to C₁₂ alkyl)carboxamide, N,N-di(C₁ to C₁₂ alkyl)carboxamide, cyano, methylsulfonylamino, thiol, C₁ to C₁₀ alkylthio or C₁ to C₁₀ alkylsulfonyl groups. The substituted acyl groups may be substituted once or more, and preferably once or twice, with the same or with different substituents.

Examples of C₁ to C₈ substituted acyl groups include 4-phenylbutyroyl, 3-phenylbutyroyl, 3-phenylpropanoyl, 2-cyclohexanylacetyl, cyclohexanecarbonyl, 2-furanoyl and 3-dimethylaminobenzoyl.

This invention also provides a pharmaceutical composition comprising an effective amount of a compound according to the invention. Such pharmaceutical compositions can be administered by various routes, for example oral, subcutaneous, intramuscular, intravenous or intracerebral. The preferred route of administration would be oral at daily doses of the compound for adult human treatment of about 0.01-5000 mg, preferably 1-1500 mg per day. The appropriate dose may be administered in a single dose or as divided doses presented at appropriate intervals for example as two, three four or more subdoses per day.

For preparing pharmaceutical compositions containing compounds of the invention, inert, pharmaceutically acceptable carriers are used. The pharmaceutical carrier can be either solid or liquid. Solid form preparations include, for example, powders, tablets, dispersible granules, capsules, cachets, and suppositories.

A solid carrier can be one or more substances which can also act as diluents, flavoring agents, solubilizers, lubricants, suspending agents, binders, or tablet disintegrating agents; it can also be an encapsulating material.

In powders, the carrier is generally a finely divided solid which is in a mixture with the finely divided active component. In tablets, the active compound is mixed with the carrier having the necessary binding properties in suitable proportions and compacted in the shape and size desired.

For preparing pharmaceutical composition in the form of suppositories, a low-melting wax such as a mixture of fatty acid glycerides and cocoa butter is first melted and the active ingredient is dispersed therein by, for example, stirring. The molten homogeneous mixture is then poured into convenient-sized molds and allowed to cool and solidify.

Powders and tablets preferably contain between about 5% to about 70% by weight of the active ingredient, preferably comprising (especially consisting of) one or more of the compounds according to this invention. Suitable carriers include, for example, magnesium carbonate, magnesium stearate, talc, lactose, sugar, pectin, dextrin, starch, tragacanth, methyl cellulose, sodium carboxymethyl cellulose, a low-melting wax, cocoa butter and the like.

The pharmaceutical compositions can include the formulation of the active compound with encapsulating material as a carrier providing a capsule in which the active component (with or without other carriers) is surrounded by a carrier, which is thus in association with it. In a similar manner, cachets are also included. Tablets, powders, cachets, and capsules can be used as solid dosage forms suitable for oral administration.

Liquid pharmaceutical compositions include, for example, solutions suitable for oral or parenteral administration, or suspensions, and emulsions suitable for oral administration. Sterile water solutions of the active component or sterile solutions of the active component in solvents comprising water, ethanol, or propylene glycol are examples of liquid compositions suitable for parenteral administration.

Sterile solutions can be prepared by dissolving the active component in the desired solvent system, and then passing the resulting solution through a membrane filter to sterilize it or, alternatively, by dissolving the sterile compound in a previously sterilized solvent under sterile conditions.

In a preferred embodiment of the invention in the compounds claimed, or the pharmaceutical acceptable salts or solvates thereof, R₁ is halogen and preferably iodine over bromine and chlorine and R₂ is H, C₁ to C₈ alkyl or C₁ to C₈ substituted alkyl.

A particularly preferred compound which may act as a partial agonist of LXR is shown in formula (II) below (MOLNAME TR1040001892). It has been demonstrated that this compound has a low effective concentration at LXR with an EC₅₀ of 2 µM in a FRET assay wherein the EC₅₀ reflects the half-maximal effective concentration, and which is higher than the EC₅₀ of 0.015 µM for the published LXR agonist TO901317 (J. Schultz et al., Genes Dev. 14, 2831-2838, 2000). Compound according to formula (II) does show selective upregulation of ABCA1 and ABCG1 in THP-1 macrophages but does not significantly upregulate FAS and much reduced SREBP-1 c in HepG2 cells (see EXAMPLE 5 further down).

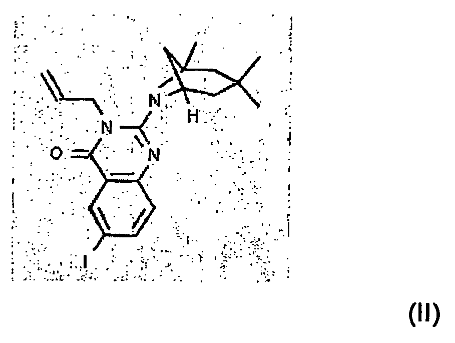

It has also been found that the compound according to formula (III) (Molname TR1040011382) to be active as partial agonist of the LXR human nuclear receptors with a selective upregulation of the target genes ABCA1 and ABCG1 in THP-1 cells compared to FAS and SREBP-1c in HepG2 cells.

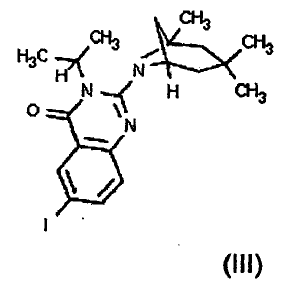

It has also been found that the compounds according to formulas (IV) (Molname TR1040002211) and (V) (Molname TR1040002212) to be active as partial agonist of the LXR human nuclear receptors however with a reduced selectivity regarding the upregulation of the target genes ABCA1 and ABCG1 in THP-1 cells versus FAS and SREBP-1c in HepG2 cells compared to compounds of formula (II) and (III) (see EXAMPLE 5 further down).

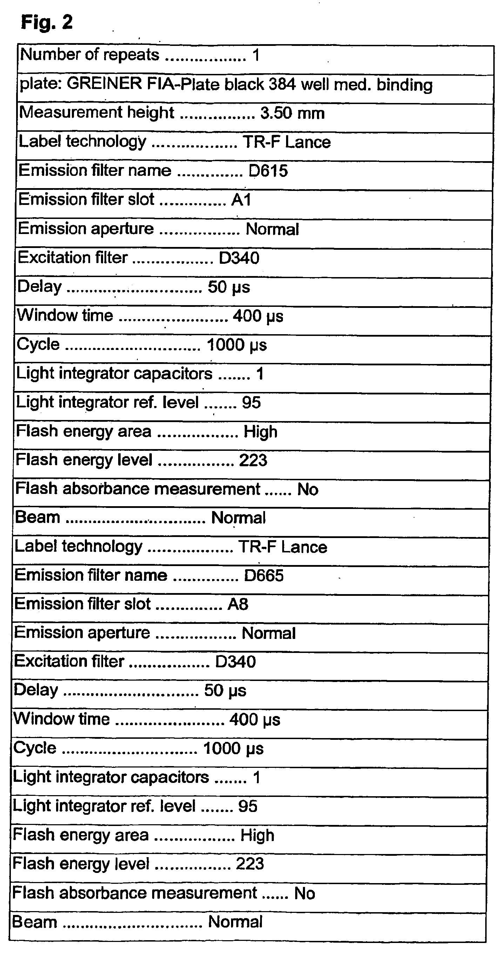

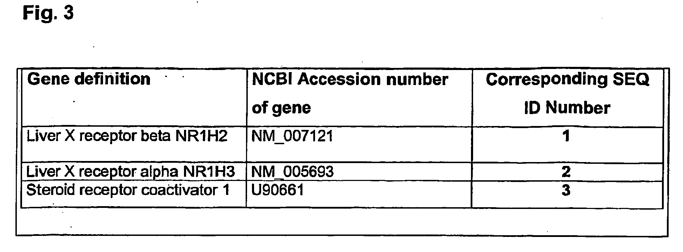

In particular the invention relates to a compound as described above wherein said compounds is capable of binding the LXR receptor protein or a portion thereof encoded by a nucleic acid according to SEQ ID NO:1 or NO:2 (Fig. 3) or a mammalian homologue thereof. This compound can bind to the LXR receptor protein or a portion thereof in a mixture comprising 10-200 ng of LXR receptor protein, a fusion protein containing LXR or a portion thereof, preferably the ligand binding domain, fused to a Tag, 5-100 mM Tris/HCl at pH 6,8-8,3; 60-1000 mM KCI; 0-20 mM MgCl2; 100-1000ng/µl BSA in a total volume of preferably about 25 µl[see also EXAMPLE 1 and Fig.2).

A mammalian receptor protein homologue of the protein encoded by a nucleic acid according to SEQ ID NO:1 or 2, as used herein is a protein that performs substantially the same task as LXR does in humans and shares at least 40% sequence identity at the amino acid level, preferably over 50 % sequence identity at the amino acid level more preferably over 65 % sequence identity at the amino acid level, even more preferably over 75 % sequence identity at the amino acid level and most preferably over 85 % sequence identity at the amino acid level.

The invention in particular concerns the use of a compound according to the invention for the manufacture of a medicament for prevention or treatment of a LXR receptor protein or LXR receptor protein homologue mediated disease or condition in a mammal wherein the prevention or treatment is directly or indirectly accomplished through the binding of a compound according to the invention to the LXR receptor protein or to the LXR receptor protein homologue.

The term mediated herein means that the physiological pathway in which the LXR receptor protein acts is either directly or indirectly involved in the disease or condition to be treated or prevented. In the case where it is indirectly involved it could be that, e.g. modulating the activity of LXR by a compound according to the invention influences a parameter which has a beneficial effect on a disease or a condition. One such example is that modulation of LXR activity leads to decreased levels of serum cholesterol or certain lipoproteins which in turn have a beneficial effect on the prevention and treatment of atherosclerosis. Herein a condition is a physiological or phenotypic state which is desirably altered. One such example would be obesity which is not necessarily medically harmful but nonetheless a non desirable phenotypic condition. In a preferred embodiment of the invention the method for prevention or treatment of a LXR receptor protein mediated disease or condition is applied to a human. This may be male or female.

Pharmaceutical compositions generally are administered in an amount effective for treatment or prophylaxis of a specific condition or conditions. Initial dosing in human is accompanied by clinical monitoring of symptoms, such symptoms for the selected condition. In general, the compositions are administered in an amount of active agent of at least about 100 µg/kg body weight. In most cases they will be administered in one or more doses in an amount not in excess of about 20 mg/kg body weight per day. Preferably, in most cases, doses is from about 100 µg/kg to about 5 mg/kg body weight, daily.

For administration particularly to mammals, and particularly humans, it is expected that the daily dosage level of active agent will be 0,1 mg/kg to 10 mg/kg and typically around 1 mg/kg.

By "therapeutically effective amount" is meant a symptom-alleviating or symptom-reducing amount, a cholesterol-reducing amount, a cholesterol absorption blocking amount, a protein and/or carbohydrate digestion-blocking amount and/or a de novo cholesterol biosynthesis-blocking amount of a compound according to the invention.

Likewise the invention concerns of the use of a compound according to the invention for the manufacture of a medicament for treating in mammal a disease which is correlated with abnormal cholesterol, triglyceride, or bile acid levels or deposits.

Accordingly, the compounds according to the invention may also be used for the manufacture of a medicament for prevention or treatment of mammalian atherosclerosis, gallstone disease, lipid disorders, Alzheimer's disease, skin disorders, inflammation, obesity or cardiovascular disorders such as coronary heart disease or stroke.

The invention further concerns the use of a compound according to the invention for the manufacture of a medicament for blocking in a mammal the cholesterol absorption in the intestine in need of such blocking, and also to treat obesity in humans.

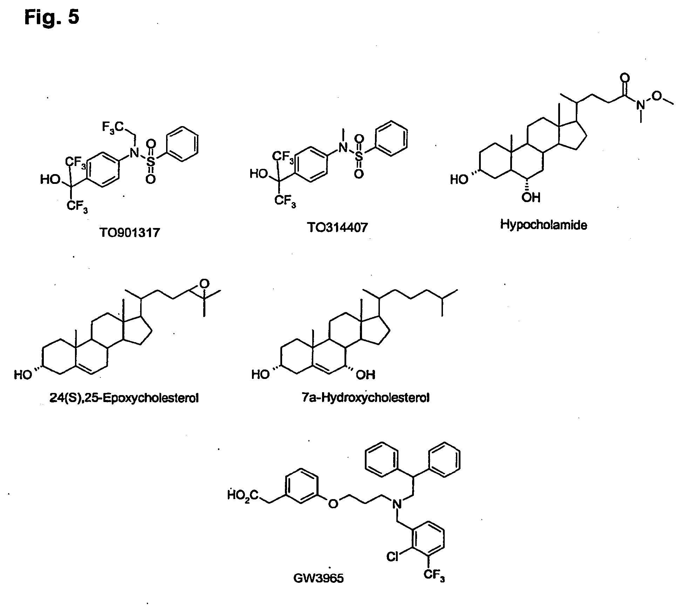

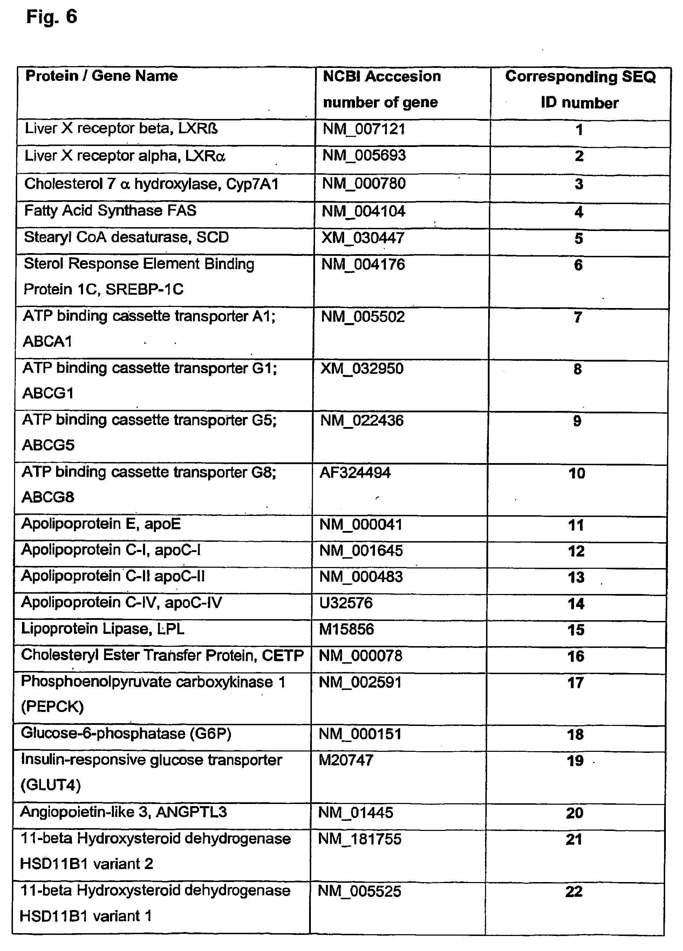

The Liver X Receptor alpha is a prototypical type 2 nuclear receptor meaning that it activates genes upon binding to the promoter region of target genes in a heterodimeric fashion with Retinoid X Receptor. The relevant physiological ligands of LXR are oxysterols. The compounds have been demonstrated to have a high binding efficacy (binding coefficients measured as EC50 in the range of 1-5 µM) as well as agonistic and/or partial agonistic properties. Consequently they may be applied to regulate genes that participate in bile acid, cholesterol and fatty acid homeostasis as well as other downstream regulated genes. Examples of such genes are but are not limited to lipid absorption, cholesterol biosynthesis, cholesterol transport or binding, bile acid transport or binding, proteolysis, amino acid metabolism, glucose biosynthesis, protein translation, electron transport, and hepatic fatty acid metabolism. LXR often functions in vivo as a heterodimer with the Retinoid X Receptor. Published non-steroidal LXR agonists such as the "Tularik" compound "TO901317" (see figure 5) are known to influence the regulation of various liver genes. Genes found to be regulated by TO901317 can be found in figure 6. Thus, the invention also concerns a method of modulating a gene whose expression is regulated by the LXR receptor in a mammal comprising administration of a therapeutically effective amount of a compound according to the invention to said mammal.

A number of direct and indirect LXR target genes have been described whose regulated expression contribute to cholesterol homeostasis and lipogenesis. In this respect the direct regulation of Cyp7A, which was shown to be a direct target gene of LXR at least in the rodent lineage is an important aspect of cholesterol removal by increased metabolism of bile acids (Lehmann et al., J Biol.Chem. 272 (6) 3137-3140; 1007). Gupta et al. (Biochem. Biophys Res.Com, 293; 338-343, 2002) showed that LXR α regulation of Cyp7A is dominant over FXR inhibitory effects on Cyp7A transcription.

A key transcription factor that was also shown to be a direct target gene for the LXR receptor is SREBP-1C (Repa et al., Genes and Development, 14:2819-2830; 2000: Yoshikawa et al.; Mol.Cell.Biol.21 (9) 2991-3000,2001). SREBP-1C itself activates transcription of genes involved in cholesterol and fatty acid synthesis in liver but also other mammalian tissues. Some of the SREBP1 c target genes involved in lipogenesis like FAS and SCD have shown to be additionally direct targets of the LXR receptors (Joseph et al.; J Biol Chem. 2002 Mar 29;277(13):11019-25; Liang et al., J Biol Chem. 2002 Mar 15;277(11):9520-8.).

A primary limitation for the applicability of LXR agonists as e.g. anti-atherosclerotic drugs comes from the observation that compounds with full agonistic activity, e.g. T0901317, not only elevate HDL cholesterol levels but do also increase plasma triglyceride levels in mice (Schultz et al., 2000 Genes Dev. 14:2831-8.). Concommitantly, not only genes that are involved in cholesterol efflux such as the cholesterol transporter ABCA1 (Venkateswaran et al., 2000 PNAS. 97:12097-102.), ABCG1 as well as the lipid binding protein Apoliprotein E (Laffite et al. 2001 PNAS 98:507-512) are induced by full LXR agonists, but also genes involved in lipogenesis, including the fatty acid synthase FAS (Joseph et al 2002 J Biol Chem. 277:11019-11025), and SREB P-1c (Yoshikawa et al., 2001 Mol Cell Biol. 21:2991-3000). Elevation of serum triglyceride levels is an indendent risk factor for atherosclerosis (for review see Miller (1999) Hosp Pract (Off Ed) 34: 67-73.). Thus, LXR activity needs to be selectively modulated for therapeutic benefit. In particular, compounds need to be found that stimulate reverse cholesterol transport, but do not significantly increase trigclyceride levels.

Another gene that has been shown to be directly regulated by LXRs is the LPL gene, that codes for a key enzyme that is responsible for the hydrolysis of triglycerides in circulating lipoprotein, releasing free fatty acids to peripheral tissues. (Zhang et al. J Biol Chem. 2001 Nov 16;276(46):43018-24.) This enzyme is believed to promote uptake of HDL cholesterol in liver, thereby promoting reverse cholesterol transport. A similar functional involvement in HDL clearance is described for the CETP gene product that facilitated the transfer of HDL cholesterol esters from plasma to the liver. LXR response elements were found in the CETP promoter and direct activation of this gene by LXR was demonstrated (Luo and Tall; J Clin Invest. 2000 Feb;105(4):513-20.).

The regulated transport of cholesterol through biological membranes is an important mechanism in order to maintain cholesterol homeostasis. A pivotal role in these processes in multiple tissues like e.g. macrophages and intestinal mucosa cells is maintained by the ATP-binding cassette transporter proteins (ABC). ABCA1 and ABCG1 were identified as direct LXR target genes (Costet et al.; J Biol Chem. 2000 Sep 8;275(36):28240-5) that mediate cholesterol efflux and prevent thereby e.g. generation of artherogenic plaques in macrophages (Singaraja et al. J Clin Invest. 2002 Jul;110(1):35-42). Other ABC transporters like ABCG5 and ABCG8 , primarily expressed in hepatocytes and enterocytes have also been reported to be directly responsive to LXR agonists (Repa et al., J Biol Chem. 2002 May 24;277(21):18793-800. Kennedy et al., J Biol Chem. 2001 Oct 19;276(42):39438-47) and mediate the secretion of sterols from the liver and efflux of dietary sterols from the gut.

Apolipoproteins E, C-I, C-II, and C-IV, that fulfill important roles in lipoprotein/lipid homeostasis have also been shown to be direct targets of the LXR receptor (Laffitte et al., Proc Natl Acad Sci U S A. 2001 Jan 16;98(2):507-12; Mak et al.; J Biol Chem. 2002 May 24 [epub ahead of print]). These proteins have been found to be crucial components of chylomicrons, VLDL, IDL, and HDL and are among other things associated with hypertriglyceridemia and arteriosclerosis.

Recently the LXRα itself was shown to be regulated by both LXR receptors in human cell types including macrophages suggesting an autoregulatory amplification event in the response to LXR ligands which could e.g. lead to an enhanced stimulation of LXR target genes like e.g. ABCA1 (Bolten et al.; Mol Endocrinol. 2002 Mar;16(3):506-14.; Laffitte et al., Mol Cell Biol. 2001 Nov;21(22):7558-68; Whitney et al.; J Biol Chem. 2001 Nov 23;276(47):43509-15).

Besides the important function of LXR receptors in tissues like liver and macrophages it has recently been reported that that stimulation of epidermal differentiation is mediated by Liver X receptors in murine epidermis. Differentiation maker genes like involucrin, loricin and profilaggrin have been shown to be upregulated upon LXR ligand treatment (Kömüves et al.; J Invest Dermatol. 2002 Jan;118(1):25-34.).

Another recent report describes the regulation of cholesterol homeostasis (primarily the regulation of ABCA1, ABCG1 and SREBP-1 C) by the LXR receptors in the central nervous system suggesting that LXRs may prove benefical in the treatment of CNS diseases such as Alzheimer's and Niemann-Pick disease that are known to be accompanied by dysregulation of cholesterol balance (Whitney et al.; Mol Endocrinol. 2002 Jun;16(6):1378-85).

Activation of LXR by an agonist improves glucose tolerance in a murine model of diet-induced obesity and insulin resistance. Gene expression analysis in LXR agonist-treated mice reveals coordinate regulation of genes involved in glucose metabolism in liver and adipose tissue, e.g. the down-regulation of peroxisome proliferator-activated receptor gamma coactivator-1 alpha (PGC-1), phosphoenolpyruvate carboxykinase (PEPCK), and glucose-6-phosphatase expression and induction of glucokinase in liver. In adipose tissue, activation of LXR led to the transcriptional induction of the insulin-sensitive glucose transporter, GLUT4. LXR agonist may limit hepatic glucose output and improve peripheral glucose uptake (Laffitte et al. (2003) PNAS 100:5419-24).

Therefore one other important embodiment of the invention concerns the use of compounds according to the invention for the manufacture of a medicament that enhances or suppresses amongst other today yet unknown LXR target genes the above mentioned genes and the associated biological processes and pathways.

The compounds according to the invention may be used in particular for the manufacture of a medicament for the prevention or treatment of a LXR receptor protein or LXR receptor protein homologue mediated disease or condition in a mammal wherein the prevention or treatment is directly or indirectly accomplished through the binding of the compound according to the invention to the LXR receptor protein or LXR receptor protein homologue. These pharmaceutical compositions contain 0,1 % to 99,5 % of the compound according to the invention, more particularly 0,5 % to 90 % of the compound according to the invention in combination with a pharmaceutically acceptable carrier.

The invention concerns also the use of a compound according to the invention for the manufacture of a medicament for the prevention or treatment of a LXR receptor protein mediated disease or condition wherein the mammal described above is a human. The medicament may be used for regulating the cholesterol transport system, for regulating levels of cholesterol, triglyceride, and/or bile acid in a mammal preferentially a human by activating the LXR receptor. The medicament may be used for the treatment of atherosclerosis, gallstone disease, lipid disorders, Alzherimer's disease, skin disorders, obesity or a cardiovascular disorder.

The invention further concerns the use of a compound according to the invention for the manufacture of a medicament capable for blocking in a mammal, preferentially in a human the cholesterol absorption in the intestine. Further the claimed compound may be used for the manufacture of a medicament for treating obesity in humans and for modulating a gene whose expression is regulated by the LXR receptor (see details above and figures).

### EXAMPLES

### EXAMPLE 1

In vitro screening for compounds which influence LXR binding to coactivators.

For screening purposes a GST and 6 x His fusion of the LBD (from amino acids 155 of hLXRalpha to 447) of human LXRalpha is constructed by first cloning a Gateway cassette (Invitrogen) in frame into the Sma I site of the pAGGHLT Polylinker (Pharmingen). Then a PCR fragment specifically amplified from human liver cDNA is cloned into the resulting pACGHLT-GW following the manufacturers instructions for Gateway cloning (Invitrogen) to yield pACGHLT-GW-hLXRalphaLBD. Primers used for Amplification are: 100 % sequence integrity of all recombinant products is verified by sequencing. Recombinant Baculovirus is constructed from pACGHLT-GW-hLXRalphaLBD using the Pharmingen Baculovirus Expression vector system according to instructions of the manufacturer. Monolayer cultures of SF9 cells are infected by the virus as recommended by Pharmingen or 200ml cultures of 1 x10⁶ cells/ml grown in 2 liter Erlenmeyer flasks on an orbital shaker at 30 rpm are infected by 10ml of same virus stock. In both cases cells are harvested 3 days after infection. All cell growth is performed in Gibco SF900 II with Glutamine (Invitrogen) medium without serum supplementation at 28°C. Since SF9 cells contain significant amounts of endogenous GST, purification is performed via His and not via GST affinity chromatography. To this end instructions of Pharmingen for purification of recombinant His tagged proteins from SF9 cells are followed with the following modifications: All detergents are omitted from the buffers and cells were lysed on ice by 5 subsequent sonication pulses using a sonicator needle at maximum power. All eluates are dialyzed against 20 mM Tris/HCl pH 6,8, 300 mM KCl; 5 mM MgCl₂; 1 mM DTT; 0,2 mM PMSF; 10% Glycerol. A typical dialyzed eluate fraction contains the fusion protein at a purity of more than 80%. Total protein concentration is 0,1-0,3 mg/ml.

For E. coli expression of a NR coactivator, pDest17-hTif2BD expressing a NR interaction domain from amino acids 548-878 of human Tif2 (Acc. No: XM_011633 RefSeq) tagged by 6 N-terminal His residues is constructed. Therefore, a PCR fragment specifically amplified from human liver cDNA is subcloned into pDest 17 (Invitrogen) following the manufacturers instructions for Gateway cloning (Invitrogen).Primers used for Amplification are: and For E. coli expression plasmid DNA is transformed into chemically competent E. coli BL21 (Invitrogen, USA) and cells are grown to an OD600 of 0.4-0.7 before expression was induced by addition of 0,5 mM IPTG according instructions of the manufacturer (Invitrogen). After induction for 8 hours at 30°C cells are harvested by centrifugation for 10 minutes at 5000 x g. Fusion proteins are affinity purified using Ni-NTA Agarose (QIAGEN) according to the instructions of the manufacturer. Recombinant Tif2 construct is dialyzed against 20 mM Tris/HCL pH 7.9; 60 mM KCl; 5 mM MgCl₂; 1 mM DTT, 0,2 mM PMSF; 10% glycerol. A typical dialyzed eluate fraction contains the fusion protein at a purity of more than 80%. Total protein concentration is 0,1-0,3 mg/ml.
The TIF2 fragment is subsequently biotinylated by addition of 5-40µl/ml Tif2 fraction of a Biotinamidocaproate N-Hydroxysuccinimide-ester (Sigma) solution (20 mg/ml in DMSO). Overhead rotating samples are incubated for 2 hours at room temperature. Unincorporated label is then separated using G25 Gel filtration chromatography (Pharmacia Biotech, Sweden). Protein containing fractions from the column are pooled and tested for activity in the assay as described below.

For screening of compound libraries as provided for by the methods shown below in the examples for substances which influence the LXR/Tif 2 interaction, the Perkin Elmer LANCE technology is applied. This method relies on the binding dependent energy transfer from a donor to an acceptor fluorophore attached to the binding partners of interest. For ease of handling and reduction of background from compound fluorescence LANCE technology makes use of generic fluorophore labels and time resoved detection (for detailed description see Hemmilä I, Blomberg K and Hurskainen P, Time-resolved resonance energy transfer (TR-FRET) principle in LANCE, Abstract of Papers Presented at the 3 rd Annual Conference of the Society for Biomolecular Screening, Sep., California (1997))
For screening, 20-200 ng of biotinylated Tif 2 fragment and 10-200 ng of GST-LXR fragment are combined with 0.5-2 nM LANCE Eu-(W1024) labelled anti-GST antibody (Perkin Elmer) and 0,1-0,5µg of highly fluorescent APC-labelled streptavidin (Perkin Elmer, AD0059) in the presence of 50µM of individual compounds to be screened in a total volume of 25 µl of 20 mM Tris /HCl pH 6,8; 300 mM KCl; 5 mM MgCl2; 100-1000 ng/µl/ BSA DMSO content of the samples is kept below 4%. Samples are incubated for a minimum of 60 minutes in the dark at room temperature in FIA-Plates black 384well med. binding (Greiner).

The LANCE signal is detected by a Perkin Elmer VICTOR2VT™ Multilabel Counter applying the detection parameters listed in Fig. 2. The results are visualized by plotting the ratio between the emitted light at 665 nm and at 615 nm. For every batch of recombinant proteins amount of proteins, including BSA and labeling reagents giving the most sensitive detection of hits is determined individually by analysis of dose response curves for 22R Hydroxycholesterol and TO 901317

### EXAMPLE 2

Experimental procedure for the preparation of the compounds according to the invention.

### o-AZIDOBENZOIC ACID SYNTHESIS (2)

The anthranilic acid (1, 1 eq., 0.5-1 M) is suspended in 6 M HCl, containing enough AcOH (0-20% dependent upon the anthranilic acid) to facilitate dissolution of the anthranilic acid and/or the intermediate diazonium salt, and cooled to 0 °C. NaNO₂ (1.1 eq., 1.3-2.5 M) dissolved in H₂O is added to the anthranilic acid solution at a rate such that the temperature of the reaction solution remains below 5 °C. The resulting homogeneous solution of the diazonium salt is slowly filtered through a sintered glass funnel into a solution of NaN₃ (1.1 eq., 0.7-1.1 M) and NaOAc (12 eq.) in H₂O. The reaction mixture is stirred/shaken for 30-60 min following cessation of vigorous N₂ evolution. Following acidification of the reaction mixture to pH 1 with concentrated HCl, the mixture was cooled to 0 °C to encourage complete precipitation of the *o-*azidobenzoic acid. The precipitate is collected by filtration and washed with 6 M HCl (2x) and H₂O (2x). The *o*-azidobenzoic acid product (2) is dried *in vacuo* (500 mtorr, 30 °C).

### ACYLATION OF HYDROXYMETHYL RESIN (4)

To hydroxymethyl resin (1.0 eq., 1.3 mmol/g) and the *o*-azidobenzoic acid (1, 2.5 eq.) is added DMF (to give 400 mM *o*-azidobenzoic acid ,1), CsCO₃ (2.0 eq.) and KI (2.0 eq.). Following agitation of the reaction mixture for 36-48 h, the resin-bound *o-*azidobenzoic acid (4) is washed with MeOH (2 cycles), CH₂Cl₂ (3 cycles), MeOH (3 cycles), DMF (3 cycles), MeOH (3 cycles) and CH₂Cl₂ (3 cycles), and dried *in vacuo*.

### AZA-WITTIG FORMATION (5)

To the resin-bound *o*-azidobenzoic acid (4,1.0 eq.) is added a solution of PPh₃ (THF, 500 mM, 5.0 eq.). After 6 h, the resin is washed with 3 cycles of the following: THF (3 cycles), toluene (3 cycles), CH₂Cl₂ (3 cycles) and hexanes (3 cycles). Followed by drying *in vacuo* to afford resin bound iminophosphorane (5)

### CARBODIIMIDE FORMATION (6)

To the resin-bound iminophosphorane (5, 1 eq.) is added isocyanate (9 , 5 eq., 450 mM) dissolved in ClCH₂CH₂Cl. The compounds are shaken at ambient temperature for 16 h, washed with 3 cycles of the following: THF (3 cycles), toluene (3 cycles), CH₂Cl₂ (3 cycles) and hexanes (3 cycles), and dried *in vacuo* to afford carbodiimide (6).

### GUANIDINE FORMATION / CYCLIZATION

To the carbodiimide functionalized resin (6) is added secondary amine (10, 0.6 eq., 500 mM) dissolved in ClCH₂CH₂Cl. The reaction mixture is heated to 50 °C in an incubator for 12-72 h to afford 2-aminoquinazoline (8).

All of the final products are analyzed using an Evaporative Light Scattering Detector (ELSD) detection to determine purity.

### EXAMPLE 3

This example illustrates that compounds according to the invention (experiments shown were done with MOLSTRUCTURE TR1040001892, TR1040011382 , TR1040002211 and TR1040002212 (see formulas (II) to (V) for structural formulas)) activate luciferase reporter gene expression in a dose dependent manner mediated through GAL4-LXRa-LBD or GAL4-LXRb-LBD constructs in HEK293 cells. TR1040001892 and TR1040011382 do activate LXR beta LBD truct mediated luciferase activity much stronger than with LXR alpha construct which is in contrast to the similar activation of both LXR alpha and LXR beta LBD containing constructs by TR1040002211 and TO901317.

HEK293 cells are grown in 96 well plates and co-transfected with pFR-luc (Stratagene) and pCMV-BD-LXRa-LBD or pCMV-BD-LXRb-LBD (each 100 ng of plasmid DNA per well). Transfection is carried using Lipfectamine 2000 (Gibco-BRL) according to the manufacturers protocol. The ligand binding domains (LBD) of LXRa and LXRb are cloned into the pCMV-BD-GW (the Gateway Reading Frame Cassette B is cloned as an EcoRV fragment into Smal site of pCMV-BD) applying the manufacturer protocols for the Gateway™ system (Invitrogen).
Luciferase reporter activity is measured in triplicates from extracts of cells after incubating cells in culture medium (DMEM [Gibco-BRL] + 10% FCS [PAA laboratories]) for 16 hours (5% CO₂, 37°C) containing 0,5% DMSO (control) or 0,5% DMSO with increasing concentrations of TR1040001892, TR1040002211 or T0901317 (Sigma T 2320, see figure 5 for structural formula). The type of assay used here is a mammalian one hybrid (M 1 H) assay that is known to those skilled in the art.

Dose-dependent luciferase activities originating from pFR-luc demonstrate the relative activities of the compounds with the LXRa or LXRb LBDs in this mammalian one hybrid type approach.

### EXAMPLE 4

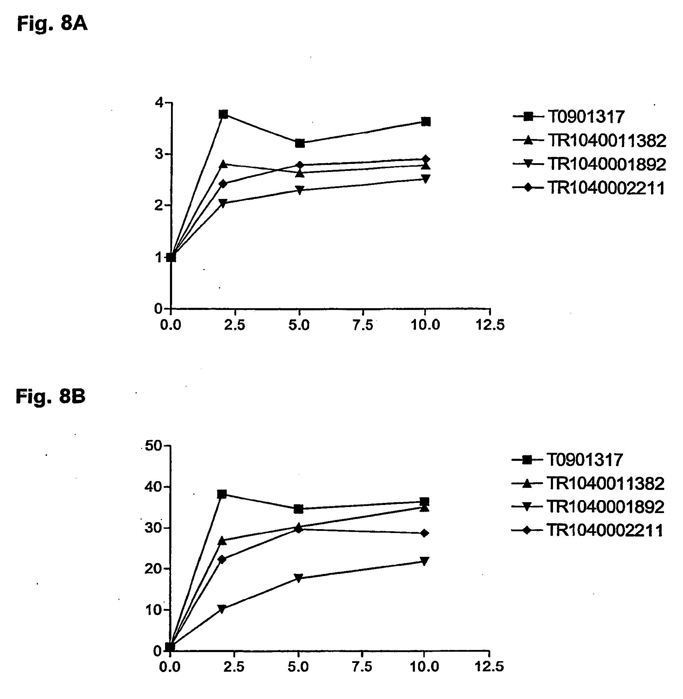

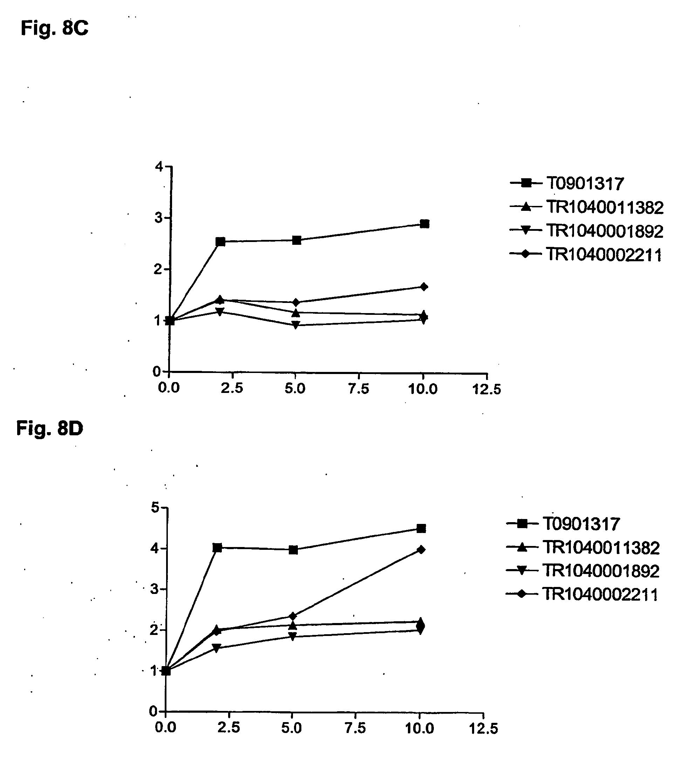

This example shows that described compounds can increase the abundance of mRNA of LXR target genes like ABCA1 and ABCG1 in THP-1 cells which are treated with TPA or FAS and SREBP-1c in HepG2 cells as shown in Fig 8A-D.

THP-1 cells are seeded in 24 well plates at 3 x10⁵ cells per well in RPMI 1640 medium containing 10 % FCS and 100 nM TPA for 24 h. HepG2 cells are seeded in poly-L-Lysine coated 24well plates at 1x10⁶ cells per well in EMEM medium containing 10 % FCS until they are appr. 60% confluent.
Before treatment with LXR compounds, the growth medium is changed to medium containing 10% charcoal/dextran-stripped FCS for 12 h. Treatment is done for 12h (THP-1 cells) and 24h (HepG2 cells), respectively, in medium containing 10% charcoal/dextran-stripped FCS (and 100 nM PMA in the case of THP-1 cells). LXR compounds are dissolved in DMSO, with the final solvent concentration never exceeding 0.125% . All treatments are done in triplicates and experiments repeated twice. Total RNA is extracted using the Qiagen Rneasy Mini Kit and treated with DNase (DNAfree kit, Ambion). RNA is reverse transcribed with Oligo(dT) primer and real-time reverse transcription PCR (TaqMan) is performed using the ABI Prism 7900HT Sequence Detection System and reagents supplied by Applied Biosystems. mRNA steady state levels are normalised to H3 histone (H3F3A) expression levels. The sequences of forward primers, reverse primers and TaqMan probes are as follows :

### EXAMPLE 5

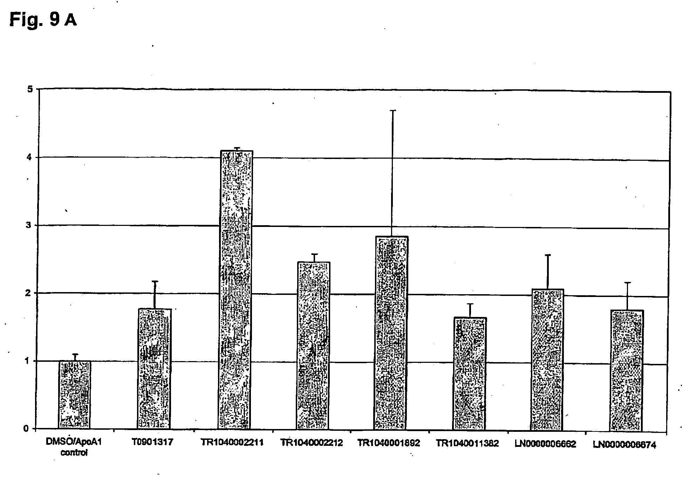

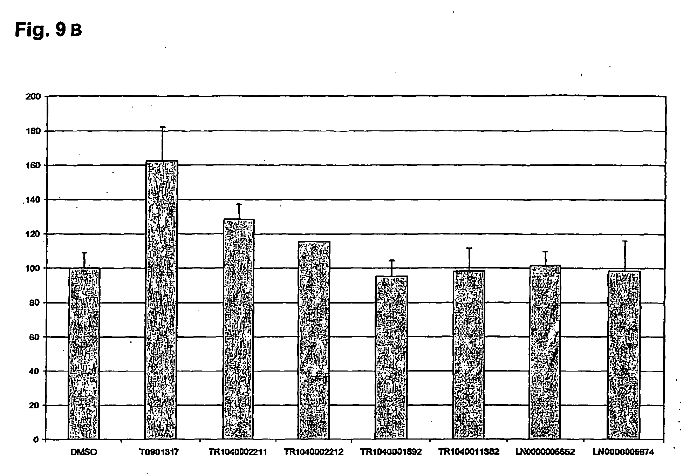

All compounds T0901317, TR1040002211, TR1040002212. TR1040001892, TR1040011382, LN0000006662 and LN0000006674 cause a marked increase in cholesterol export in differentated THP-1 macrophages (see Figure 9 A). Strikingly, the compound T0901317 causes a marked increase in triyglyceride mass in HepG2 liver cells, while compounds like TR1040001892 and TR1040011382 do not cause a significant increase in triglyceride mass. Compounds TR1040002211 and TR1040002212 cause a slight increase in triglyceride mass.
This behavior is similar to the selective transcriptional effect of compounds like TR1040001892 and TR1040011382 on the LXR target genes in HepG2 versus THP-1 cells.

Methods: Cultures of the monocyte-macrophage cell line and the hepatocytes HepG2 are obtained from the American Type tissue Culture Collection, Rockville, MD and were grown in RPMI 1640 medium supplemented with 10% fetal bovine serum, 10 mM HEPES, 2 mM Pyruvat, 50 µM 13-Mercaptoethanol (THP-1) and Minimum essential medium (Eagle) with 2 mM L-glutamine and Earle's BSS supplemented with 10% fetal bovine serum, 2 mM glutamine, 0.1 mM non-essential amino acids, 1 mM sodium pyruvate (HepG2), respectively, at 37°C in 5% CO2.
THP-1 cells are differentiated into macrophages by addition of 100 nM Phorbol 12-Myristate 13-Acetate (PMA; Sigma P8139) and PMA included in the medium of all subsequent experiments to maintain differentiation.
For cholesterol efflux measurements and triglyceride analysis cells are seeded in 6well plates at 1.8x106 cells (THP-1) and 1x106 cells (HepG2) per well, respectively.

### CHOLESTEROL EFFLUX

THP-1 cells are seeded in 6well plates at 1X10⁶ cells per well in RPMI 1640 medium containing 10 % FCS and 100 nM TPA for 72 h. After washing with PBS cells are incubated 24 h with fresh in RPMI 1640 medium containing 10 % FCS and 100 nM TPA. Cells are washed twice with PBS and RPMI 1640 medium containing 0.15% BSA and 100 nM TPA is added for further 24 h. Treatment with LXR compounds is done for 24 h in RPMI 1640 medium containing 0.15% BSA, 100 nM TPA and 40 µg/ml ApoA1 (Calbiochem # 178452). Medium is collected, centrifuged to remove cell debris and assayed for cholesterol using a commercial fluorometric kit (Molecular Probes A-12216). The remaining cellular proteins are lysed with 0.3 N NaOH and protein content measured with the Biorad Bradford reagent.

### TRIGLYCERIDE ASSAY

HepG2 cells are seeded in poly-L-Lysine coated 6well plates at 1x10⁶ cells per well in EMEM medium containing 10 % FCS until they were appr. 60% confluent. Before treatment with LXR compounds growth medium is changed to medium containing 10% charcoal/dextran-stripped FCS for 12 h. Treatment is done for 24h in medium containing 10% charcoal/dextran-stripped FCS. Cells are washed twice with ice-cold PBS/0.2% BSA arid twice with cold PBS and all liquid carefully removed. Triglyceride are extracted with with 1.5 ml hexane/ isopropanol = 3:2 per well with gentle shaking for 2-3 h at RT according to Pan et al. (2002) JBC 277, 4413-4421 and Goti et al. (1998) Biochem J, 332, 57-65 . The extraction solution is collected, dried under vacuum and redissolved in isopropanol/ 1.5% triton. The remaining cellular proteins are lysed with 0.3 N NaOH and protein content measured with the Biorad Bradford reagent.
Triglyceride levels are measured as esterified glycerol using a commercial enzymatic colorimetric kit (Sigma 343-25P). In a preliminary assay it is checked by omitting the lipase enzyme that contribution of free glycerol is negligible.

### FIGURE CAPTIONS

**FIG. 1**

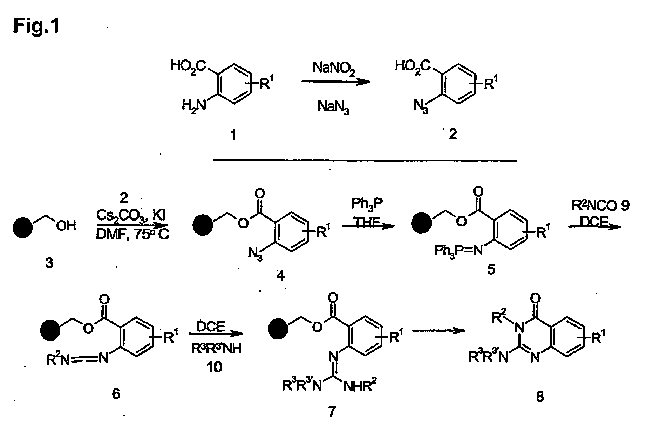

   Fig. 1 shows the synthesis of the compounds according to the invention as also described in EXAMPLE 2.
**FIG. 2**
   Fig. 2 shows the measurement parameters employed by the Wallace VICTOR2V™ Multilabel Counter which was used for measuring the EC₅₀ values (see also EXAMPLE 1)
**FIG. 3**
   Shows a table with the accession numbers for the key genes
**FIG. 4**

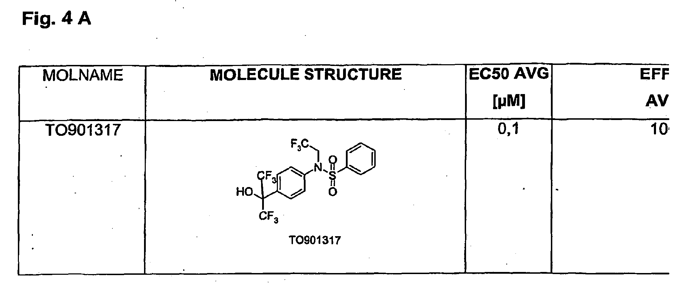

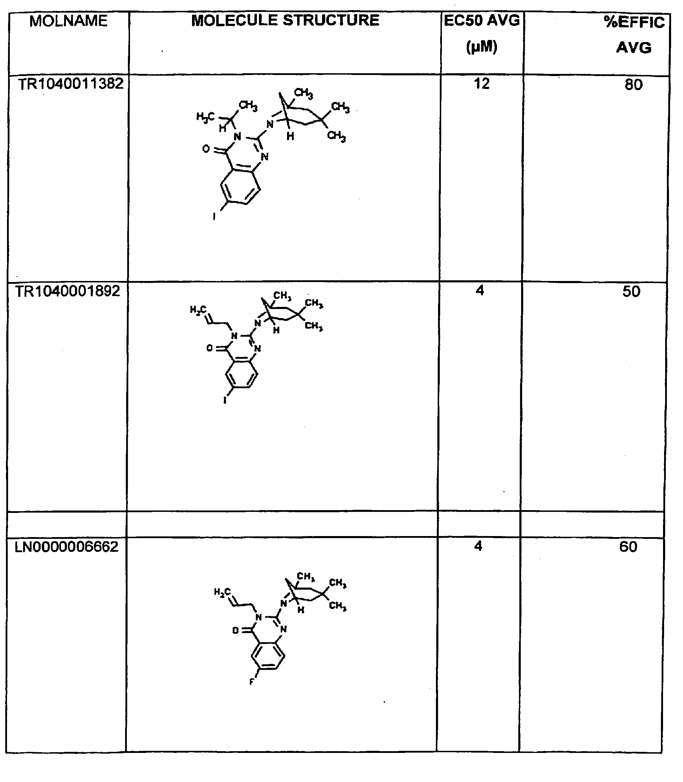

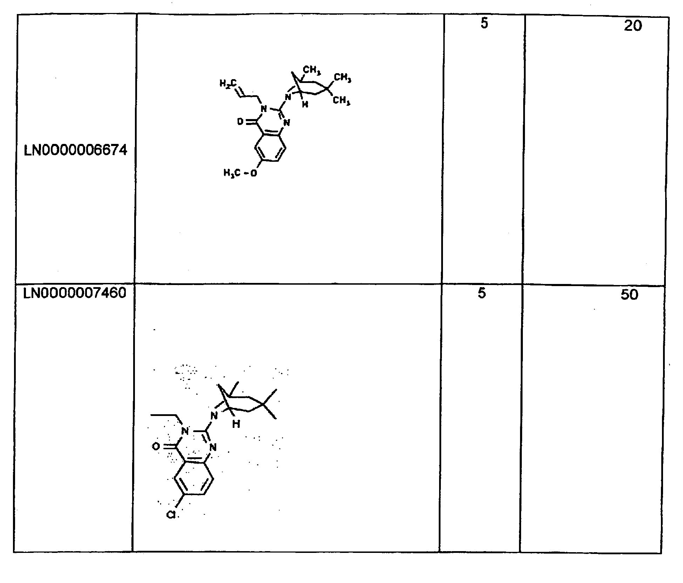

   Fig. 4 shows the intemal molecular name used by the applicant (MOLNAME) as well as the corresponding structures of preferred compounds according to the invention. The figure further shows their respective EC₅₀ values (EC50 AVG) as established according to the Example 1 in multiple experiments (see above), as well as their respective average efficacy (% activity relative to TO901317 control agonist).
**FIG. 5**
   Figure 5 shows various known LXR ligands. The compound TO901317 is used as a reference compound here. It is apparent from their structures that the inventors have identified novel compounds which are structurally not related to these known ligands.
**FIG. 6**
   Figure 6 shows various genes that have been found to be regulated through binding of an LXR agonist to the LXR protein.
**FIG. 7**

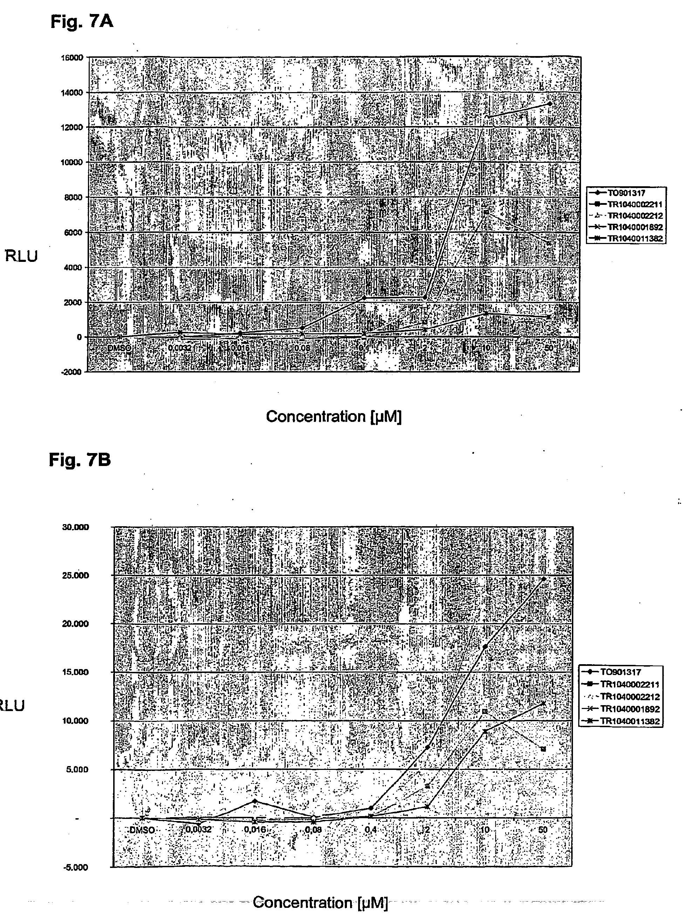

   Fig 7A and Fig 7B show dose dependence of inidcated compounds with LXR alpha LBD (7A) or LXR beta (7B) LBD containing constructs in mammalian one hybrid (M1H) type assays. The respective µM concentrations of the compounds T0901317, TR1040002211, TR1040002212. TR1040001892 and TR1040011382 are given on the x-axis and the relative light units (RLU) are depicted on the y-axis.
**Fig. 8**
   Analysis of mRNA content of the indicated genes (ABCA1, ABCG1, FAS and SREBP-1c) in total RNA isolated from THP-1 cells (8A and 8B) or HepG2 cells (8C and 8D) treated for 12 or 24 hours with indicated concentrations (µM on x-axis) of T0901317, TR1040011382, TR1040001892 and TR1040002211. The relative fold inuction is depicted on the y-axis.
**Fig. 9**
   Analysis of relative fold increase in total cholesterol from supematants of cultivated THP-1 cells (indicated on the y-axis) incubated with ApoA1 and with or without 10µM of the compounds T0901317, TR1040002211, TR1040002212. TR1040001892, TR1040011382, LN0000006662 and LN0000006674 as indicated on the X-axis of Fig 9A.
   Analysis of relative levels of total triglyceride (TG) content in HepG2 cells (indicated on the y-axis) treated with 25µM of the indicated compounds T0901317, TR1040002211, TR1040002212. TR1040001892, TR1040011382, LN0000006662 and LN0000006674 (indicated on the x-axis).

## Claims

1. A compound of the formula (I), or pharmaceutically acceptable salts or solvates thereof, 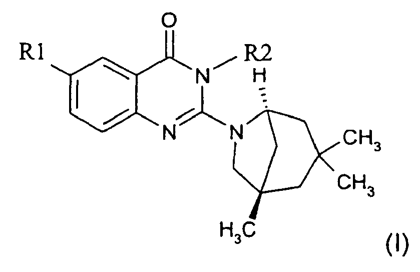 wherein substituents and indices have the following meanings:
R₁ is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl,
R₂ is H, C₁ to C₈ alkyl, C₂ to C₆ alkenyl, C₁ to C₈ substituted alkyl, C₇ to C₁₂ phenylalkyl or C₇ to C₁₂ substituted phenylalkyl,
with the proviso that
6-iodo-3-propyl-2-(1,3,3-trimethyl-6-azabicyclo[3.2.1]oct-6-yl)-4(3H)-quinazolinone,
6-bromo-4-oxo-2-(1,3,3-trimethyl-6-azabicyclo[3.2.1]oct-6-yl)-3(4H)-quinazolinepropanoic acid ethyl ester,
6-bromo-3-(2-propenyl)-2-(1,3,3-trimethyl-6-azabicyclo[3.2.1]oct-6-yl)-4(3H)-quinazolinone,
6-bromo-3-propyl-2-(1,3,3-trimethyl-6-azabicyclo[3.2.1]oct-6-yl)-4(3H)-quinazolinone,
8-methyl-4-oxo-2-(1,3,3-trimethyl-6-azabicyclo[3.2.1]oct-6-yl)-3(4H)-quinazolinepropanoic acid ethyl ester,
8-methyl-3-(2-propenyl)-2-(1,3,3-trimethyl-6-azabicyclo[3.2.1]oct-6-yl)-4(3H)-quinazolinone,
2-[ethyl(4-pyridinylmethyl)amino]-8-methoxy-3-(2-phenylethyl)-4(3H)-, quinazolinone
are excluded.

2. A compound according to claim 1, or pharmaceutically acceptable salts or solvates thereof, wherein the substituents and indices have the following meanings:
R₁ is halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, and R₂ is H, C₁ to C₈ alkyl,
C₂ to C₆ alkenyl, C₁ to C₈ substituted alkyl.

3. A compound according to claim 1 or 2 with: R₁ being halogen and preferably iodine over bromine and chlorine and R₂ being H, C₁ to C₆ alkyl, C₃ to C₅ alkenyl, C₁ to C₆ substituted alkyl.

4. A compound according to any of claims 1 to 3 being selected from 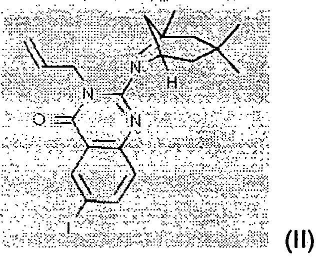 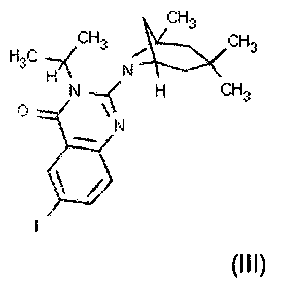 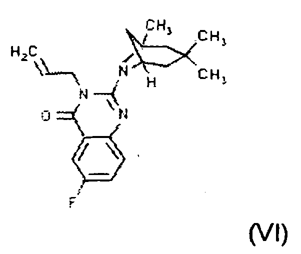 and

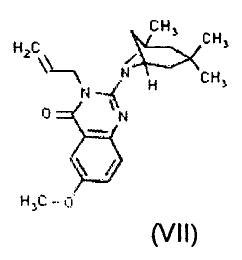

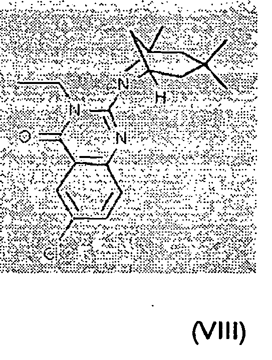

5. A compound according to formula (I), or pharmaceutically acceptable salts or solvates thereof, 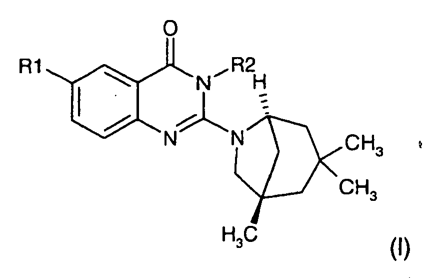 wherein substituents and indices have the following meanings:
R₁ is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy,
protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl,
R₂ is H, C₁ to C₈ alkyl, C₂ to C₆ alkenyl, C₁ to C₈ substituted alkyl, C₇ to C₁₂ phenylalkyl or C₇ to C₁₂ substituted phenylalkyl, for use as a medicament.

6. Use of a compound according to formula (I), or pharmaceutically acceptable salts or solvates thereof, 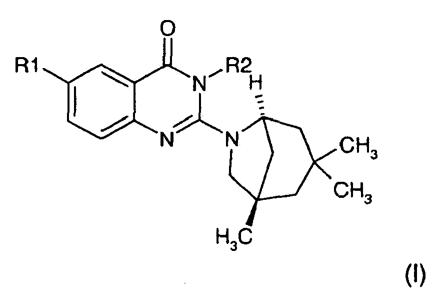 wherein substituents and indices have the following meanings:
R₁ is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl,
R₂ is H, C₁ to C₈ alkyl, C₂ to C₆ alkenyl, C₁ to C₈ substituted alkyl, C₇ to C₁₂ phenylalkyl C₇ to C₁₂ substituted phenylalkyl,
for the manufacture of a medicament for the prevention or treatment of a disease or condition mediated by a NR1H3 receptor protein encoded by a nucleic acid comprising SEQ ID No. 2, a portion thereof or a mammalian homologue thereof, and/or a disease or condition mediated by a NR1H2 receptor protein encoded by a nucleic acid comprising SEQ ID No: 2, a portion thereof or a mammalian homologue thereof in a mammal, wherein the prevention or treatment is directly or indirectly accomplished through the binding of the compound to the NR1H3 and/or NR1H2 receptor protein or NR1H3 and/or NR1H2 receptor protein homologue.

7. Use according to claim 6 for the manufacture of a medicament for prevention or treatment of a NR1H3 and/or NR1H2 receptor protein mediated disease or condition wherein the mammal is a human.

8. Use according to claim 6 or 7 for the manufacture of a medicament for regulating the cholesterol synthesis or transport in a mammal by activating the NR1H3 and/or NR1H2 receptor.

9. Use of a compound as specified in claim 6 for the manufacture of a medicament for regulating levels of cholesterol, triglyceride, bile acid, glucose and/or glucocorticoid.

10. Use of a compound as specified in claim 6 for the manufacture of a medicament for treating in a mammal atherosclerosis, alzheimer disease, gallstone disease, lipid disorders, inflammatory disorder, type II diabetis, obesity or a cardiovascular disorder.

11. Use of a compound as specified in claim 6 for the manufacture of a medicament capable for blocking in a mammal the cholesterol and/or fatty acid absorption in the intestine.

12. Use of a compound as specified in claim 6 for the manufacture of a medicament for modulating a gene whose expression is regulated by the NR1H3 and/or NR1H2 receptor.

13. Use of a compound as specified in claim 6 in a mammal for the manufacture of a medicament for selective up-regulation of one or more genes selected from the group comprising ABCA1, ABCG1, ABCG5, ABCG8, apolipoprotein-Cl, -CII, -CIV, -E, LPL (lipoprotein lipase), CETP (cholesteryl ester transfer protein) or other genes that positively regulate cholesterol homeostasis.

14. Use of a compound as specified in claim 6 for the manufacture of a medicament which reduces the expression of one or more of the genes out of the group comprising 11-β-HSD (11-β hydroxysteroid dehydrogenase), PEPCK (phosphoenolpyruvat carboxykinase), G-6-P (glucose-6-phosphatase).

15. Use of a compound as specified in claim 6 for the manufacture of a medicament which does not increase or weakly increases the expression of one or more of the genes out of the group comprising FAS (Fatty Acid Synthase), SREBP-1c (Sterol-response element binding protein), SCD-1 (Stearoyl-CoA Desaturase), Angiopoietin like protein 3 (Angptl3) or other genes which are relevant for controlling serum triglyceride or glucose levels in liver and or other organs compared to administration of a full agonist like T0901317.

16. A medicament comprising a compound according to formula (I), or pharmaceutically acceptable salts or solvates thereof, 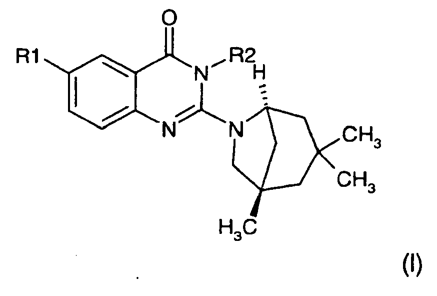 wherein substituents and indices have the following meanings:
R₁ is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl,
R₂ is H, C₁ to C₈ alkyl, C₂ to C₆ alkenyl, C₁ to C₈ substituted alkyl, C₇ to C₁₂ phenylalkyl or C₇ to C₁₂ substituted phenylalkyl, and a pharmaceutically acceptable carrier.

## Patentansprüche

1. Eine Verbindung der Formel (I), oder pharmazeutisch annehmbare Salze oder Solvate davon, 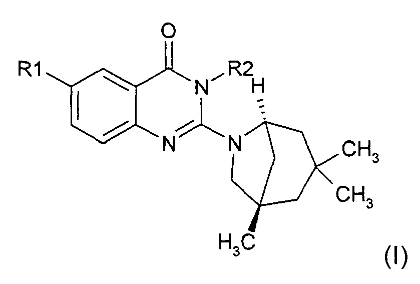 wobei die Substituenten und Indizes die folgende Bedeutung besitzen:
R₁ ist H, Halogen, Hydroxy, geschütztes Hydroxy, Cyano, Nitro, C₁ bis C₆ Alkyl, C₁ bis C₆ substituiertes Alkyl, C₁ bis C₇ Alkoxy, C₁ bis C₇ substituiertes Alkoxy, C₁ bis C₇ Acyl, C₁ bis C₇ substituiertes Acyl, C₁ bis C₇ Acyloxy, Carboxy, geschütztes Carboxy, Carboxymethyl, geschütztes Carboxymethyl, Hydroxymethyl, geschütztes Hydroxymethyl, Amino, geschütztes Amino, (monosubstituiertes) Amino, geschütztes (monosubstituiertes) Amino, (disubstituiertes) Amino, Carboxamid, geschütztes Carboxamid, N-(C₁ bis C₆ Alkyl)carboxamid, geschütztes N-(C₁ bis C₆ Alkyl)carboxamid, N, N-di(C₁ bis C₆ Alkyl)carboxamid, Trifluoromethyl, N-((C₁ bis C₆ Alkyl)sulfonyl)amino, N-(Phenylsulfonyl)amino oder Phenyl,
R₂ ist H, C₁ bis C₈ Alkyl, C₂ bis C₆ Alkenyl, C₁ bis C₈ substituiertes Alkyl, C₇ bis C₁₂ Phenylalkyl oder C₇ bis C₁₂ substituiertes Phenylalkyl,
mit der Maßgabe, dass
6-Iod-3-propyl-2-(1,3,3-trimethyl-6-azabicyclo[3.2.1]oct-6-yl)-4(3H)-chinazolinon,
6-Brom-4-oxo-2-(1,3,3-trimethyl-6-azabicyclo[3.2.1]oct-6-yl)-3(4H)-chinazolinpropansäureethylester,
6-Brom-3-(2-propenyl)-2-(1,3,3-trimethyl-6-azabicyclo[3.2.1]oct-6-yl)-4(3H)-chinazolinon,
6-Brom-3-propyl-2-(1,3,3-trimethyl-6-azabicyclo[3.2.1]oct-6-yl)-4(3H)-chinazolinon,
8-Methyl-4-oxo-2-(1,3,3-trimethyl-6-azabicyclo[3.2.1]oct-6-yl)-3(4H)-chinazolinpropansäureethylester,
8-Methyl-3-(2-propenyl)-2-(1,3,3-trimethyl-6-azabicyclo[3.2.1]oct-6-yl)-4(3H)-chinazolinon,
2-[Ethyl(4-pyridinylmethyl)amino]-8-methoxy-3-(2-phenylethyl)-4(3H)-chinazolinon
ausgeschlossen sind.

2. Verbindung gemäß Anspruch 1, oder pharmazeutisch annehmbare Salze oder Solvate davon, wobei die Substituenten und Indizes die folgende Bedeutung besitzen:
R₁ ist Halogen, Hydroxy, geschütztes Hydroxy, Cyano, Nitro, C₁ bis C₆ Alkyl, C₁ bis C₆ substituiertes Alkyl, C₁ bis C₇ Alkoxy, C₁ bis C₇ substituiertes Alkoxy, C₁ bis C₇ Acyl, C₁ bis C₇ substituiertes Acyl, C₁ bis C₇ Acyloxy, Carboxy, geschütztes Carboxy, Carboxymethyl, geschütztes Carboxymethyl, Hydroxymethyl, geschütztes Hydroxymethyl, Amino, geschütztes Amino, (monosubstituiertes) Amino, geschütztes (monosubstituiertes) Amino, (disubstituiertes) Amino, Carboxamid, geschütztes Carboxamid, N-(C₁ bis C₆ Alkyl)carboxamid, geschütztes N-(C₁ bis C₆ Alkyl)carboxamid, N, N-di(C₁ bis C₆ Alkyl)carboxamid, Trifluoromethyl, N-((C₁ bis C₆ Alkyl)sulfonyl)amino, N-(Phenylsulfonyl)amino oder Phenyl,
und R₂ ist H, C₁ bis C₈ Alkyl,
C₂ bis C₆ Alkenyl, C₁ bis C₈ substituiertes Alkyl.

3. Verbindung gemäß Anspruch 1 oder 2 mit: R₁ ist Halogen und bevorzugt Jod gegenüber Brom und Chlor und R₂ ist H, C₁ bis C₆ Alkyl, C₃ bis C₅ Alkenyl, C₁ bis C₆ substituiertes Alkyl.

4. Verbindung gemäß einem der Ansprüche 1 bis 3 ausgewählt aus 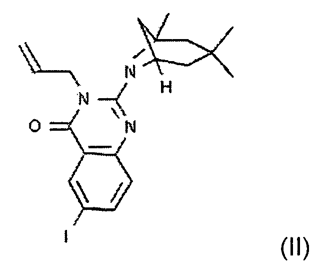 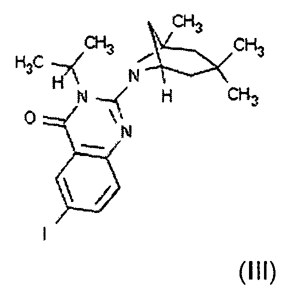 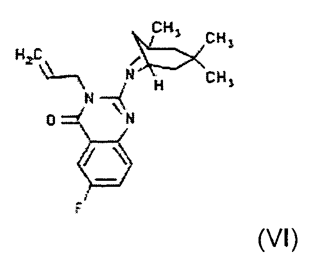 und

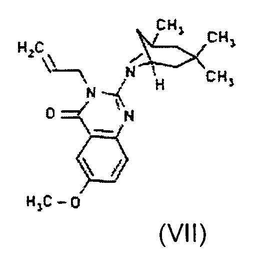

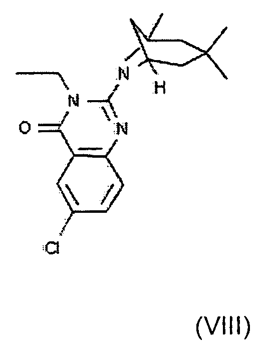

5. Verbindung gemäß Formel (I), oder pharmazeutisch annehmbare Salze oder Solvate davon, 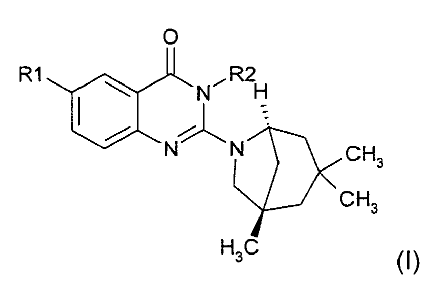 wobei die Substituenten und Indizes die folgende Bedeutung besitzen:
R₁ ist H, Halogen, Hydroxy, geschütztes Hydroxy, Cyano, Nitro, C₁ bis C₆ Alkyl, C₁ bis C₆ substituiertes Alkyl, C₁ bis C₇ Alkoxy, C₁ bis C₇ substituiertes Alkoxy, C₁ bis C₇ Acyl, C₁ bis C₇ substituiertes Acyl, C₁ bis C₇ Acyloxy, Carboxy, geschütztes Carboxy, Carboxymethyl, geschütztes Carboxymethyl, Hydroxymethyl, geschütztes Hydroxymethyl, Amino, geschütztes Amino, (monosubstituiertes) Amino, geschütztes (monosubstituiertes) Amino, (disubstituiertes) Amino, Carboxamid, geschütztes Carboxamid, N-(C₁ bis C₆ Alkyl)carboxamid, geschütztes N-(C₁ bis C₆ Alkyl)carboxamid, N, N-di(C₁ bis C₆ Alkyl)carboxamid, Trifluoromethyl, N-((C₁ bis C₆ Alkyl)sulfonyl)amino, N-(Phenylsulfonyl)amino oder Phenyl,
R₂ ist H, C₁ bis C₈ Alkyl, C₂ bis C₆ Alkenyl, C₁ bis C₈ substituiertes Alkyl, C₇ bis C₁₂ Phenylalkyl, substituiertes C₇ bis C₁₂ Phenylalkyl, zur Verwendung als Medikament.

6. Verwendung einer Verbindung gemäß Formel (I), oder pharmazeutisch annehmbare Salze oder Solvate davon, 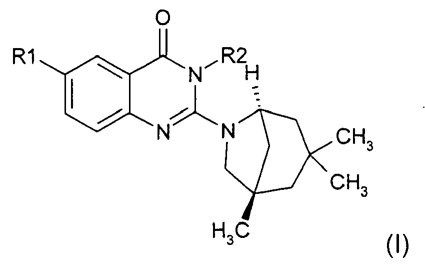 wobei die Substituenten und Indizes die folgende Bedeutung besitzen:
R₁ ist H, Halogen, Hydroxy, geschütztes Hydroxy, Cyano, Nitro, C₁ bis C₆ Alkyl, C₁ bis C₆ substituiertes Alkyl, C₁ bis C₇ Alkoxy, C₁ bis C₇ substituiertes Alkoxy, C₁ bis C₇ Acyl, C₁ bis C₇ substituiertes Acyl, C₁ bis C₇ Acyloxy, Carboxy, geschütztes Carboxy, Carboxymethyl, geschütztes Carboxymethyl, Hydroxymethyl, geschütztes Hydroxymethyl, Amino, geschütztes Amino, (monosubstituiertes) Amino, geschütztes (monosubstituiertes) Amino, (disubstituiertes) Amino, Carboxamid, geschütztes Carboxamid, N-(C₁ bis C₆ Alkyl)carboxamid, geschütztes N-(C₁ bis C₆ Alkyl)carboxamid, N, N-di(C₁ bis C₆ Alkyl)carboxamid, Trifluoromethyl, N-((C₁ bis C₆ Alkyl)sulfonyl)amino, N-(Phenylsulfonyl)amino oder Phenyl,
R₂ ist H, C₁ bis C₈ Alkyl, C₂ bis C₆ Alkenyl, C₁ bis C₈ substituiertes Alkyl, C₇ bis C₁₂ Phenylalkyl oder C₇ bis C₁₂ substituiertes Phenylalkyl,
zur Herstellung eines Medikaments zur Vorbeugung oder Behandlung einer Krankheit oder eines Zustandes, vermittelt durch ein NR1H3 Rezeptorprotein, das durch eine SEQ ID Nr.2 enthaltende Nucleinsäure verschlüsselt ist, einen Teil davon oder ein Säugetierhomologes davon, und/oder eine Krankheit oder ein Zustand, vermittelt durch ein NR1H2 Rezeptorprotein, das durch eine SEQ ID Nr:2 enthaltende Nucleinsäure verschlüsselt ist, eines Teils davon oder eines Säugetierhomologen davon in einem Säugetier, wobei die Vorbeugung oder Behandlung direkt oder indirekt durch die Bindung der Verbindung an das NR1H3 und/oder NR1H2 Rezeptorprotein oder NR1H3 und/oder NR1H2 Rezeptorproteinhomologe erreicht wird.

7. Verwendung gemäß Anspruch 6 zur Herstellung eines Medikaments zur Vorbeugung oder Behandlung einer durch NR1H3 und/oder NR1H2 Rezeptorprotein vermittelten Krankheit oder Zustandes, wobei das Säugetier ein Mensch ist.

8. Verwendung gemäß Anspruch 6 oder 7 zur Herstellung eines Medikaments zur Regulierung der Cholesterinsynthese oder -transports in einem Säugetier durch Aktivierung des NR1H3 und/oder NR1H2 Rezeptors.

9. Verwendung einer Verbindung wie in Anspruch 6 angegeben zur Herstellung eines Medikaments zur Regulierung der Spiegel von Cholesterin, Triglycerid, Gallsäure, Glukose und/oder Glukocorticoid.

10. Verwendung einer Verbindung wie in Anspruch 6 angegeben zur Herstellung eines Medikaments zur Behandlung eines Säugetiers bei Atherosclerose, Alzheimerkrankheit, Gallensteinkrankheit, Lipid-Störungen, entzündlichen Störungen, Typ II Diabetes, Fettleibigkeit oder einer Herzkreislaufstörung.

11. Verwendung einer Verbindung wie in Anspruch 6 angegeben zur Herstellung eines Medikaments, das zur Blockade der Cholesterin- und/oder Fettsäureabsorption im Darm bei Säugetieren geeignet ist.

12. Verwendung einer Verbindung wie in Anspruch 6 angegeben zur Herstellung eines Medikaments zur Modulation eines Gens, dessen Expression durch den NR1H3 und/oder NR1H2 Rezeptor gesteuert wird.

13. Verwendung einer Verbindung wie in Anspruch 6 angegeben in einem Säugetier zur Herstellung eines Medikaments zur selektiven Hoch-Regulierung eines oder mehrerer Gene, ausgewählt aus der Gruppe umfassend ABCA1, ABCG1, ABCG5, ABCG8, Apolipoprotein-CI, -CII, -CIV, -E, LPL (Lipoproteinlipase), CETP (Cholesterylester-Transferprotein) oder anderen Genen, die die Homöostase von Cholesterin zwangsregulieren.

14. Verwendung einer Verbindung wie in Anspruch 6 angegeben zur Herstellung eines Medikaments, das die Expression eines oder mehrerer Gene der Gruppe umfassend 11-β-HSD (11-β Hydroxysteroiddehydrogenase), PEPCK (Phosphoenolpyruvatcarboxykinase), G-6-P (Glukose-6-phosphatase) vermindert.

15. Verwendung einer Verbindung wie in Anspruch 6 angegeben zur Herstellung eines Medikaments, das die Expressionen eines oder mehrerer Gene, aus der Gruppe umfassend FAS (Fettsäuresynthase), SREBP-1c (Sterol-response Elementbindungsprotein), SCD-1 (Stearoyl-CoA Desaturase), Angiopoietin ähnliches Protein 3 (Angpt13) oder andere Gene, die für die Kontrolle der Serumspiegel von Triglycerid oder Glukose in der Leber oder anderen Organen im Vergleich mit der Verabreichung eines vollen Agonisten wie TO901317 nicht erhöht oder schwach erhöht.

16. Ein Medikament enthaltend eine Verbindung gemäß Formel (I), oder pharmazeutisch annehmbare Salze oder Solvate davon, 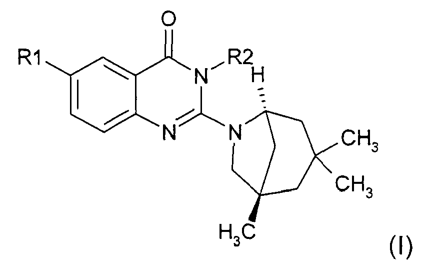 wobei die Substituenten und Indizes die folgende Bedeutung besitzen:
R₁ ist H, Halogen, Hydroxy, geschütztes Hydroxy, Cyano, Nitro, C₁ bis C₆ Alkyl, C₁ bis C₆ substituiertes Alkyl, C₁ bis C₇ Alkoxy, C₁ bis C₇ substituiertes Alkoxy, C₁ bis C₇ Acyl, C₁ bis C₇ substituiertes Acyl, C₁ bis C₇ Acyloxy, Carboxy, geschütztes Carboxy, Carboxymethyl, geschütztes Carboxymethyl, Hydroxymethyl, geschütztes Hydroxymethyl, Amino, geschütztes Amino, (monosubstituiertes) Amino, geschütztes (monosubstituiertes) Amino, (disubstituiertes) Amino, Carboxamid, geschütztes Carboxamid, N-(C₁ bis C₆ Alkyl)carboxamid, geschütztes N-(C₁ bis C₆ Alkyl)carboxamid, N, N-di(C₁ bis C₆ Alkyl)carboxamid, Trifluoromethyl, N-((C₁ bis C₆ Alkyl)sulfonyl)amino, N-(Phenylsulfonyl)amino oder Phenyl,
R2 ist H, C1 bis C8 Alkyl, C2 bis C6 Alkenyl, C1 bis C8 substituiertes Alkyl, C7 bis C12 Phenylalkyl oder C7 bis C12 substituiertes Phenylalkyl, und einen pharmazeutisch annehmbaren Träger.

## Revendications

1. Un composé de formule (I) ou un de ses sels ou dérivés de solvatation pharmaceutiquement acceptables, 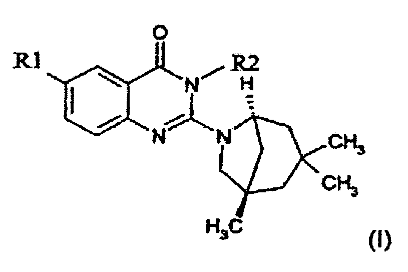 dans laquelle les substituants et les indices ont les significations suivantes :
R₁ est H, halogène, hydroxy, hydroxy protégé, cyano, nitro, alkyle en C₁ à C₆, alkyle en C₁ à C₆ substitué, alcoxy en C₁ à C₇, alcoxy en C₁ à C₇ substitué, acyle en C₁ à C₇, acyle en C₁ à C₇ substitué, acyloxy en C₁ à C₇, carboxy, carboxy protégé, carboxyméthyle, carboxyméthyle protégé, hydroxyméthyle, hydroxyméthyle protégé, amino, amino protégé, amino (monosubstitué), amino (monosubstitué) protégé, amino (disubstitué), carboxamide, carboxamide protégé, N-(alkyl en C₁ à C₆)-carboxamide, N-(alkyl en C₁ à C₆) carboxamide protégé, N,N-di(alkyl en C₁ à C₆)-carboxamide, trifluorométhyle, N-[alkyl en C₁ à C₆)-sulfonyl]-amino, N-phényl-sulfonyl)-amino ou phényle.
R₂ est H, alkyle en C₁ à C₈, alcényle en C₂ à C₆, alkyle en C₁ à C₈ substitué, phénylalkyle en C₇ à C₁₂ ou phénylalkyle substitué en C₇ à C₁₂,
avec cette condition que sont exclus les :
6-iodo-3-propyl-2-(1,3,3-triméthyl-6-azabicyclo[3.2.1]oct-6-yl)-4(3H)-quinazoline,
ester éthylique de l'acide 6-bromo-4-oxo-2-(1,3,3-triméthyl-6-azabicyclo[3.2.1]oct-6-yl)-3(4H)-quinazolinepropanoïque,
6-bromo-3-(2-propényl)-2-(1,3,3-triméthyl-6-azabicyclo[3.2.1]oct-6-yl)-4(3H)-quinazolinone,
6-bromo-3-propyl-2-(1,3,3-triméthyl-6-azabicyclo[3.2.1]oct-6-yl)-4(3H)-quinazolinone,
ester éthylique de l'acide 8-méthyl-4-oxo-2-(1,3,3-triméthyl-6-azabicyclo[3.2.1]oct-6-yl)-4(3H)-quinazolinone,
8-méthyl-3-(2-propényl)-2-(1,3,3-triméthyl-6-azabicyclo[3.2.1]oct-6-yl)-4(3H)-quinazolinone,
2-[éthyl-(4-pyridinylméthyl)-amino]-8-méthoxy-3-(2-phényléthyl)-4(3H)-quinazolone.

2. Un composé selon la revendication 1, ou un de ses sels ou dérivés de solvatation pharmaceutiquement acceptables, où les substituants et indices ont les significations suivantes :
R₁ est halogène, hydroxy, hydroxy protégé, cyano, nitro, alkyle en C₁ à C₆, alkyle en C₁ à C₆ substitué, alcoxy en C₁ à C₇, alcoxy en C₁ à C₇ substitué, acyle en C₁ à C₇, acyle en C₁ à C₇ substitué, acyloxy en C₁ à C₇, carboxy, carboxy protégé, carboxyméthyle, carboxyméthyle protégé, hydroxyméthyle, hydroxyméthyle protégé, amino, amino protégé, amino (monosubstitué), amino (monosubstitué) protégé, amino (disubstitué), carboxamide, carboxamide protégé, N-(alkyl en C₁ à C₆)-carboxamide, N-(alkyl en C₁ à C₆) carboxamide protégé, N,N-di(alkyl en C₁ à C₆)-carboxamide, trifluorométhyle, N-[alkyl en C₁ à C₆)-sulfonyl]-amino, N-phényl-sulfonyl)-amino ou phényle,
et R₂ est H, alkyle en C₁ à C₈, alcényle en C₂ à C₆, alkyle en C₁ à C₈ substitué.

3. Un composé selon l'une quelconque des revendications 1 ou 2, où R₁ est un halogène et de préférence l'iodure plutôt que le brome ou le chlore et R₂ est H, alkyle en C₁ à C₆, alcényle en C₃ à C₅, alkyle en C₁ à C₆ substitué.

4. Un composé selon l'une quelconque des revendications 1 à 3, choisi parmi les : 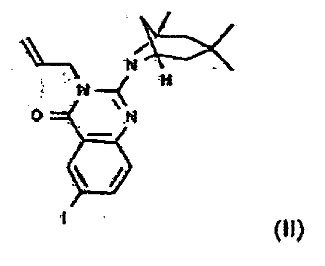 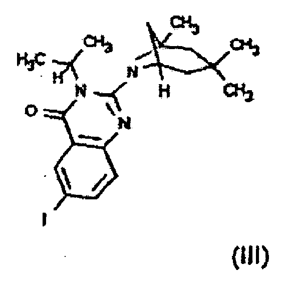 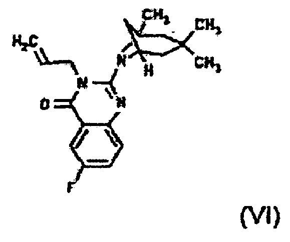 et

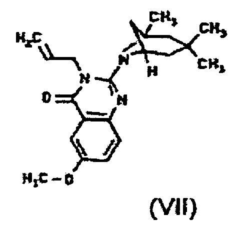

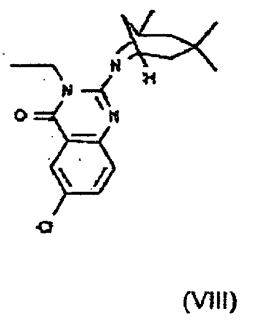

5. Un composé selon la formule (I) ou un de ses sels ou dérivés de solvatation pharmaceutiquement acceptables, 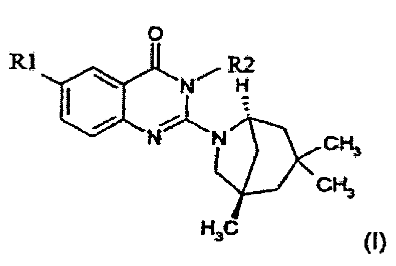 où les substituants et indices ont les significations suivantes :
R₁ est H, halogène, hydroxy, hydroxy protégé, cyano, nitro, alkyle en C₁ à C₆, alkyle en C₁ à C₆ substitué, alcoxy en C₁ à C₇, alcoxy en C₁ à C₇ substitué, acyle en C₁ à C₇, acyle en C₁ à C₇ substitué, acyloxy en C₁ à C₇, carboxy, carboxy protégé, carboxyméthyle, carboxyméthyle protégé, hydroxyméthyle, hydroxyméthyle protégé, amino, amino protégé, amino (monosubstitué), amino (monosubstitué) protégé, amino (disubstitué), carboxamide, carboxamide protégé, N-(alkyl en C₁ à C₆)-carboxamide, N-(alkyl en C₁ à C₆) carboxamide protégé, N,N-di(alkyl en C₁ à C₆)-carboxamide, trifluorométhyle, N-[alkyl en C₁ à C₆)-sulfonyl]-amino, N-phényl-sulfonyl)-amino ou phényle.
R₂ est H, alkyle en C₁ à C₈, alcényle en C₂ à C₆, alkyle en C₁ à C₈ substitué, phénylalkyle en C₇ à C₁₂ ou phénylalkyle en C₇ à C₁₂ substitué, destinés à être utilisé comme médicament.

6. Utilisation d'un composé de formule (I) ou d'un de ses sels ou dérivés de solvatation pharmaceutiquement acceptables, 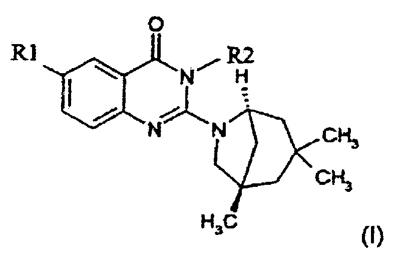 où les substituants et indices ont les significations suivantes :
R₁ est H, halogène, hydroxy, hydroxy protégé, cyano, nitro, alkyle en C₁ à C₆, alkyle en C₁ à C₆ substitué, alcoxy en C₁ à C₇, alcoxy en C₁ à C₇ substitué, acyle en C₁ à C₇, acyle en C₁ à C₇ substitué, acyloxy en C₁ à C₇, carboxy, carboxy protégé, carboxyméthyle, carboxyméthyle protégé, hydroxyméthyle, hydroxyméthyle protégé, amino, amino protégé, amino (monosubstitué), amino (monosubstitué) protégé, amino (disubstitué), carboxamide, carboxamide protégé, N-(alkyl en C₁ à C₆)-carboxamide, N-(alkyl en C₁ à C₆) carboxamide protégé, N,N-di(alkyl en C₁ à C₆)-carboxamide, trifluorométhyle, N-[alkyl en C₁ à C₆)-sulfonyl]-amino, N-phényl-sulfonyl)-amino ou phényle.
R₂ est H, alkyle en C₁ à C₈, alcényle en C₂ à C₆, alkyle en C₁ à C₈ substitué, phénylalkyle en C₇ à C₁₂, phénylalkyle en C₇ à C₁₂ substitué,
en vue de la fabrication d'un médicament pour la prévention ou le traitement d'une maladie ou d'un état susceptible d'être médié par la protéine réceptrice NR1H3 codée par un acide nucléique comprenant SEQ ID N° 2, une de ces parties ou de ces homologues de mammifères, et/ou une maladie ou état susceptible d'être médié par une protéine réceptrice NR1H2 codée par un acide nucléique comprenant SEQ ID N° 2, une de ces parties ou un de ces homologues de mammifère chez un mammifère, où la prévention ou le traitement est effectué directement ou indirectement par la fixation du composé à la protéine réceptrice NR1H3 et/ou NR1H2 ou un homologue de protéine réceptrice NR1H3 et/ou NR1H2.

7. Utilisation selon la revendication 6 en vue de la fabrication d'un médicament pour la prévention ou le traitement d'une maladie ou d'un état susceptible d'être médié par une protéine réceptrice NR1H3 et/ou NR1H2 où le mammifère est un être humain.

8. Utilisation selon l'une quelconque des revendications 6 ou 7 en vue de la fabrication d'un médicament pour réguler la synthèse ou le transport du cholestérol chez un mammifère en activant le récepteur NR1H3 et/ou NR1H2.

9. Utilisation d'un composé selon la revendication 7 en vue de la fabrication d'un médicament pour la régulation des taux de cholestérol, de triglycéride, d'acide biliaire, de glucose et/ou de glucocorticoïde.

10. Utilisation d'un composé selon la revendication 6 en vue de la fabrication d'un médicament pour le traitement chez un mammifère de l'athérosclérose, de la maladie d'Alzheimer, de calculs biliaires, des troubles lipidiques, du désordre inflammatoire, du diabète du type II, de l'obésité ou des troubles cardiovasculaires.

11. Utilisation d'un composé selon la revendication 6 en vue de la fabrication d'un médicament pouvant bloquer chez un mammifère l'absorption du cholestérol et/ou des acides gras dans l'intestin.

12. Utilisation d'un composé selon la revendication 6 en vue de la fabrication d'un médicament pour moduler un gène dont l'expression est régulée par le récepteur NR1H3 et/ou NR1H2.

13. Utilisation d'un composé selon la revendication 6 chez un mammifère en vue de la fabrication d'un médicament pour la sur-régulation sélective d'un ou plusieurs gènes choisis dans le groupe comprenant ABCA1, ABCG1, ABCG5, ABCG8, apolipoprotéine-C1, -CII, -CIV, -E, LPL (lipoprotéine lipase), CETP (protéine de transfert de l'ester de cholestéryle) ou autres gènes qui régulent positivement l'homéostase du cholestérol.

14. Utilisation d'un composé selon la revendication 6 en vue de la fabrication d'un médicament qui abaisse l'expression d'un ou plusieurs des gènes appartenant au groupe comprenant les 11-β-HSD(11-β-hydroxystéroïde déshydrogénase), PEPCK (phosphoénolpyruvate carboxykinase), G-6-P (glucose-6-phosphatase).

15. Utilisation d'un composé selon la revendication 6 en vue de la fabrication d'un médicament qui n'élève pas ou n'élève que peu l'expression d'un ou plusieurs des gènes appartenant au groupe comprenant FAS (acide gras synthase), SREBP-1c (protéine de liaison de l'élément de réponse au stérol), SCD-1 (stéaroyl-CoA-désaturase), protéine de type angiopoiétine 3 (Angptt3) ou autres gènes qui sont pertinents pour le contrôle des taux de triglycéride ou de glucose sérique dans le foie et autres organes par rapport à l'administration d'un agoniste complet comme le TO901317.

16. Un médicament comprenant un composé selon la formule (I) ou un de ses sels ou dérivés de solvatation pharmaceutiquement acceptables, 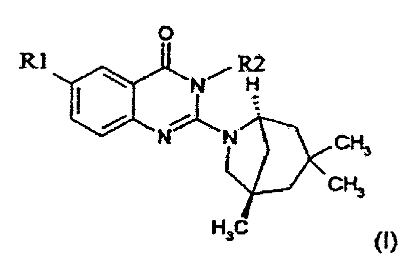 où les substituants et indices ont les significations suivantes :
R₁ est H, halogène, hydroxy, hydroxy protégé, cyano, nitro, alkyle en C₁ à C₆, alkyle en C₁ à C₆ substitué, alcoxy en C₁ à C₇, alcoxy en C₁ à C₇ substitué, acyle en C₁ à C₇, acyle en C₁ à C₇ substitué, acyloxy en C₁ à C₇, carboxy, carboxy protégé, carboxyméthyle, carboxyméthyle protégé, hydroxyméthyle, hydroxyméthyle protégé, amino, amino protégé, amino (monosubstitué), amino (monosubstitué) protégé, amino (disubstitué), carboxamide, carboxamide protégé, N-(alkyl en C₁ à C₆)-carboxamide, N-(alkyl en C₁ à C₆) carboxamide protégé, N,N-di(alkyl en C₁ à C₆)-carboxamide, trifluorométhyle, N-[alkyl en C₁ à C₆)-sulfonyl]-amino, N-phényl-sulfonyl)-amino ou phényle.
R₂ est H, alkyle en C₁ à C₈, alcényle en C₂ à C₆, alkyle en C₁ à C₈ substitué, phénylalkyle en C₇ à C₁₂ ou phénylalkyle en C₇ à C₁₂ substitué, et un support pharmaceutiquement acceptable.
